# EUROPEAN PATENT APPLICATION

(11) **EP 4 183 432 A1**
(43) Date of publication of application: **24.05.2023**
(21) Application number: 20949945.8
(22) Date of filing: 27.08.2020
(51) Int. Cl.: A61M 1/34, A61M 1/38, A61M 1/36, B01D 61/00, B01D 61/14, B01D 61/02, C07K 1/34

(54) **FLUID TREATMENT METHOD AND FLUID TREATMENT DEVICE**

(30) Priority: 19.08.2020 CN 202010838769
(71) Applicant: Shanghai Xinguang Bio-Pharmaceutical Ltd., Shanghai 201210 (CN)
(72) Inventor: LI, Xianghai, Shanghai 201210 (CN)
(74) Representative: Canzler & Bergmeier Patentanwälte Partnerschaft mbB
(86) International application number: PCT/CN2020/111881
(87) International publication number: WO 2022/036738

(57) **Abstract**

Disclosed in the present application are a fluid treatment method, a fluid treatment device, a circulating separation device, a circulating treatment system, medical equipment, and a computer-readable storage medium. The fluid treatment method traps target materials in a fluid by means of an enrichment pipeline and a first separation module and cyclically enriches the target materials in the enrichment pipeline, and in this cyclic enrichment mode, the flow rates of the fluid at a fluid inlet and a fluid outlet in the enrichment pipeline are equal so as to dynamically balance the total amount of the fluid in the enrichment pipeline. Thus, the fluid treatment method of the present application is sustainable in treatment, and can collect some components in the fluid, that is, target materials. The fluid drained out of a first drainage port of the enrichment pipeline can be connected to a treatment module or returned to the original system. This method effectively avoids the continuous loss of beneficial components when harmful components are separated during component separation or exchange of the fluid through membrane separation.

## Description

### Technical Field

The present disclosure relates to fluid treatment technology, and in particular to a fluid treatment method, a fluid treatment device, a cycle separation device, a cycle treatment system, a medical device, and a non-transitory computer-readable storage medium.

### Background

Separation of substances is commonly achieved by membrane separation; for example, in the medical field, filtering out a specific component or changing concentration thereof in various tissue fluids of human, such as blood or plasma, is achieved by membrane separation, water can be purified through membrane separation in water treatment, and mixed gases can be purified through membrane separation.

However, in existing membrane separation technologies, especially in medical applications, when intercepting desired components, removing or reducing concentration of a specific component, waste liquid will be inevitably produced, and the waste liquid not only contains the aforementioned specific component, but also contains beneficial components. In other words, it is difficult to achieve targeted or selective separation of components using the existing membrane separation technologies. Since the membrane separation process is accompanied by the loss of beneficial components such as glucose, amino acids, albumin, vitamins, hormones, electrolytes, there are many limitations in the clinical application of the separation technology. For example, the duration of the separation process is limited; in addition, the removal effect or removal rate of membrane separation cannot be guaranteed, resulting in, among other things, failures to precisely control the concentration of specific components; further, the continuous generation of waste liquid not only causes direct damage to patients, but also brings about secondary risks of infection and pathogenic microorganism transmission.

### Summary

The present disclosure provides a fluid treatment device, a cycle separation device and a fluid treatment method to solve problems of membrane separation in the related art, such as poor selectivity, loss of beneficial components, and insufficient sustainability.

A first aspect of the present disclosure provides a fluid treatment method, including: introducing a fluid into an enrichment pipeline; the enrichment pipeline includes a first section and a second section, an entrance of the first section is communicated with at least one first inlet, and an exit of the second section is communicated with the first section; intercepting a target substance in the fluid by at least one first separation module; each of the at least one first separation module includes a first separation component to divide a corresponding first separation module into a first side and a second side, two opposite ends of the first side of each of the at least one first separation module are communicated with the first section and the second section respectively, and the second side of each of the at least one first separation module is communicated with at least one first outlet; and driving the fluid in the enrichment pipeline to cyclically flow at a first preset flow rate based on at least one first driving device to enrich the target substance; the at least one first driving device is arranged at the first section and is configured to dynamically balance a total amount of the fluid in the enrichment pipeline.

A second aspect of the present disclosure provides a fluid treatment method, including: introducing a fluid into a separation pipeline through at least one second separation module; the separation pipeline has an entrance and an exit, each of the at least one second separation module includes a second separation component to divide a corresponding first separation module into a first side and a second side, two opposite ends of the first side of each of the at least one second separation module are communicated with at least one second inlet and at least one second outlet respectively, and two opposite ends of the second side of each of the at least one second separation module are communicated with the entrance and exit of the separation pipeline; and driving a fluid in the separation pipeline to flow from the entrance of the separation pipeline to the exit of the separation pipeline at a third preset flow rate by at least one second driving device, to dynamically balance a total amount of the fluid in the separation pipeline.

A third aspect of the present disclosure provides a fluid treatment device, including: at least one cycle enrichment module; the cycle enrichment module includes: an enrichment pipeline, including a first section and a second section; an entrance of the first section is communicated with at least one first inlet, and an exit of the second section is communicated with the first section; at least one first separation module; each of the at least one first separation module includes a first separation component to divide a corresponding first separation module into a first side and a second side; two opposite ends of the first side are communicated with the first section and the second section respectively, and the second side is communicated with at least one first outlet; and at least one first driving device, arranged at the first section, for driving a fluid to cyclically flow in the enrichment pipeline to dynamically balance a total amount of the fluid in the enrichment pipeline, so that the at least one first separation module enriches the target substance in a cycle enrichment mode.

A fourth aspect of the present disclosure provides a cycle separation device, for performing fluid treatment, including: at least one cycle separation module, the cycle separation module includes: a separation pipeline, having an entrance and an exit; a second separation module, including a second separation component; the second separation component divides the second separation module into a first side and a second side; two opposite ends of the first side of the second separation module are communicated with at least one second inlet and at least one second outlet respectively, and two opposite ends of the second side of the second separation module are communicated with the inlet of the separation pipeline and the outlet of the separation pipeline respectively; and at least one second driving device, arranged at the separation pipeline, for driving a fluid in the separation pipeline flow from the inlet of the separation pipeline to the outlet of the separation pipeline at a preset flow rate, to dynamically balance a total amount of the fluid in the separation pipeline in a cycle separation mode.

A fifth aspect of the present disclosure provides a cycle treatment system, including: at least one fluid treatment device as described in any one of the embodiments provided in the third aspect of the present disclosure and/or at least one cycle separation device as described in any one of the embodiments provided in the fourth aspect of the present disclosure; and a pipeline system, including a fluid-introducing pipeline and a fluid-returning pipeline.

A sixth aspect of the present disclosure provides a medical device, including: a cycle treatment system as described in any one of the embodiments provided in the fifth aspect of the present disclosure.

A seventh aspect of the present disclosure provides a non-transitory computer-readable storage medium, at least one program is stored on the computer-readable storage medium, and the fluid treatment method as described in any one of the embodiments provided in the first aspect of the present disclosure, or the fluid treatment method as described in any one of the embodiments provided in the second aspect of the present disclosure is implemented when the at least one program is executed by a processor.

As described above, the fluid treatment method, the fluid treatment device, the cycle separation device, the cycle treatment system, and the medical device of the present disclosure have the following beneficial effects: the enrichment pipeline and the first separation module intercept the target substance of the fluid and enable the target substance to cyclically flow in the enrichment pipeline. In the cycle enrichment mode, the first driving device drives the fluid in the enrichment pipeline to flow at the preset flow rate, thereby dynamically balancing the total amount of the fluid in the enrichment pipeline. The fluid treatment method of the present disclosure can sustainably treat the fluid, collect or treat particular components (e.g., the target substance) of the fluid, and flexibly control the collection duration or the treatment duration. The fluid discharged from the first outlet of the enrichment pipeline is introduced into the treatment module or a storage space, which avoids the continuous loss of beneficial components during the separation or exchange of components of the fluid. The treatment device may be connected to any pipeline transporting the fluid to be treated, in which case when enriching/removing the target substance, the enrichment pipeline can also maintain mobile and air-tight. The treatment device may also be connected to any container storing the fluid to be treated, in which case when enriching/removing the target substance, the enrichment pipeline can also stabilize the total amount of the fluid.

### Brief Description of the Drawings

The specific features of the present disclosure are shown in the appended claims. The features and advantages of the present disclosure can be better understood by referring to the exemplary embodiments and drawings described in detail below. A brief description of the drawings is as follows:
FIG. 1 shows a schematic flowchart of a fluid treatment method according to an embodiment of the present disclosure.
FIG. 2 shows a schematic diagram of a cycle enrichment module according to an embodiment of the present disclosure.
FIG. 3A shows a schematic diagram of a cycle enrichment module according to an embodiment of the present disclosure.
FIG. 3B shows a schematic diagram of a cycle enrichment module according to an embodiment of the present disclosure.
FIG. 4 shows a schematic diagram of a cycle enrichment module according to an embodiment of the present disclosure.
FIG. 5 shows a schematic diagram of a cycle enrichment module according to an embodiment of the present disclosure.
FIGs. 6A-6C show schematic diagrams of a cycle enrichment module in different operating states according to an embodiment of the present disclosure.
FIGs. 7A-7B show schematic diagrams of a cycle enrichment module in different operating states according to an embodiment of the present disclosure.
FIG. 8 shows a schematic diagram of a cycle enrichment module according to an embodiment of the present disclosure.
FIG. 9 shows a schematic diagram of cascaded cycle enrichment modules according to an embodiment of the present disclosure.
FIG. 10 shows a flowchart of a fluid treatment method according to an embodiment of the present disclosure.
FIG. 11 shows a schematic diagram of a cycle enrichment module and a cycle separation module cascaded one after the other according to an embodiment of the present disclosure.
FIG. 12 shows a schematic diagram of a cycle separation module according to an embodiment of the present disclosure.
FIG. 13 shows a flowchart of a fluid treatment method according to an embodiment of the present disclosure.
FIG. 14 shows a schematic diagram of cascaded cycle separation modules according to an embodiment of the present disclosure.
FIG. 15A shows a schematic diagram of a cycle separation module and a cycle enrichment module cascaded one after the other according to an embodiment of the present disclosure.
FIG. 15B shows a schematic diagram of a cycle separation module and a cycle enrichment module cascaded one after the other according to an embodiment of the present disclosure.
FIG. 16 shows a flowchart of a fluid treatment method according to an embodiment of the present disclosure.
FIG. 17 shows a schematic diagram of a cycle separation module and a cycle enrichment module cascaded one after the other according to an embodiment of the present disclosure.
FIG. 18 shows a schematic diagram of a cycle treatment system according to an embodiment of the present disclosure.
FIG. 19 shows a schematic diagram of a cycle treatment system according to an embodiment of the present disclosure.
FIG. 20 shows a schematic diagram of a cycle treatment system according to an embodiment of the present disclosure.

### Detailed Description

The implementations of the present disclosure are described below through specific examples. Those skilled in the art can easily understand the other advantages and effects of the present disclosure from the content disclosed in this specification

In the following description, several embodiments of the present disclosure are described by referring to the drawings. It should be understood that other embodiments can also be used to implement the present disclosure, and changes in mechanical composition, structure, electricity and operation can be made without departing from the spirit and scope of the present disclosure. The following detailed description should not be considered as limited, and the scope of the present disclosure is limited only by the claims of the published patent. The terms used herein are only intended to describe specific embodiments and are not intended to limit the present disclosure. Spatial-related terms, such as "up", "down", "left", "right", "below", "under", "beneath", "above", "over", etc., can be used herein to facilitate the description of the relationship between one element or feature and another element or feature shown in the figures.

In some embodiments, although the terms "first", "second", and the like are used herein to describe various elements or parameters, these elements or parameters should not be limited by these terms. These terms are only used to distinguish one element or parameter from another. For example, a first separation module may be referred to as a second separation module, and similarly, a second separation module may be referred to as a first separation module without departing from the scope of the various described embodiments. "A first separation module" and "a second separation module" are both used to describe a separation module, but they are not the same separation module unless the context clearly indicates otherwise. Similarly, a first separation component and a second separation component, or a first side and a second side are similar to the above.

Furthermore, as used herein, the singular forms "one", "a/an" and "the" are intended to include the plural form, unless the context indicates otherwise. It should be further understood that the terms "include" and "comprise" indicate the existence of the described features, steps, operations, elements, components, items, categories, and/or groups, but do not exclude the existence, presence, or addition of one or more other features, steps, operations, elements, components, items, categories, and/or groups. The terms "or" and "and/or" as used herein are interpreted to be inclusive or to mean any one or any combination thereof. Therefore, "A, B or C" or "A, B and/or C" means any of the following: "A; B; C; A and B; A and C; B and C; A, B and C". Exceptions to this definition occur only when combinations of components, functions, steps, or operations are inherently mutually exclusive in some way.

Take membrane separation as an example, the membrane separation is a process of separation, purification and concentration of different components in a mixture by utilizing selective separation of membranes, which is an efficient, energy-saving and environment-friendly separation method. Methods of membrane separation include normal flow filtration (NFF, also known as dead end filtering) and tangential/cross flow filtration (TFF). During an NFF process, a liquid passes through a membrane vertically and molecules are directly filtered out, but the intercepted molecules are easy to form a layer of high-concentration gel and a layer of particle on the membrane's surface, which will lead to a sharp drop of the flow rate and flux. During a TFF process, the movement direction of the liquid is parallel to (tangential to) the membrane's surface. The trans-membrane pressure generated by the liquid drives some solutions and small molecules to cross the membrane, and some other solutions and molecules with large molecular weights are intercepted. During the whole process, the liquid continuously flows through the surface of the membrane at a certain speed, thereby washing the surface of the membrane and bringing particles deposited on the surface of the membrane out of the TFF module.

During membrane separation, it is difficult to avoid producing waste liquid when filtering out and separating specific components or molecules for removal. And the waste liquid in medical applications may contain glucose, amino acids, albumin, vitamins, hormones, electrolytes, etc. Therefore, the technology of membrane separation has the problem of consumption of beneficial components during filtration in many scenarios.

For example, membrane separation based on steric effect is essentially a process of intercepting components with larger molecular weight or particle size in a mixture and allowing components with smaller molecular weight or particle size to pass through, which reflects the directivity of the separation process. To facilitate the description of the method, device and system provided in the present disclosure, different types of membrane separation are classified into the following two modes based on their purposes:
Mode A: large molecules are beneficial and small molecules are harmful; that is, small molecules are components that need to be removed.
Mode B: large molecules are harmful and small molecules are beneficial; that is, large molecules are components that need to be removed.

In practical applications, mode B is relatively common, it is also relatively developed, and is now applied in filtration and sterilization process, water treatment, and purification process, etc. Mode B is generally realized by the dead end filtering; however, when there is a high content of molecules with a large molecular weight, the membrane gets blocked easily, and therefore the membrane cannot be used continuously, i.e., there is an upper limit on the amount of liquid treated by the membrane. In this case, pretreatment, such as precipitation, distillation or other physical methods, is generally required. If pretreatment is not practical, such as when the mixture is blood, plasma, etc., the dead end filtering will be inoperable as a result.

Mode A is more common in biological applications, such as the concentration and purification of recombinant proteins, hemodialysis and plasma exchange, which are processes to remove harmful or worthless small molecules from the system. Traditional plasma exchange technology, as a typical application of mode A, has the same problems as mode A. Since large molecules are beneficial, tangential flow is generally used to facilitate the intercepted molecules returning to the original system, and to avoid deposition of these molecules on the surface of the membrane, which negatively affects the continuity of the treatment process. However, in practical applications, due to poor selectivity, the small molecules removed in mode A often include beneficial components, such as sugars, amino acids, vitamins, etc. This means there will be a substantial loss of beneficial components when using a physical method of membrane separation for blood treatment, making the treatment process unsustainable.

Take applications in the medical field as an example, when plasma exchange equipment is applied in clinical scenarios such as kidney diseases, cryoglobulinemia, hyperacute or acute antibody-mediated rejection after renal transplantation, plasma exchange can be achieved based on membrane separation to filter out macromolecular pathogenic substances and immune complexes in the blood. In addition to pathogenic factors, there are often beneficial components in the waste liquid. Therefore, the treatment process will be accompanied by the loss of beneficial components in the blood, which lows the sustainability of the plasma exchange process and limits the clearance efficiency of pathogenic factors. In other scenarios, especially for a traditional plasma exchange technology, blood renewal is achieved by separating plasma and delivering plasma substitutes to patients. However, plasma substitutes may cause allergies to the human body, which limits their scope of applications, and it is usually only used for acute indications such as familial hypercholesterolemia, hyperlipoproteinemia, systemic lupus erythematosus (SLE), myasthenia gravis, rapidly progressive glomerulonephritis, and autoimmune diseases. Besides, when plasma is separated and extracted for replacement, there is also a problem that beneficial components other than plasma, such as platelets, are extracted. Therefore, this method still has the defect of loss of beneficial components, thus making the sustainability of the method low.

For traditional technology of double filtration plasmapheresis ( DFPP), the plasma is usually filtered twice in a corresponding filtering system, where a technology for separating plasma (i.e., separating plasma, red blood cells, platelets and hemameba from the blood) and a technology for separating plasma component (i.e., separating specific components from the plasma) are adopted to form a two-stage filtration to remove macromolecular pathogenic factors such as autoantibodies, immunoglobulins, immune complexes, inflammatory molecules or low-density lipoproteins in the separation of plasma components. Therefore, DFPP is usually used for severe diseases (such as myasthenia gravis, Guillain Barre syndrome, systemic lupus erythematosus (SLE), rheumatoid arthritis, hyperlipidemia severe acute pancreatitis, sepsis) with macromolecules as pathogenic factors, but DFPP is not suitable for the removal of small and medium molecular pathogenic substances combined with albumin, and not suitable for the removal of free small and medium molecular solutes.

DFPP is a combination of mode A and mode B where intercepted molecules whose size or molecular weight is between the two pore sizes of membrane or two molecular weight cutoffs corresponding to the two modes are the molecules to be removed. In DFPP, the tangential flow filtration can be used to solve the problems in mode B, and can effectively avoid the problem of blocking caused by dead end filtering. However, in practical applications of DFPP, when removing harmful macromolecules, beneficial small molecules will still be removed as well. In the process of separation, a plasma abandonment rate is generally 10%-30%, and the plasma abandonment rate refers to the ratio of the transport rate of a plasma abandonment pump to that of a plasma separation pump; more specifically, in the process of double filtration plasmapheresis, the flow rate of discarded plasma is usually 2.5 - 6 ml/min, which means that patients will lose more than 300-720 mL of plasma after receiving treatment for 2 hours even if the double filtration plasmapheresis is used. That is, if DFPP is used to exchange the plasma of patients, a certain volume of plasma to be discarded, i.e., waste liquid, will inevitably be produced after a period of time. In fact, the waste liquid contains beneficial components from human body. If a replacement liquid, such as exogenous plasma, is used for delivering to patients, it may cause allergic reactions in the human body. Due to the continuous loss of beneficial components in the traditional DFPP technology, this treatment method has significant defects and risks, and can only be used in emergencies and ICUs. Therefore, in clinical treatment, it is necessary to limit the treatment time (usually limited to 2-5 h) and application scenarios of DFPP. Due to the limitation of treatment time, DFPP also has a limited removal effect.

For another example, CN103263704A has disclosed an adsorption filtration purification system, where a certain amount of plasma is separated from the human blood, and then the plasma is introduced into a purification unit to perform multiple adsorption processes to regenerate part of the discarded plasma and reduce the demand of fresh plasma in plasma exchange. However, during the absorption processes of plasma, the filtrate produced by the high-throughput filter used in the purification unit is waste liquid. Therefore, although a certain proportion of regenerated plasma is obtained in the plasma regeneration processes, waste liquid can still be generated. The problem of waste liquid and the continuous loss of beneficial components that may be caused by the waste liquid still exist in CN103263704A, and the filtrate is supplemented by replacement liquid, resulting in the risk of allergies caused by foreign substances in the aforementioned DFPP. Further, the high-throughput filter in the adsorption filtration purification system is only applicable to the treatment of small molecule plasma toxins, and multiple filtrations are carried out to improve the filtering effect on small molecule toxins to regenerate a certain proportion of plasma in the waste plasma. However, this adsorption filtration purification system cannot remove the pathogenic factors with a large molecular weight from blood, so that its application scenarios are limited, and it is difficult to collect or remove different components in blood using this system.

Herein, the present disclosure provides solutions for how to avoid the continuous loss of beneficial small molecules or components as much as practical while removing harmful large molecules or components in Mode B. The present disclosure provides a fluid treatment method, a fluid treatment device, and a cycle separation device that can be used to treat various fluids (including a fluid in medical scenarios), for target enrichment or removal (or change in concentration) of specific molecules or components of a fluid based on a predetermined treatment target, and the fluid treatment method and the fluid treatment device of the present disclosure can be used to perform a sustainable separation process that achieves controlled removal effects and effectively avoids or reduces the continuous loss of beneficial components.

It should be noted that in the embodiments provided in the present disclosure, "large" and "small" are relative concepts. For example, "large" and "small" may be determined based on the relative size (such as, particle size or weight) of the molecules or substances compared to each other, e.g., for lipoproteins and amino acids, lipoproteins are the large molecules and amino acids are the small molecules. "Large" and "small" may also be based on their molecular particle size or molecular weight relative to the molecular weight cutoff or pore size of the membrane, e.g., when the pore size of separation components is 0.5 µm, cells and platelets are large molecules while plasma proteins are small molecules; while for a 10 kD porous membrane, lipoproteins, albumin and cells are large molecules while glucose, amino acids, etc. are small molecules; i.e., large molecules, the macromolecular components, small molecules, small molecular components, etc., described in the present disclosure are relative concepts used to describe molecules that are intercepted or filtered out.

A first aspect of the present disclosure provides a fluid treatment method, including: introducing a fluid into an enrichment pipeline; the enrichment pipeline includes a first section and a second section, an entrance of the first section is communicated with at least one first inlet; intercepting a target substance in the fluid by at least one first separation module; each of the at least one first separation module includes a first separation component to divide a corresponding first separation module into a first side and a second side, two opposite ends of the first side of each of the at least one first separation module are communicated with the first section and the second section respectively, and the second side of each of the at least one first separation module is communicated with at least one first outlet; and driving the fluid in the enrichment pipeline to cyclically flow at a first preset flow rate based on at least one first driving device to enrich the target substance; the at least one first driving device is arranged at the first section and is configured to dynamically balance a total amount of the fluid in the enrichment pipeline.

It should be noted that in the embodiment provided by the present disclosure, the target substance of a certain enrichment pipeline is determined based on a predetermined target for fluid treatment. Target substances to be enriched and cycled in different enrichment pipelines can be of the same class or have the same composition or molecule; they can also be different substances; the specific composition of the target substance can be changed based on a composition of the fluid, and a predetermined separation purpose. Take medical uses as an example, the target substance can be a cell, bacteria, microorganism, protein, lipoprotein, antibody, DNA, etc. In the fluid treatment method, the target substance is the substance intercepted in the pipeline, i.e., when the fluid treatment method provided in the present disclosure is adopted to treat the fluid, any substance intercepted in the enrichment pipeline for enrichment can be considered as the target substance. In practical applications, the target substance can be a component to be collected or a component to be removed from the original fluid.

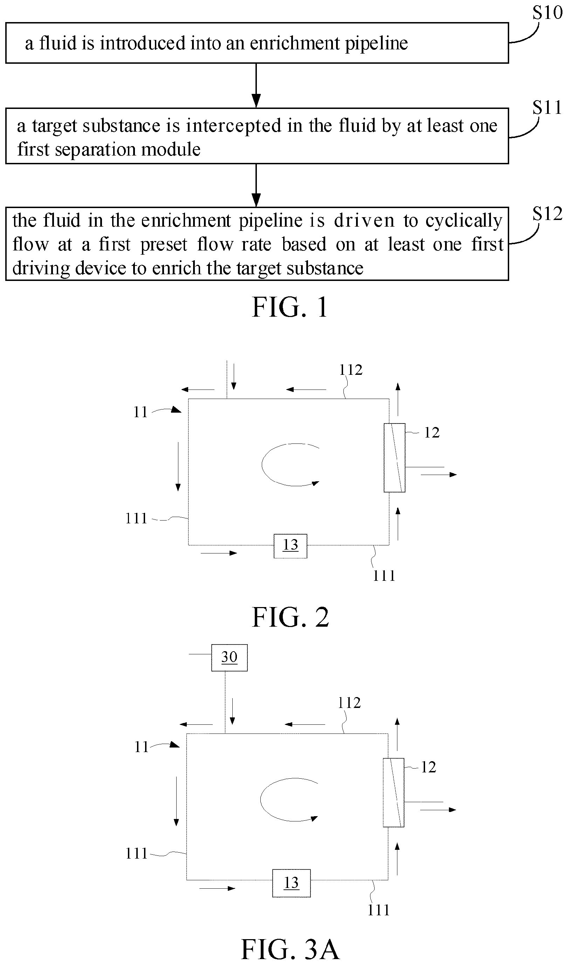

Referring to FIG. 1, FIG. 1 shows a flowchart of a fluid treatment method according to an embodiment of the present disclosure.

In step S10, a fluid is introduced into an enrichment pipeline, the enrichment pipeline includes a first section and a second section, an entrance of the first section is communicated with at least one first inlet, and an exit of the second section is communicated with the first section. In the present disclosure, the word "communicated" means that the fluid can flow between the two mechanical structures. In some occasions, the word "communicated" may be replaced by "interconnected" or "connected" and have the same meaning.

Refer to FIGs. 1 and 2. FIG. 2 shows a flowchart of an enrichment pipeline of a fluid treatment method according to an embodiment of the present disclosure.

The fluid contains the target substance; in some embodiments, the fluid includes, but is not limited to, one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, light distillate oil, methane, and liquefied gas. In some embodiments, the fluid may also be a component obtained after a fluid, such as blood, plasma, etc., has undergone treatment such as filtering. In other embodiments, the fluid may also be a mixture of gas, such as a gas mixture including methane.

The fluid treatment method of the present disclosure can be applied to different types of fluid treatment. Generally speaking, in order to make the separation process meaningful, the fluid needs to have different material components, such as components having different permeabilities when passing through the separation membrane, and to have a certain degree of mobility. For example, the fluid can be a mixture of liquid or gas as described above, or the fluid can be other solid-liquid mixed-phase fluids or colloids.

The first section 111 and the second section 112 in the enrichment pipeline 11 are so connected that the liquid can cyclically flow in the enrichment pipeline 11. Specifically, the exit of the second section 112 is communicated with the first section 111. The fluid enters the first section 111 from the first inlet, passes through the first separation module 12, flows to the second section 112, and flows from the outlet of the second section 112 to the first section 111; that is, the fluid cyclically flows in the enrichment pipeline 11.

A path pattern of fluid circulation in the enrichment pipeline 11 can be determined by the first section 111 and the second section 112. For example, as shown in FIG. 2, the path pattern is a rectangle. In other embodiments, the path pattern can include a broken line composed of connected line segments, or arcs (e.g., a circular arc). In actual scenarios, the first section 111 is communicated with the second section 112 and the fluid can flow between the first section 111 and the second section 112. Based on equipment needs of different scenarios, the pipeline diameters, materials and paths of the first section 111 and the second section 112 can be adjusted accordingly.

In actual scenarios, the materials of the pipelines of the first section 111 and the second section 112 can materials specifically selected for fluid-related applications; for example, part or all of the first section 111 and the second section 112 can be a dedicated pipeline for blood, a dedicated pipeline for peristaltic pump, a dedicated pipeline for corrosive liquid, a pipeline with high biocompatibility, etc. When the fluid treatment method is applied in medical scenarios, the first section 111 and the second section 112 can be a blood delivery pipeline or a liquid medicine delivery pipeline, and the dedicated pipeline's materials include one or more soft polyvinyl chloride, high-performance thermoplastic polyolefin elastomer (TPE), nano biomedical materials, and resin materials.

The entrance of the first section 111 is communicated with the at least one first inlet, and the at least one first inlet is used as an entrance to introduce a fluid into the enrichment pipeline. In most embodiments, one first inlet is enough to achieve the introduction of a fluid into the enrichment pipeline. In some scenarios, if there are multiple first inlets, the multiple first inlets may introduce a fluid with different substance components into the enrichment pipeline; for example, plasma whose immunoglobulins have been filtered out is introduced from one first inlet and plasma whose rheumatoid factor have been filtered out is introduced from another first inlet.

In step S11, a target substance in the fluid is intercepted by at least one first separation module; each of the at least one first separation module includes a first separation component to divide a corresponding first separation module into a first side and a second side, two opposite ends of the first side of each of the at least one first separation module are communicated with the first section and the second section respectively, and the second side of each of the at least one first separation module is communicated with at least one first outlet.

The at least one first separation module 12 is communicated with the enrichment pipeline 11 and contact the fluid to achieve the separation of specific components of the fluid. The intercepted target substance may be a component to be retained in the fluid or a component to be removed from the fluid. That is, the enrichment pipeline 11 can be used to enrich the beneficial components of the fluid or to enrich the components to be removed from the fluid.

In some embodiments, the first separation module 12 is a relatively closed cavity structure; as shown in FIG. 2, two opposite ends of the first side of the first separation module 12 is communicated with the first section 111 and the second section 112 respectively. When the first section 111 and the second section 112 are selectively closed, the first side of the first separation module 12 forms a closed cavity. In other embodiments, when the first outlet communicated with the second side of the first separation module 12 is closed, the second side of the first separation module 12 also forms a closed cavity. After the fluid is introduced into the enrichment pipeline 11 by the first inlet, the fluid will no longer be in contact with the external environment. In some specific situations, such as lipid removal or antibody extraction in medical scenarios, the relatively closed structure can create a sterile environment or reduce infection by bacteria, microorganisms, viruses, etc. The fluid treatment method of the present disclosure reduces disturbance coming from the external environment by forming a relatively closed circulation environment where the fluid cyclically flows in the enrichment line 11 and is processed, thereby increasing the reliability of the treatment process.

The first separation module 12 includes a first separation component, and the first separation component contacts the fluid and selectively allows specific material components of the fluid to permeate through. The first separation component divides the first separation module 12 into two sides so that the fluid passing through the first separation component and the intercepted fluid can be located on two different sides of the first separation component respectively.

The fluid in the enrichment pipeline 11 can flow from the first section 111 to the second section 112 through the first separation module 12 and return to the first section 111 from the outlet of the second section 112, thus forming a cycle. At the same time, the components of the fluid other than the target substance pass through the first separation component in the first separation module 12 to the second side of the first separation module 12, and is discharged from the first outlet, i.e., the above process achieves the separation of the target substance from the fluid.

It should be understood that, in the present disclosure, the "separation effect" indicates a difference between the concentration of at least one component (e.g. filterable components) of the fluid as intercepted in the first side of the first separation module, and the concentration of the at least one component as in the second side of the first separation module, and therefore the "separation effect" used herein does not refer to an absolute separation or removal effect. For example, the components in the fluid located on the second side of the first separation module may also be present in the first side of the first separation module. Similarly, a tiny portion of the target substance may also flow to the second side of the first separation module. But there is at least one component with different concentrations on the two different sides of the first separation module.

The first side of the first separation module 12 and the second side of the first separation module 12 make the position of the component of the fluid that can pass through the first separation component different from the position of the intercepted fluid. The positional relationship of the first side and the second side is determined by the cavity structure of the first separation module 12 and the structure of the first separation component. For example, when the first separation component has a planar structure and is horizontally arranged in the first separation module 12, the first side and the second side refer to an upper side and a lower side of the first separation module 12 respectively; for another example, when the first separation component has a planar structure and is vertically arranged in the first separation module, the first side and the second side refer to a left side and a right side of the first separation module 12 respectively. In another example, when the first separation component is a hollow fiber structure arranged in the first separation module 12, the first side and the second side refer to an inner side and an outer side of the hollow fiber respectively. When a plurality of separation components form a separation module, different second sides corresponding to the separation components may share a cavity.

It should be understood that in the present disclosure, a cavity in the first separation module that is communicated with the first section and the second section of the enrichment pipeline is the first side, and the other cavity or cavities in the first separation module are the second side. In one embodiment, the first separation module includes a plurality of cavities, for example, the first separation module is separated into a plurality of cavities by a plurality of first separation components in the form of a plate membrane, at least one of the cavities that is communicated with the first section and the second section is the first side, and the other cavities are the second side.

The structure or material of the first separation component has a selective permeability to some specific compositions of the fluid, and the first separation component may be a filter, a filter membrane, and a porous metal material, etc.

In one embodiment, the first separation component is a porous membrane, a reverse osmosis membrane, or a gas separation membrane.

In some embodiments, the first separation component is a porous membrane, and the porous membrane includes a microfiltration membrane, an ultrafiltration membrane, or a nanofiltration membrane. Herein, the average pore diameter or molecular weight cutoff (MWCO) of the porous membrane or reverse osmosis membrane is related to the target substance. In actual scenarios, the porous membrane suitable for intercepting the target substance is selected based on the components of the fluid and the target substance. For example, when the size of the target substance to be intercepted in the fluid is 10 nm (i.e., 0.01 µm), the corresponding separation membrane can be a nanofiltration membrane or a reverse osmosis membrane to achieve the interception for the target substance. Herein, the specific type of the separation membrane can be determined based on the difference in physical and chemical properties of each component of the fluid and the target substance. For example, potential separation membranes include: reverse osmosis membrane (with an average pore size of 0.0001-0.001 µm), nanofiltration membrane (with an average pore size of 0.001-0.01 µm), ultrafiltration membrane (with an average pore size of 0.01-0.1 µm), microfiltration membrane (with an average pore size of 0.1-10 µm), electrodialysis membrane, pervaporation membrane, liquid membrane, gas separation membrane, electrode membrane, etc.

In some embodiments, especially in medical applications, the separation membrane is made of high-purity polymer with inactive chemical properties and good blood compatibility and tissue compatibility.

In some embodiments, the separation membrane can intercept, filter out, or exchange the target substance through steric hindrance effect, Donnan effect or electrostatic effect, adsorption, diffusion, charge repulsion effect, pore effect, or dissolution effect. The composition of substances permeable through the separation membrane is related to the type of the separation membrane and the composition of the target substance. In actual scenarios, one or more separation membrane can be set up based on a predetermined composition of the target substance to treat the fluid . For example, when the target substance is a macromolecular pathogenic factor that needs to be removed from blood or plasma, the separation membrane is a porous membrane, whose average pore size is smaller than the molecular particle size of the macromolecular pathogenic factor, so that the macromolecular pathogenic factor is intercepted in the enrichment pipeline. For example, when the target substance is charged ions in a neutral electrolyte fluid, the first separation module is a nanofiltration membrane and achieves filtration based on the characteristics of nanofiltration membranes that selectively transfer ions based on the charge repulsion effect and the pore effect. Furthermore, when the fluid is a mixture of gas, the first separation module is a gas separation membrane that only allows specific gases in the fluid to pass through the gas separation membrane, thereby enabling the separation and interception of different gaseous components.

In some examples, the treatment of the target substance may be determined as interception based on the composition of the fluid and the composition of the target substance, and the average pore size or molecular weight cutoff of the separation membrane suitable for interception or filtration of the target substance may be determined based on the molecular size of the target substance, to set the separation membrane used in actual scenarios. In other examples, the specific type of the separation membrane may also be determined in light of the Dornan effect, adsorption, solubilization, etc. of the aforementioned separation membrane in order to have a membrane in the first separation module that can better achieve the preset separation effect.

In some practical applications, the pore diameter and type of the separation membrane can be determined based on a goal that more than 90% of the target substance can be intercepted, or a higher goal that more than 95% or even more than 99% of the target substance can be intercepted.

In terms of microstructure, the separation membrane can include symmetrical membrane, asymmetrical membrane, composite membrane, multilayer composite membrane, etc.

The first separation component may also be provided with different geometries of separation membranes to accommodate different fluids or to achieve different filtration effects.

In some embodiments, the first separation component includes one or more of a planar membrane, a tubular membrane, a roll membrane, a spiral membrane, and a hollow fiber membrane.

The angle between the flow direction of the fluid and the separation membrane may vary; for example, the angle may be 0°-90°. When the angle between the flow direction of the fluid and the separation membrane is 0°, the fluid flows parallel to the surface of the separation membrane, as in tangential flow filtration; when the angle between the flow direction of the fluid and the separation membrane is 90°, the fluid flows in a vertical direction toward the surface of the membrane, as in dead end filtration (also called vertical filtration).

In some embodiments, a flow channel in the first separation module can be in a folded round-trip form. For example, the contact area between the fluid and the planar membrane is increased by folding the planar membrane, and the flow channel is accordingly folded and in a round-trip form to match the structural shape of the membrane, thereby ensuring that the separation membrane divides the first separation module into the first side and the second side.

In some embodiments, the first separation module is a tangential flow filtration module (TFFM).

It should be understood that in the tangential flow filtration, the separation is driven by the transmembrane pressure difference generated across the membrane. In some embodiments, filtration in the tangential flow filtration module may be achieved using tubular membranes or hollow fiber membranes, in which setting the filtration efficiency can be increased by increasing the contact area between the fluid and the separation membrane.

In some embodiments, the TFFM can use a traditional membrane cassette of tangential flow filtration, such as the Pellicon box cassette with ultrafiltration membrane of Merck & Co Inc; the leucocyte filter, the virus filter, the LEOCEED dialyzer, and the membrane plasma separator of Asahi Kasei; and the plasma separator of Braun GmbH. It should be noted that, the TFFM can be used in different fields to achieve different separation effects, for example, it can be used to achieve material collection (intercepted materials in the enrichment pipeline cyclically flow to be collected), or treating industrial wastewater, etc.; that is, the TFFM can be applied in scenarios other than those mentioned in previous paragraphs. Also, in actual scenarios, the pore size or molecular weight cutoff, as well as the geometry of the membrane in TFFM is determined based on the purpose of filtration or interception of the target substance.

In step S11, the enrichment pipeline may be provided with one or more of the first separation modules, and two opposite ends of the first side of each first separation module are communicated with the enrichment pipeline, which enables the target substance intercepted by the first separation module to flow in the enrichment pipeline. In actual scenarios, the number of the first separation modules can be determined based on the diameter of the enrichment pipeline, the length of the enrichment pipeline, the separation efficiency of the first separation module, the economic cost, and other factors.

In step S12, the fluid in the enrichment pipeline is driven to cyclically flow at a first preset flow rate based on at least one first driving device to enrich the target substance; the at least one first driving device is arranged at the first section and is configured to dynamically balance a total amount of the fluid in the enrichment pipeline.

Herein, the first driving device includes, but is not limited to, a peristaltic pump, a pressure pump, an electric field, a heater, a hydraulic pump, or a vacuum pump, which is used to provide power to the fluid in the enrichment pipeline 11, thereby making the fluid flow in a preset flow direction. Specifically, the fluid flows from the first section 111 to the second section 112 through the first separation module 12 and flows from the exit of the second section 112 to the first section 111, to form a dynamic circulation. In medical scenarios, the first driving device is not in direct contact with the liquid, but only applies pressure to the pipeline and indirectly drives the liquid to flow. The first driving device is a peristaltic pump in some examples.

The preset flow rate may be determined based on the safety of the actual scenario, the separation effect of the first separation module 12, the cost of the device and other factors together. For example, in the treatment involving blood, when the fluid introduced by the first inlet is human blood, the flow rate of the fluid introduced by the first inlet needs to be controlled within a preset range to ensure patient's safety.

The enrichment of the target substance can be achieved as the fluid cyclically flows in the enrichment pipeline 11 at the preset flow rate.

In the embodiments provided in the present disclosure, the total amount of the fluid in the enrichment pipeline 11 is dynamically balanced when the enrichment pipeline 11 treats the fluid in a cycle enrichment mode. In the cycle enrichment mode, the first inlet continuously introduces a to-be-treated fluid into the enrichment pipeline 11, the fluid flows in the preset flow direction in the enrichment pipeline 11, and the first separation module 12 intercepts the target substance in the fluid when the fluid cyclically flows in the enrichment pipeline 11. At the same time, the fluid from which the target substance has been removed through the first separation component is discharged by the first outlet. In above process, the first driving device 13 provides power to make the fluid cyclically flow in the enrichment pipeline, and the continuous introduction of the fluid at the first inlet makes the amount of intercepted target substance gradually increase, so as to realize the enrichment of specific components, i.e., the target substance.

Herein, the first section 111 is provided with the at least one first driving device 13 to dynamically balance the total amount of the fluid in the enrichment pipeline 11.

In accordance with the direction of fluid circulation in the enrichment line 11, the first section 111 is provided upstream of the first separation module 12. The first driving device 13 can be used to control the flow rate of the fluid passing through the first separation module 12. It should be understood that the first driving device 13, which is set at different positions in the enrichment pipeline, can drive the fluid in the enrichment pipeline 11. In the flow direction of the fluid, the flow rate of the fluid may be influenced by factors such as pipeline resistance, temperature, and pressure. In the embodiments provided in the present disclosure, the first driving device is arranged at the first section 111, that is, upstream of the first separation module 12, thereby controlling the flow rate of the fluid at the first separation module 12. Take the first separation component being a separation membrane as an example, the angle between the flow direction of the fluid and the separation membrane can be different angles, such as 0°-90°. The flow rate of the fluid relative to the separation membrane is related to the separation effect. The fluid treatment method of the present disclosure can control the separation effect by changing the position of the first driving device 13. The separation effect includes, but is not limited to, membrane flux, separation rate, removal effect (removal rate) of small molecules, and interception ratio of large molecules. It should be noted that herein "upstream" is used to indicate the flow direction of the fluid between the first section 111 and the first separation module 12, i.e., when the first section 111 is located upstream of the first separation module 12, the flow direction of the fluid is pointing from the first section 111 to the first separation module 12.

In some embodiments, the at least one first driving device 13 controls the flow rate of the first section 111 to be above a preset threshold so that the target substance is able to flow from the outlet of the first section 111 to the inlet of the second section 112.

For example, when the first separation module 12 is a TFFM, the flow direction of the fluid is parallel to the separation membrane in the TFFM. When the fluid passes through the separation membrane in the TFFM, a pressure difference across the membrane is generated and drives small molecules in the fluid to pass through the separation membrane and then reach the second side of the first separation module 12. Small molecules passing through the separation membrane can be discharged from the enrichment pipeline 11 at the first outlet, while the intercepted large molecules, driven by fluid momentum, are washed away from the surface of the membrane and continue to cycle in the enrichment pipeline 11. The pressure difference across the membrane in the TFFM is related to the preset flow rate. Besides, the intercepted target substances require a certain amount of momentum to overcome the polymerization inhibition between macromolecular components, or macromolecular components and the membrane, so as to continue the cycle. The at least one first driving device 13 controls the flow rate of the first section 111 to be above the preset threshold, which can be further used to control the pressure difference across the separation membrane to achieve the separation effect, and can be used to prevent polymerization inhibition to ensure the sustainability of the cycle in the enrichment pipeline 11.

In some embodiments, the preset threshold is determined based on at least one of a composition of the fluid, a temperature of the fluid, a structure of the first separation component, a material of the first separation component 12, a cavity structure of the first separation module, and a diameter of the enrichment pipeline 11.

The flow rate of the fluid at the first separation module 12 is related to several different parameters, for example, the flow rate of the fluid at the first separation module 12 may be changed according to parameters including: characteristics of the fluids such as the density and viscosity of the fluid; a shape of a boundary layer of the fluid at the first separation module 12, such as the surface shape of the separation membrane (i.e. structure of the membrane) and the cavity structure of the first separation module 12; an interaction force between the fluid and the separation membrane, such as the surface roughness of the membrane determined by the membrane material, and an attraction between the fluid and the separation membrane; and a relationship between the flow rate at the first separation module 12 and the flow rate at the enrichment pipeline 11 determined by the cavity structure of the first separation module 11 and the diameter of the enrichment pipeline 11. The preset threshold can be used to determine the flow rate of the fluid at the entrance of the enrichment pipeline 11 when flowing to the first separation module 12, the value of the preset threshold can be used as the initial flow rate at the first separation module 12. According to the initial flow rate and the flow rate of the fluid at the first separation module 12, the flow rate of the flow at the first separation module 12 can be controlled by controlling the preset threshold, resulting in a pressure difference to achieve separation, preventing intercepted macromolecular substances such as the target substance from gathering on the surface of the membrane, thereby, the target substance can flow from the exit of the first section 111 to the entrance of the second section 112.

In some embodiments, the second section is further provided with one of the at least one first driving device.

In the above embodiments, the first driving device arranged at the first section may be used to control the flow rate of the fluid at the first separation module. In other embodiments, the second section is also provided with one of the at least one first driving device, and multiple first driving devices in the enrichment pipeline may cooperatively control the flow rate of the fluid in the enrichment pipeline to determine the flow rate at different positions in the enrichment pipeline. In the above embodiments, the first section and the second section are provided with first driving devices. It should be understood that the pipeline communicating the first inlet with the first separation module may be considered as the first section. In some examples, the first inlet is communicated with the first section and the second section, or the first section is communicated with a first inlet and the second section is communicated with another first inlet. That is, as shown in FIG. 2, the first section 111 and the second section 112 may be determined based on the circulation direction of the fluid in the cycle enrichment mode, i.e., the first section 111 is always located upstream of the first separation module 12, and when the circulation direction in the enrichment pipeline 11 is changed by the first driving device 13, the positions of the first section 111 and the second section 112 are switched accordingly.

In some embodiments, in the fluid treatment method of the present disclosure, a total volume or total velocity of the fluid introduced into the enrichment pipeline from the at least one first inlet is equal to that of a fluid discharged from the enrichment pipeline by the at least one first outlet to cyclically enrich the target substance in a sustainable manner.

It should be understood that when the total volume or total velocity of the fluid introduced into the enrichment pipeline from the at least one first inlet is equal to that of a fluid discharged from the enrichment pipeline at the at least one first outlet, the total volume of the fluid in the enrichment pipeline can be in a dynamic balance. In some examples, the total volume of the fluid in the above description can also be replaced with volumetric flow, the total velocity replaced with average velocity; or a mass flow rate of the fluid at the first inlet and a mass flow rate of the fluid at the first outlet are controlled to be equal.

It should be understood that when operating the cycle enrichment alone, the first separation module will discharge filtered fluid and therefore generate a negative pressure inside the cycle enrichment. The negative pressure inside the cycle enrichment drives the inflow of the fluid at the first inlet, and the driving force varies with the filtration efficiency of the first separation module. If the fluid to be treated flows into the first inlet not at a constant flow rate, but at a dynamic flow rate, or it is a non-dynamic fluid, the cycle enrichment can introduce the fluid to be treated according to its own filtration effect. If the filtration efficiency of the first separation module decreases due to the high concentration of target molecules in the cycle enrichment, the inflow of the fluid to be treated will be reduced in order to passively maintain the above dynamic balance.

The flow rate of the fluid is controlled by the first driving device to ensure the dynamic balance of the total amount of the fluid in the enrichment pipeline. The pressure in the enrichment pipeline stays at a constant value or fluctuates within a range determined by the constant value during the cycle enrichment mode, thereby avoiding problems such as pipeline rupture, negative pressure suction, destruction of fluid components, such as the rupture of red blood cells, etc., caused by drastic pressure changes in the pipeline. Therefore, the cycle enrichment mode is sustainability.

The pressure in the enrichment pipeline is related to the total amount of the fluid in the enrichment pipeline. For example, a continuous decrease of the fluid in the enrichment pipeline can easily lead to a negative pressure in the relatively closed enrichment pipeline. On the contrary, a continuous increase of the fluid in the enrichment pipeline can easily lead to an increase of pressure in the enrichment pipeline. Both negative pressure and excessive pressure have a negative impact on the separation effect of the fluid treatment and the safety of the enrichment pipeline. For medical applications, when blood is used as the fluid, excessive pressure in the enrichment pipeline will lead to a pipeline rupture or a damaged first separation module, thereby leading to failed treatment and even endangering the patient's health. Herein, the first driving device not only can provide power for the fluid to cycle, but also can be used to ensure that the pressure in the enrichment pipeline is in a preset state, or adjust the pressure in the enrichment pipeline to the preset state.

It should be understood that in the embodiments provided in the first aspect of the present disclosure, the first section is provided with at least one first driving device. Besides, the first driving device can be arranged at any pipeline and position in the enrichment pipeline to synergistically control the flow rate of the fluid and overall balance in the enrichment pipeline. In actual scenarios, the number and location of the first driving devices can be determined based on the shape of the enrichment pipeline, the total amount of the fluid, the power of the first driving device, the preset flow rate, etc.

In the fluid treatment method of the present disclosure, the first driving device controls the fluid to cyclically flow in the enrichment pipeline and dynamically balance the total amount of the fluid, and the specific form of the first separation module is determined based on the components of the fluid and the target substance. The fluid treatment method can continuously collect the target substance in the enrichment pipeline, i.e., a suitable solution for continuous treatment of large amounts of harmful macromolecules in Mode B is achieved. The enrichment pipeline can be used both for fluid flow and as containers for enrichment of the target substance. In actual scenarios, such as medical applications, the structure of the equipment for performing the fluid treatment method of the present disclosure can be effectively simplified or the space of the equipment can be reduced, to obtain easy-to-use or portable or wearable simple devices. In addition, the cycle enrichment duration can be determined based on predetermined processing targets of the fluid, such as: the separation effect, the amount of the target material collected, etc., i.e., the number or durations of cycles in the enrichment pipeline can be controlled based on the preset processing targets.

In some embodiments of the first aspect of the present disclosure, an separation efficiency is adjusted by controlling a ratio of the total volume of the fluid introduced into the enrichment pipeline per unit time to a total volume of the fluid in the enrichment pipeline. The volume of the fluid introduced into the cycle per unit time is the flow rate of the fluid to be treated at the inlet of the cycle.

In a balance state, the enrichment pipeline remains full, and the total volume of the fluid in the corresponding enrichment pipeline remains relatively constant. By controlling the flow rate of the fluid to be treated introduced into the enrichment pipeline, an average time of the fluid to be treated stays in the enrichment pipeline can be determined, and a total cycle time of the fluid to be treated in the enrichment pipeline can be determined accordingly. For example, for the enrichment pipeline and the first separation module used to perform the fluid treatment method of the present disclosure, a holding space of the enrichment pipeline in the cycle is of a relatively fixed size, and the smaller the flow rate of the fluid to be treated introduced into the enrichment pipeline is, the longer the cycle time of the fluid to be treated in the enrichment pipeline is, thereby enabling adjustment of the separation effect or efficiency of the first separation module.

In some embodiments of the first aspect of the present disclosure, the fluid treatment device includes at least one treatment module, for performing pretreatment or retreatment on the fluid in the fluid treatment device, and the pretreatment or retreatment includes at least one of filtration, adsorption, heating, catalysis, enrichment, concentration, a chemical treatment, an optical treatment, and an electrical treatment. In practical applications, the treatment modules can be adsorption devices, extraction devices, ion exchange treatment devices, filtration devices, heating devices, etc.

The treatment module pretreats the fluid, i.e., treats an initially obtained fluid in order to introduce the treated fluid into the enrichment pipeline through the first inlet. The treatment module completes the pretreatment by allowing the fluid treated by the treatment module to be introduced into the first inlet of the enrichment pipeline.

The retreatment of the fluid by the treatment module refers to treating the target substance in the enrichment pipeline, or, treating fluid on the second side of the first separation module.

The type of pretreatment or retreatment performed by the treatment module may be determined based on treatment needs. For example, when the components of the fluid to be filtered out are the target substance intercepted in the enrichment pipeline, the treatment module may concentrate the fluid discharged from the first outlet due to the need to purify the beneficial components of the fluid.

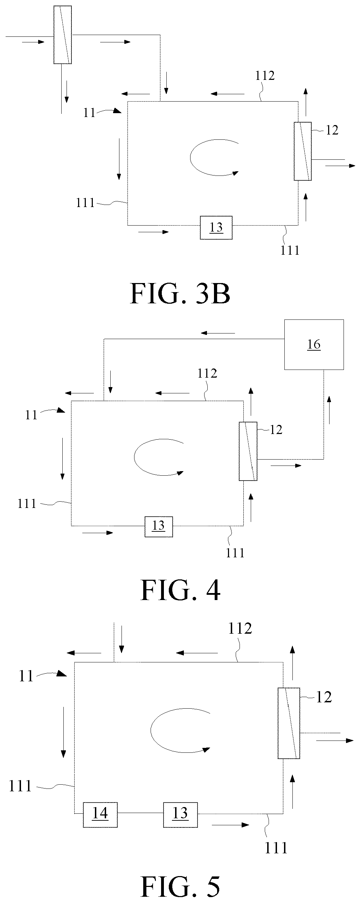

In one example, FIG. 3A shows a schematic diagram of the enrichment pipeline in one embodiment. Referring to FIG. 3A and FIG. 3B. FIG. 3B shows a schematic diagram of the enrichment pipeline in an embodiment in which the treatment module described in FIG. 3A is a separation device.

In an embodiment, as shown in FIG. 3B, the treatment module 30 is a separation device, through which the fluid is pretreated. The separated fluid (containing the target substance) that has passed through the separation device is introduced into the enrichment pipeline for further treatment. The separation device may be used for pretreating the fluid to remove impurities from the fluid by means of precipitation, centrifugal separation, ion exchange, or membrane separation. In an example, the enrichment pipeline 11 treats the fluid to enrich the target substance in the separated fluid. In actual scenarios, such as a plasma exchange scenario, initially obtained whole blood may be separated by the separation device to obtain plasma and cellular components, and a target substance in the plasma may be enriched by the enrichment pipeline 11. When the target substance is a pathogenic factor, the filtered plasma from the second side of the first separation module and the cellular components obtained from the separation of the treatment module are mixed and then returned to the human body, such that the concentration of the pathogenic factor in the plasma of the human blood circulation system is changed during the cycle enrichment process. When the separation device is a porous membrane, the pore size or molecular weight cutoff of the separation membranes of the separation device and the first separation module 12 is determined based on the particle size or molecular weight of the target substance, i.e., the target molecule should be able to pass through a porous membrane into the cycle enrichment, and be intercepted by the porous membrane of the first separation module 12, and enriched in the cycle enrichment. The selection of the pore size of the membranes in this case is similar to that of the DFPP process.

In some examples, in the fluid treatment method provided in the first aspect of the present disclosure, the at least one first inlet and the at least one first outlet are connected to a same storage section; the storage section includes a fluid storage device, a container, or a human body.

Refer to FIG. 4. FIG. 4 shows a schematic diagram of a cycle enrichment module according to an embodiment of the present disclosure. The storage section 16 may be a container or a storage space, as long as it can achieve storage or connection of the fluid introduced into the first inlet or the fluid discharged from the first outlet. In specific application scenarios, the storage section 16 may also be a blood circulation system of human or animal bodies.

For example, in one case, the first inlet and first outlet are connected to the same storage device or container. The first driving device dynamically balances the total amount of the fluid in the enrichment pipeline, and correspondingly, the total amount of the fluid in the storage device or container is dynamically balanced. In the cycle enrichment mode, the fluid from the storage device or container is introduced into the enrichment pipeline to enrich the target substance, the fluid from which the target substance has been removed is returned to the storage device or container through the second side of the first separation module, continuing in the circle of enrichment. The target substance in the fluid in the storage device or container gradually decreases while the total amount of the fluid remains unchanged. The target substance is collected without generating waste fluid, and the total amount of the fluid in the storage device or container remains unchanged, as a result, the cycle of enrichment can be sustained.

In another case, when the storage section is a human body, the fluid can be blood, plasma, serum, tissue fluid or other body fluid of the human body. In this case, the first inlet and the first outlet may be directly or indirectly connected to the human body. The direct connection means that the fluid to the first inlet and from the first outlet may be connected to the circulatory system of the human body; the indirect connection means that a first inlet or/and a first outlet may be connected to a treatment module, and then the treatment module is connected to the circulatory system of the human body. The treatment module may be used, for example, for separating the whole blood of the human body to obtain the plasma, and the first inlet is connected to the obtained plasma. Thus, based on the fluid treatment method of the present disclosure, plasma is introduced into the enrichment pipeline to circulate and enrich pathogenic factor (e.g., low-density lipoprotein) that needs to be filtered out, while the beneficial components in the plasma are continuously separated to the second side of the first separation module and leave the cycle following the circulation process. This process of enrichment of the target substance avoids the generation of waste plasma, effectively solving the problem of continuous loss of beneficial components.

In some embodiments of the first aspect of the present disclosure, the enrichment pipeline further includes an air inlet-outlet, arranged at the first section and/or the second section, for adjusting an air pressure inside the enrichment pipeline.

The air inlet-outlet can be used to connect the enrichment pipeline to the atmosphere or communicate with a gas storage device, and the air inlet-outlet performs gas exchange to adjust the air pressure inside the enrichment pipeline. The air inlet-outlet can be closed or opened selectively: when the fluid in the enrichment pipeline is in the cycle enrichment mode, the air inlet-outlet may be set to a closed state to keep the pipeline in a relatively closed state; in another example, when the first inlet is closed, the enrichment pipeline performs a concentration cycle on the fluid and the total amount of the fluid in the enrichment pipeline is reduced, in which case the air inlet-outlet may be set to open to keep the air pressure in the enrichment pipeline equal to the external atmospheric pressure, thereby avoiding forming a negative pressure inside the system. In another example, in the cycle enrichment mode, the air inlet-outlet may be set to open to communicate with a gas storage device. In actual scenarios, the opened air inlet-outlet can prevent the fluid in the enrichment pipeline from being infected, contaminated, or reacting with gas components in the air during the concentration process, etc. For example, in medical applications, the gas stored in the gas storage device can be controlled to ensure a sterile environment in the enrichment pipeline.

In other examples, the air inlet-outlet is provided with a sterile filter membrane, and the enrichment pipeline and external gases, such as air, are exchanged through the sterile filter membrane, thus ensuring a sterile environment inside the enrichment pipeline in the process of adjusting the pressure inside the enrichment pipeline.

In some embodiments of the first aspect of the present disclosure, the first section and/or the second section is further provided with a channel-adjusting device, for adjusting a flow direction of the fluid in the enrichment pipeline. The channel-adjusting device may be used to switch the operating mode in the enrichment pipeline; for example, the enrichment pipeline can be switched from the cycle enrichment mode to a concentration cycle mode or a cleaning mode.

The channel-adjusting device adjusts the flow direction of the fluid in the enrichment pipeline by at least one of the following ways:
adjusting the flow direction of the fluid in the enrichment pipeline by adjusting a switching state of the at least one first inlet and/or the at least one first outlet; and
adjusting the flow direction of the fluid in the enrichment pipeline by adjusting a switching state of at least one adjusting channel connected to the enrichment pipeline; wherein the switching state includes an open state or a closed state.

In the embodiment of adjusting the flow direction of the fluid in the enrichment pipeline by adjusting a switching state of the at least one first inlet and/or the at least one first outlet, the channel-adjusting device further includes a first pipeline switch arranged at the first inlet and/or the first outlet. In an embodiment, one first pipeline switch is arranged at each of the at least one first inlet, and is for switching the fluid in the enrichment pipeline to the concentration cycle mode when the first pipeline switch is closed. Herein, the pipeline switch, for example, can be pipeline clamp, an on-off valve, a water flow switch with a sensor, etc.

The pipeline switch is used to control the opening or closing of the first inlet. When the first inlet is opened, the to-be-treated fluid is continuously supplemented to the enrichment pipeline and the fluid separated by the first separation module is discharged from the enrichment pipeline, so that the target substance can be enriched in the enrichment pipeline; when the first inlet is closed by the first pipeline switch, the fluid in the enrichment pipeline is driven by the first driving device to cyclically flow, and the treated fluid is discharged passing through the separation module, at which time, the total amount of the fluid decreases, and the concentration of the target substance increases, that is, the fluid in the enrich pipeline is switched to the concentration cycle mode. In actual scenarios, in the case where the target substance needs to be collected, the fluid can be treated to obtain a high concentration of the target substance under the concentration cycle mode. For example, when platelets are the target substance, platelet enrichment can be completed through an enrichment cycle, and the platelets can be concentrated by a concentration cycle without going through a separation process in vitro, which can avoid repeated treatment in vitro and waste of blood. The platelets obtained through the concentration cycle can be used in heart surgery, wound treatment, etc.

In some embodiments, the flow direction of the fluid in the enrichment pipeline is adjusted by adjusting a switching state of at least one adjusting channel connected to the enrichment pipeline. The channel-adjusting device further includes at least one adjusting pipeline and at least one second pipeline switch arranged at the at least one adjusting pipeline so that an entrance of a cleaning solution and an exit of a waste fluid are formed in the enrichment pipeline.

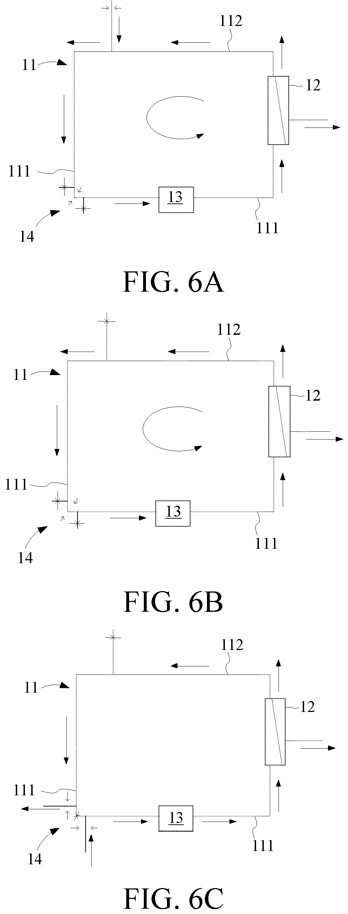

Refer to FIG. 5, and FIGs. 6A-6C. FIGs. 6A-6C show schematic diagrams of the enrichment pipeline 11 and the channel-adjusting device 14 in an embodiment in different cycle modes. In another embodiment, as shown in the figures, the channel-adjusting device 14 includes at least one adjusting pipeline and at least one pipeline switch that are matched with the enrichment pipeline 11; the adjusting pipeline is arranged at the enrichment pipeline 11, and the pipeline switch is arranged at each adjusting pipeline and the enrichment pipeline 11 between two adjusting pipelines.

In some embodiments, the enrichment pipeline is switched to a concentration cycle mode or a dilution mode, to adjust a separation efficiency of the at least one first separation module.

Refer to FIG. 2. FIG. 2 shows a schematic diagram of an enrichment pipeline and a first separation module performing the fluid treatment method according to an embodiment of the present disclosure. In actual scenarios, when the enrichment pipeline 11 stays in the cycle enrichment mode to intercept the target substance, the concentration of the target substance in the enrichment pipeline 11 will increase, which may lead to the reduction of the separation efficiency of the first separation module. For example, when the first separation module is a TFFM, in some optional embodiments, the enrichment pipeline 11 can be a replaceable consumable component, in which case the enrichment pipeline 11 can be replaced when the filtration efficiency drops to a certain extent. In other embodiments, as shown in FIG. 5, the channel-adjusting device 14 adjusts the flow direction of the fluid in the enrichment pipeline in order to achieve the sustainability of the enrichment pipeline 11. Herein, the "flow direction" of the fluid is used in a general sense, and includes not only the commonly-understood cycling direction of the fluid but also flow channels of the fluid in the enrichment pipeline 11. It should be understood that the channel-adjusting device 14 is provided at the first section in the example shown in FIG. 5, and in actual scenarios, the channel-adjusting device 14 may also be provided at the second section, or the channel-adjusting device 14 may be provided at both the first and second sections.

Referring to FIGs. 6A-6C, the cycling direction and flow channel of the fluid in the enrichment pipeline 11 can be changed by controlling the pipeline switches in the channel-adjusting device 14. For example, as shown in FIG. 6A, when the pipeline switch arranged at the adjusting pipeline is turned off and the pipeline switch arranged at the enrichment pipeline 11 is turned on, the fluid in the enrichment pipeline 11 flows in accordance with the flow channel of the enrichment pipeline 11, which enables the enrichment pipeline to cyclically enrich the target substance. As shown in FIG. 6B, when the amount of the target substance intercepted in the enrichment pipeline 11 has reached a preset target, the pipeline switch of the first inlet can be turned off to make the fluid in the enrichment pipeline 11 be in the concentration cycle mode. It should be noted that in actual scenarios, the operation of concentrating and cycling the target substance obtained by enrichment is optional, and when a molecular concentration in the fluid has reached a preset value or the separation effect of small molecular substances in the enrichment pipeline 11 has reached the preset target, the operation of concentrating and cycling can be omitted. As shown in FIG. 6C, when the molecular concentration in the enrichment pipeline 11 reaches the present value or starts to deteriorate the filtration effect, the pipeline switch arranged at the adjusting pipeline is turned on and the pipeline switch arranged at the enrichment pipeline 11 is turned off, the pipeline switch arranged at the first inlet is turned off, one end of each of the two adjusting pipelines is communicated with the enrichment pipeline 11, the other end of each of the two adjusting pipelines can serve as the entrance or exit of the enrichment pipeline 11, respectively, and the two adjusting pipelines cooperate with the enrichment pipeline 11 to form a new flow channel, where the fluid obtained by cycle enrichment or concentration cycle in the enrichment pipeline 11 can be discharged from the enrichment pipeline 11 through the entrance and exit. The cleaning solution can also be introduced into the enrichment pipeline 11 to switch the operation mode of the enrichment pipeline 11 to a cleaning mode. The cleaning solution can enter the enrichment pipeline 11 and the first separation module 12 through the entrance, and flow to the exit to clean internal walls of the enrichment pipeline 11 and the first separation module 12, so that the target substance intercepted in the enrichment pipeline 11 and the first separation module 12 is removed after cleaning, and as a result, the enrichment pipeline 11 and the first separation module 12 can continue to be used for cyclic enrichment for the target substance in the fluid, and the channel-adjusting device 14 can be accordingly set to a state shown in FIG. 6A to repeat the fluid treatment.

The cleaning solution is a solution without the target substance, and used to remove the target substance in the enrichment pipeline 11 and the first separation module 12. In application fields such as industrial wastewater treatment, the cleaning solution can contain a surfactant or disinfectant; in medical applications, the cleaning solution includes, for example, a physiological buffer that can participate in human blood cycle, such as a physiological saline, a phosphate buffered saline (PBS), etc.

In some embodiments, the channel-adjusting device 14 is a four-way valve. In specific implementations, the four-way valve may be a four-way rotary valve.

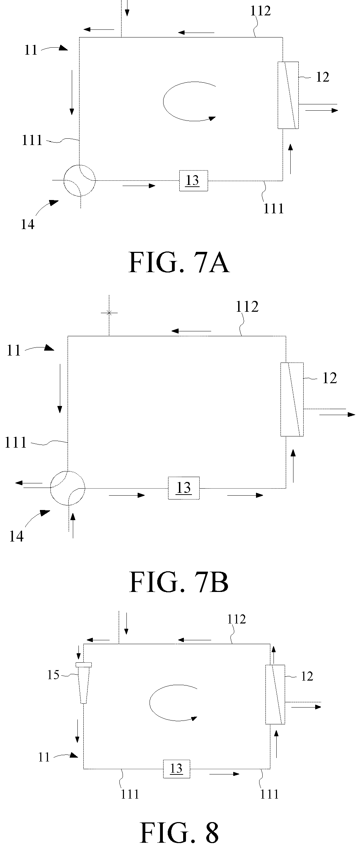

Referring to FIGs. 7A-7B, the figures show schematic diagrams of the enrichment pipeline 11 and the channel-adjusting device 14 in different cycle modes according to an embodiment of the present disclosure. As shown in the figures, the enrichment pipeline 11 is provided with a four-way valve, and the flow direction of the fluid in the enrichment pipeline 11 can be controlled by changing the connection status of different pipelines in the four-way valve. The adjustment process performed by the four-way valve is similar to that of the embodiment depicted in FIGs. 6A-6C. In some examples, the different operating modes in the enrichment pipeline 11 can be adjusted or switched by rotating the four-way valve.

In practical applications, structural parameters of the four-way valve can be determined based on the pipeline parameters of the enrichment pipeline 11, and the position of the four-way valve in the enrichment pipeline 11. For example, the four-way valve can be set at a corner of the enrichment pipeline 11 as shown in the figures, or can be set in a straight portion of a pipeline, and the internal structure of the four-way valve may be different according to the exact position of the four-way valve. In some embodiments, the four-way valve can be obtained by 3D printing.

In medical applications, in order to minimize the amount of small molecular components in the concentrated target substance, an entrance for the cleaning solution in the channel-adjusting device 14 is turned on after concentration, the physiological saline or PBS is introduced into the cycle to dilute the concentrated solution, and then the entrance of the cleaning solution is turned off to perform the concentration cycle again. After repeating the above operations for several times, most of the small molecular components remaining in the concentrated solution of the target substance can be removed. For example, the cycle enrichment directly enriches red blood cells in the donor's blood and removes most of proteins and small molecules through multiple above-mentioned concentration and dilution processes, reducing the loss of proteins and small molecules and also effectively reducing the impact of the donor's allergic and pathogenic factors on the patient. After cleansing, the red blood cells can be then fed directly into the patient via the channel-adjusting device 14, avoiding contact with the outside world.

In some examples, the enrichment pipeline further includes at least one collection device, arranged at the first section and/or the second section, for adjusting a volume of the enrichment pipeline 11 to collect the target substance, or bubbles.

Refer to FIG. 8. FIG. 8 shows a flowchart of the enrichment pipeline 11 performing the fluid treatment method according to an embodiment of the present disclosure.

It should be understood that in the fluid treatment method of the present disclosure, when the fluid flows in the enrichment pipeline 11, the enrichment pipeline 11 enriches the target substance in the cycle enrichment mode, and therefore, an inner cavity of the enrichment pipeline 11 can also serve as a holding space for the target substance. In some examples, the enrichment pipeline 11 is provided with the collection device 15 to increase the internal volume of the enrichment pipeline 11, which is for collecting more of the target substance. The collection device 15 can be a container or storage space, such as a collecting cylinder shown in FIG. 8. Of course, the number, location, and structure of the collection device 15 can be determined based on actual needs.

In one embodiment, the location of the collection device 15 in the enrichment pipeline 11 depends on the location of the first separation module 12; for example, in the embodiment shown in FIG. 8, the collection device 15 is located upstream of the first separation module 12, and in the concentration cycle mode, the fluid in the collection device 15 tends to flow in the direction of the cycle due to gravity . Usually, the air inlet-outlet is open in the concentration cycle mode to avoid or reduce air bubbles in the cycle. In another embodiment, the location of the collection device 15 can be determined based on the location of an exit of the adjusting pipeline of the channel-adjusting device; for example, by setting the collection device 15 above the exit of the adjusting pipeline, the fluid in the collection device 15 will tend to flow to the exit of the adjusting pipeline when the enrichment pipeline 11 is placed naturally.

In some examples, the collection device 15 is provide with the air inlet-outlet. For example, the air inlet-outlet is arranged at an upper part of the collection device 15, to communicate the space in the collection device 15 not filled with the fluid with the atmosphere or an external gas storage device. In some examples, the collection device 15 can also be provide with an inlet and outlet of the adjusting pipeline of the channel-adjusting device; therefore, the channel-adjusting device can be integrated into the collection device 15 to form a module.

In some embodiments of the present disclosure, the enrichment pipeline may further include at least one of a control device, a storage device, a pressure detection device, a temperature detection device, a temperature control device, a bubble detection and elimination device, an alarm device, and a concentration detection device.

The control device may be used to control the first driving device to determine the flow rate of the fluid in the enrichment pipeline, or to control the pipeline switch of the first inlet to determine the cycle mode of the fluid treatment in the enrichment pipeline, or to control the channel-adjusting device to determine the flow direction of the fluid; furthermore, the control device may also be used to receive detected parameters of the fluid in the enrichment pipeline in different cycle modes in order to better adjust control parameters of the control device. In this way, the enrichment pipeline and the first separation module can form a negative feedback system. For example, the control device controls the temperature control device to perform heating or cooling based on temperature of the fluid detected by the temperature detection device. In some examples, the temperature control device may also be a thermostat device.

The storage device may be used to store the fluid (i.e., the fluid after separation by the first separation module) on the second side of the first separation module in the enrichment pipeline. In some examples, the storage device may also be connected to a treatment module.

The bubble detection device can obtain a control signal. For example, during a concentration cycle, bubble detection can reflect whether the collection device is close to emptying, and if so, that will mean that the concentrated target substance can be diluted or discharged. The bubble detection device can obtain an early warning signal indicating that the equipment is not in a proper working state, for example, excessive emptying of liquid within the cycle, incorrect placement of the collection device, liquid to be treated containing bubbles, or leakage of liquid, etc.

In an example, the control device determines the internal state of the enrichment pipeline based on the detected parameters and controls the control signal controlling the alarm device. In some embodiments, there are multiple control signals which correspond to different alarm types; for example, when the control device determines that the pressure inside the pipeline is abnormal based on a detected value of the pressure detection device, the control signal a is triggered to cause the alarm device to issue an alarm corresponding to the abnormal pressure; when the control device determines that the concentration inside the enrichment pipeline reaches a preset value (which makes it impossible to continue the cycle) or the concentration is abnormal based on a detected value of the concentration detection device, a control signal b is triggered to cause the alarm device to issue an alarm corresponding to the abnormal concentration.

In some embodiments, the enrichment pipeline also includes a flow rate detection device for detecting a dynamic state inside the enrichment pipeline to generate information for adjusting an internal working state of the enrichment pipeline.

The flow rate detection device detects at least one of the flow rate at the inlet, the flow rate at the outlet, and the flow rate at the separation module, to determine the dynamic state inside the pipeline. In an example, whether the fluid inside the pipeline is in a weak dynamic state can be determined through the flow rate detection device. For example, the weak dynamic state may be a state that the flow rate of the fluid is lower than a preset range or the flow rate of the filtered fluid at the outlet is dropping significantly, based on which the working state of the first separation module can be further determined, such as whether there is molecular obstruction, blockage, etc. in the first separation component. The flow rate detection device can thus generate adjustment information of the internal working state of the pipeline.

The adjustment information may be working-mode switching information for switching the mode of the enrichment pipeline to be the concentration cycle mode or the cleaning mode, or information for adjusting the flow rate in different areas of the enrichment pipeline.

In actual scenarios, the control device receives the flow rate signal to generate the adjustment information. The control device controls the channel-adjusting device and the driving device in the pipeline based on the adjustment information to adjust the internal working state of the pipeline.

In some embodiments, the adjustment information is generated by the control device based on at least one of detected values of the flow rate detection device, the pressure detection device, and the concentration detection device. For example, the control device generates comprehensive adjustment information after receiving at least one of the flow rate information, the pressure information, and the fluid concentration information in the pipeline to control the channel-adjusting device, the driving device, etc.

In some embodiments of the first aspect of the present disclosure, N enrichment pipelines are cascaded to further enrich the target substance, and the N enrichment pipelines includes a first-stage enrichment pipeline and a second-stage enrichment pipeline adjacent to the first-stage enrichment pipeline, and the fluid treatment method further includes: introducing a fluid discharged from at least one first outlet of the first-stage enrichment pipeline into at least one first inlet of the second-stage enrichment pipeline, where N is a positive integer not less than 2 (N ≥ 2).

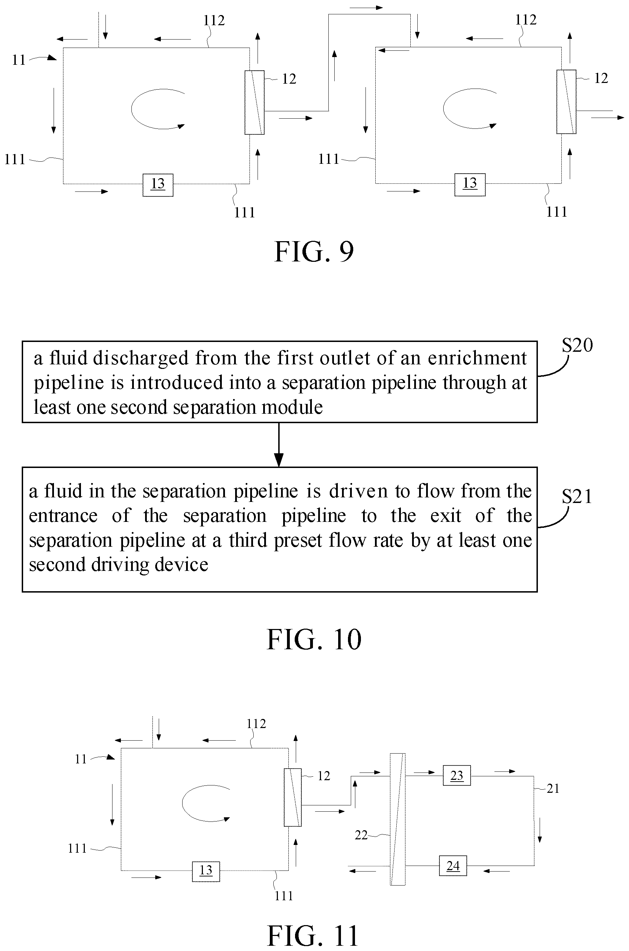

Refer to FIG.9. FIG.9 is a schematic diagram of two cascaded enrichment pipelines according to an embodiment of the present disclosure.

The terms "first enrichment pipeline" and "second enrichment pipeline" are used to describe any two adjacent enrichment pipelines of the N enrichment pipelines connected in series; "first" and "second" denote the connection order, and the connection order is distinguished based on the flow direction of the fluid through the enrichment pipelines, that is, the fluid always flows from the first enrichment pipeline into the second enrichment pipeline for retreatment, and therefore the fluid always flows from the first outlet of the first enrichment pipeline to the first inlet of the second enrichment pipeline.

By connecting the N enrichment pipelines in series, N cycle treatments are performed on the fluid in accordance with the connection order; for a cycle treatment corresponding to each enrichment pipeline, flow rates and flow volumes of the fluid at its first inlet and first outlet are equal in the cycle enrichment mode, i.e., the N cascaded enrichment pipelines are in a dynamic balance in the cycle enrichment mode, so that a continuous cycle of enrichment can be carried out in each enrichment pipeline. Therefore, the enrichment process is sustainable. It should be noted that target substances are sequentially intercepted in the cascaded enrichment pipelines in accordance with the connection order. Therefore, the composition of the fluid introduced at the first inlet of each enrichment pipeline is unique, and correspondingly the target substances intercepted in different enrichment pipelines can be different.

Herein, in each of the examples provided by the present disclosure, compositions of fluids may differ from each other in that they include different categories of components, and/or that concentrations or amounts of one or more components thereof are different.

Herein, the present disclosure provides embodiments for treating fluids by using N cascaded enrichment pipelines, which can be used to intercept components with different particle sizes, molecular weights, or chemical properties, or molecules with different charge properties in fluids, respectively; by providing different first separation modules, the enrichment of different components under the fine-grained classification is achieved based on the selective permeability of the different first separation modules to components in the fluids.

In some examples, the fluid obtained by each of the N cascaded enrichment pipelines can be discharged from the corresponding enrichment pipeline from the exit of the adjusting pipeline of the channel-adjusting device, or by the treatment module, so that a fine-grained target substance can be targeted for extraction or treating, thereby changing the concentration of the target substance in the fluid under the fine-grained classification. For example, when the molecular weight of each component in the fluid is in the range of 200 to 2000 Dalton and the molecular weight of the target substance to be extracted or treated is 1000 to 1100 Dalton, take N=2 as an example and referring to the embodiment shown in FIG. 9, in accordance with the connection order, the first separation module 12 in the first enrichment pipeline 11 intercepts components in the fluid with a molecular weight of above 1100 Dalton, and then the first separation module 12 in the second enrichment pipeline 11 intercepts components with a molecular weight of below 1000 Dalton, the separated fluid in the second enrichment pipeline therefore includes components with a molecular weight ranging from 1000 to 1100 Dalton.

Of course, in actual scenarios, the number of the cascaded enrichment pipelines may be any positive integer not less than 2 (N ≥ 2); correspondingly, independent enriching, extracting, or treating may be performed on each of multiple components of the fluid or each of multiple classification segments of the components. The classification segments may be obtained by classifying components of the fluid based on at least one of chemical properties, molecular weights or molecular particle sizes, and charge properties of the components. It should be understood that the classification segments may be obtained by various classification criteria, and each classification segment can be obtained by using a corresponding first separation component so that materials falling within the classification segment are enriched. Take the first separation components being separation membranes as an example, since different types of separation membranes have different selective permeability, the components of the fluid can be classified or intercepted for N times by N separation membranes.

For example, when the N cascaded enrichment pipelines treat the fluid, large molecule components may be first enriched at the first enrichment pipeline based on molecular weights; based on chemical properties of the fluid, the second enrichment pipeline enriches components that are not selectively permeable to the first separation module of the second enrichment pipeline; then based on charge properties, charged ions are enriched by the third enrichment pipeline. In actual scenarios, for example in a medical application, the N cascaded enrichment pipelines can be used, for example, to separate antibiotics, amino acids, enzymes, other proteins, etc. from the fluid.

It should be understood that, in general, categories of components of the fluid are limited, and by determining the connection order, the first separation module of each enrichment pipeline only intercepts one component of the fluid. It is also understood that only one component of the fluid is contained in one of the classification segments, the N cascaded enrichment pipelines can enrich any particular component of the fluid, remove any particular component of the fluid, change the concentration of any particular component of the fluid, or perform retreatment, such as, filtration, adsorption, heating, catalysis, enrichment, concentration, chemical treatment, optical treatment, electrical treatment, etc. on any particular component of the fluid.

In some embodiments, a molecular size or a molecular weight of the target substance, or particle sizes of components of the target substance as enriched by each of the N cascaded enrichment pipelines, decreases as enrichment sequentially proceeds in the N cascaded enrichment pipelines. In the above example, the components of the fluid are classified according to molecular size or molecular weight; the selective permeability of the first separation modules corresponding to the N cascaded enrichment pipelines to the components of the fluid can be designed based on the particle sizes or molecular weights of the components of the fluid; for example, average pore sizes of the separation membranes in the N first separation modules corresponding to the N cascaded enrichment pipelines decrease in sequence, or molecular weights of intercepted molecules decrease in sequence, and correspondingly, molecular sizes or molecular weights of the target substances in the enrichment pipelines decrease in sequence. In this case (where each enrichment pipeline only intercepts one component of the fluid), the enrichment of any component of the fluid can be achieved by controlling the difference in particle size or molecular weight cutoff between the separation membranes of the first enrichment pipeline and the second enrichment pipeline. In actual scenarios, the smaller the difference in particle size or molecular weight cutoff between the separation membranes is, the more favorable it is to reduce the concentration of interfering molecules, but there is the possibility of also reducing the enrichment efficiency of target substances. When determining or selecting first separation modules of the N cascaded enrichment pipelines, the composition of the fluid, the target substance, interfering molecules, and enrichment efficiency can also be taken into consideration.

For example, in medical applications, compared to the existing DFPP technology, which is only used to filter out large molecules of pathogenic factors from plasma, the N cascaded enrichment pipelines can be used to classify the different components of plasma on the basis of particle sizes or molecular weights and enrich components of different categories of particle sizes or molecular weights in different enrichment pipelines, so that the different components can be collected in a targeted manner, and therefore the scope of clinical indications for the N cascaded enrichment pipelines can be extended, for example, both small molecule pathogenic factors and multiple pathogenic factors can be enriched and treated; at the same time, the N cascaded enrichment pipelines are sustainable and do not produce waste liquid during the treatment, and only the pathogenic factors in the plasma are extracted and processed in a targeted manner, and the problem of continuous loss of beneficial components in the separation can be effectively solved in the treatment. The duration of the cycle can be determined based on a predetermined therapeutic goal such as reducing the concentration of a particular component such as a pathogenic factor below a predetermined value, i.e., the fluid treatment method can control the removal effect regarding the pathogenic factor and can determine the treatment time accordingly, solving the problem of difficulty in controlling the removal rate.

Refer to FIG. 1 and FIG. 10. FIG. 10 shows a flowchart of a fluid treatment method according to an embodiment of the present disclosure. In some embodiments of the first aspect of the present disclosure, the fluid treatment method further includes:

In step S20, a fluid discharged from the first outlet of one enrichment pipeline is introduced into a separation pipeline through at least one second separation module, the separation pipeline has an entrance and an exit, each of the at least one second separation module includes a second separation component to divide a corresponding first separation module into a first side and a second side, two opposite ends of the first side of each of the at least one second separation module are communicated with at least one second inlet and at least one second outlet respectively, and two opposite ends of the second side of each of the at least one second separation module are communicated with the entrance and exit of the separation pipeline.

In step S21, a fluid in the separation pipeline is driven to flow from the entrance of the separation pipeline to the exit of the separation pipeline at a second preset flow rate by at least one second driving device, to dynamically balance a total amount of the fluid in the separation pipeline.

The first aspect of the present disclosure also provides embodiments in which at least one enrichment pipeline is cascaded with a separation pipeline.

Specifically, in one embodiment, one enrichment pipeline is followed by one separation pipeline, and the fluid is discharged from the first outlet of the enrichment pipeline to the second inlet of the separation pipeline; in another embodiment, N cascaded enrichment pipelines are followed by one separation pipeline, the fluid is treated by the N cascaded enrichment pipelines, and the first outlet of the last enrichment pipeline of the N cascaded enrichment pipelines is connected to the second inlet of the separation pipeline according to the connection order, where N is a positive integer not less than 2 (N ≥ 2).

**FIG.** 11 shows a simplified diagram of cascaded enrichment pipeline and separation pipeline according to an embodiment of the present disclosure.

The fluid in the separation pipeline 21 is the separated fluid passing through the second separation module 22, and the entrance and exit of the separation pipeline 21 are respectively connected to two opposite ends of the second side of the second separation module 22 to form a reversible cycle separation. The reversible cycle separation means that the separated fluid in the separation pipeline 21 can return to the first side of the second separation module 22 according to the direction of cycle. When the separated fluid is the target substance, the target substance can be quantitatively collected based on the separation pipeline 21, for example, active ingredients of Chinese medicine may be extracted from a medicinal solution, and the collected target substance cyclically flows in the separation pipeline 21. At the same time, target substances exceeding a predetermined collection or treatment volume may return to the first side of the second separation module 22 based on the reversible cycle.

By means of the reversible cycle separation formed by the separation pipeline 21, the fluid treatment method can be used to achieve treatment of specific components of the fluid. For example, by introducing a fluid containing components a, b, and c into the enrichment pipeline 11 and enriching component a in the enrichment pipeline 11, the fluid components b and c discharged from the first outlet are transferred to the second separation module 22 corresponding to the separation pipeline 21, the component b is intercepted to the first side of the second separation module 22 by the second separation component of the second separation module 22, and the fluid in the separation pipeline 21 is a fluid containing only component c. The treatment module 24 can treat the fluid in the separation pipeline 21, so that only the specific component c in the fluid can be treated.

Based on the second separation module 22 and separation pipeline 21, part of the components of the fluid discharged from the first outlet can be intercepted at the second separation module 22, so that the separated fluid cyclically flowing in the separation pipeline 21 contains only a specific component. The total fluid volume in the separation pipeline 21 is dynamically balanced, i.e., the cycle separation can be continuously performed.

The separation pipeline and the second separation module may form different cycle paths, for example, the cycle path has a rectangular shape in FIG. 11, and in other examples, the cycle paths may include multiple fold lines to form a convex shape or arc lines (such as round arcs). In practical applications, fluid flows and is transferred in the separation pipeline, the diameter, material, and path of the separation pipeline may be adjusted according to the needs of the equipment in different scenarios.

In actual scenarios, the material of the separation pipeline can be a special material corresponding to the fluid, for example, the separation pipeline may be a special blood pipeline, a special peristaltic pump pipeline, a disposable hemodialysis blood pipe, a high-pressure sterilized silicone pipe, a special corrosive liquid pipeline, a high biocompatible pipeline, etc. When the fluid treatment method is applied in the medical scene, take the pipeline such as a blood delivery pipeline or a liquid medicine delivery pipeline as an example, the pipeline materials include, but are not limited to, soft polyvinyl chloride plastic, high-performance polyolefin thermoplastic elastomer (TPE), nano biomedical materials, and resin materials.

The second separation module 22 has a first side and a second side separated by a second separation component, the opposite ends of the first side of the second separation module are communicated with at least one second inlet and at least one second outlet, respectively. The second separation module can be used to separate specific components of the fluid discharged from the first outlet of the enrichment pipeline 11. The separated specific component is the separated fluid reaching the second side of the second separation module, and the separated fluid may be a fluid component to be retained or a component to be removed from the fluid.

In some embodiments of the present disclosure, the second separation module has a relatively closed chamber structure, i.e., the second inlet and the second outlet are respectively communicated with two ends of the first side of the second separation module, and when the second inlet and the second outlet are selectively closed, the first side of the second separation module forms a closed cavity. In some embodiments, when the entrance and exit of the separation pipeline communicated with the second side of the second separation module are closed, the second side of the second separation module also forms a closed cavity. The fluid from the first outlet is transferred to the second inlet of the second separation module and enters the separation pipeline, during which contact with the outside space can be avoided, and in some specific situations such as blood delivery in medical scenarios, the relatively closed structure can form a sterile environment or reduce infection by bacteria, microorganisms, viruses, etc.

The second separation module includes a second separation component, the second separation component is accessible to the fluid and selectively enables specific material components of the fluid to permeate. The second separation component divides the second separation module and allows the fluid flowing through the second separation component to enter the second side of the second separation module.

Based on the second separation module 22, the fluid discharged from the first outlet of the enrichment pipeline 11 can flow from the entrance of the separation pipeline connected to the second side of the second separation module to the exit of the separation pipeline, thus forming a cycle.

The first side and the second side of the second separation module distinguish the position of the fluid that passes through the separation component from the position of the intercepted fluid. The relationship of the position of the first side and the second side is determined by the cavity structure of the second separation module and the structure of the second separation component. For example, when the second separation component has a planar structure and is horizontally arranged in the second separation module, the first side and the second side refer to the upper side and lower side respectively. For another example, when the second separation component has a planar structure and is vertically arranged in the second separation module, the first side and the second side refer to the left side and right side respectively.

The structure or material of the second separation component has a selective permeability to some specific components of the fluid discharged from the first outlet of the enrichment pipeline, such as a filter, a filter membrane, and a porous metal material.

**In** some embodiments, the second separation component includes a separation membrane. The specific type of the separation membrane can be determined based on the difference in physical and chemical properties between each component of the fluid and the target substance. For example, the type of the separation membrane includes: reverse osmosis membrane, nanofiltration membrane, ultrafiltration membrane, microfiltration membrane, electrodialysis membrane, pervaporation membrane, liquid membrane, gas separation membrane, electrode membrane, etc.

In some embodiments, especially in medical applications, the separation membrane is a high-purity polymer with inactive chemical properties and good blood compatibility and tissue compatibility.

In an embodiment, the second separation component may be provided as a porous membrane, a reverse osmosis membrane, or a gas separation membrane.

The average pore diameter or molecular weight cutoff (MWCO) of the porous membrane or the reverse osmosis membrane is related to the target substance. The porous membrane includes a microfiltration membrane, an ultrafiltration membrane, or a nanofiltration membrane. In practical scenarios, the membrane suitable for intercepting or filtering the target substance is selected based on the component in the fluid and the target substance. For example, when the size of the target substance to be intercepted in the fluid is 10 nm (i.e. 0.01 µm), the corresponding separation membrane can be a nanofiltration membrane or reverse osmosis membrane to achieve the interception for the target substance.

In some embodiments, the separation membrane can intercept, filter or exchange the target substances in the fluid through steric hindrance effect, Donnan effect or electrostatic effect, adsorption, diffusion, charge repulsion effect, pore effect, or dissolution effect. A component selectively permeated by the separation membrane is related to the type of the separation membrane and a component of the target substance. In practical scenarios, a corresponding separation membrane can be set up to treat the fluid based on a predetermined component of the target substance. For example, when the target substance is a large molecule pathogenic factor in blood or plasma , it is determined that the separation membrane is, for example, a porous membrane with an average pore size smaller than the molecular size of the pathogenic factor, thereby intercepting the large molecule pathogenic factor on the first side of the second separation module. When the target substance is a charged ion in a neutral electrolyte fluid, the second separation component may be a nanofiltration membrane for filtration based on a characteristic that nanofiltration membranes selectively transfer ions according to the charge repulsion effect and the pore effect. Further, when the fluid is a mixture of gases, correspondingly, the second separation component may be a gas separation membrane to allow specific gases in the fluid to pass through the gas separation membrane, thereby achieving separation of different gas components.

In some examples, the treatment of the target substance may first be determined to be interception or filtration based on the fluid components and the target substance components, the average pore size or molecular weight cutoff of the separation membrane suitable for interception or filtration of the target substance is then determined based on the molecular size of the target substance, thus selecting the separation membrane to be used in actual scenarios. In some other examples, when determining the specific type of the separation membrane, the Donnan effect, adsorption, and dissolution effect of the separation membrane can also be considered, thereby setting a suitable separation membrane in the second separation module to achieve a preset interception and separation effect.

In actual scenarios, the pore diameter and type of the separation membrane can be determined based on a goal that more than 90% of the target substance can be intercepted, or a higher goal that more than 95% or even more than 99% of the target substance can be intercepted.

**In** terms of microstructure, the separation membrane may include, for example, a symmetric membrane, an asymmetric membrane, a composite membrane, a multilayer composite membrane, etc.

The second separation component may be a separation membrane with different geometries to adapt to different fluids or to achieve different filtration effects.

In some embodiments, the second separation component includes one or more of a planar membrane, a tubular membrane, a roll membrane, a spiral membrane, and a hollow fiber membrane.

The relationship of position between the first side and the second side of the second separation module may be different based on the corresponding separation membranes with different geometric shapes of the second separation component. For example, when the second separation component is a planar membrane, the first side and the second side refer to two opposite sides separated by a planar structure. As another example, when the second separation component is a hollow fiber membrane, the first side refers to the inside of the wall of each fiber membrane, and the second side refers to the outside of the wall of each fiber membrane.

The flow angle of the fluid relative to the separation membrane may be set at different angles such as ranging from 0° to 90°. When the fluid flows at an angle of 0° relative to the separation membrane, i.e. the fluid flows parallel to the surface of the separation membrane, it is refer to as a common tangential flow filtration. When the fluid flows at an angle of 90° relative to the separation membrane, i.e., the fluid flows in a direction perpendicular to the surface of the separation membrane, it is refer to as a conventional dead-end filtration (also referred to as vertical filtration).

In some embodiments, the flow channel in the second separation module may be set in a folded round-trip form, for example, by folding a planar membrane to increase the contact surface area between the fluid and the planar membrane, correspondingly, the flow channel is also in a folded round-trip form matching the structure of the separation membrane to ensure that the separation membrane separates the second separation module into the first side and the second side.

In some embodiments, the second separation module is a tangential flow filtration module.

The principle of the tangential flow filtration module to achieve separation and the type of tangential flow filtration module that can be configured in the separation pipeline can be referred to the previous embodiment, i.e. the embodiment in which the first separation module is set as the tangential flow filtration module, which will not be repeated here.

In step S21, the fluid is driven to flow from the entrance to the exit of the separation pipeline at a predetermined flow rate based on at least one second driving device, so that the total amount of fluid in the separation pipeline is controlled in the dynamic balance.

**As** shown in FIG. 11, the second driving device 23 includes, but is not limited to, a peristaltic pump, a pressure pump, an electric field, a heater, a hydraulic pump, or a vacuum pump for powering the fluid in the enrichment pipeline so that the fluid flows in a predetermined flow direction, i.e., from the entrance to the exit of the separation pipeline 21, to form a dynamic cycle.

In the embodiments provided in the present disclosure, the separation pipeline 21 is dynamically balanced in terms of the total amount of fluid in the separation pipeline 21 when the fluid is treated in a cycle separation mode. The cycle separation mode means that the second inlet keeps introducing fluid to the second separation module 22, so that the specific components of the fluid flow through the second separation component into the separation pipeline 21 connected to the second side of the second separation module 22 and the specific components flow from the entrance of the separation pipeline 21 to the second side of the second separation module 22 connected to the exit of the separation pipeline 21 to form a cycle, at the same time, the second outlet on the first side of the second separation module 22 discharges the fluid out of the second separation module 22. In a specific implementation, the balance of the total amount of fluid in the separation pipeline 21 is achieved by controlling a first flow rate of introducing fluid from the second inlet to the first side of the second separation module 22 to be the same as a second flow rate of discharging fluid from the second outlet to the first side of the second separation module 22.

It should be understood that the second driving device 23 can achieve the driving effect of the fluid in the pipeline at different positions in the separation pipeline 21, and the flow rate of the fluid in its flowing direction may change due to pipe resistance, temperature, pressure and other factors. In some examples, the separation pipeline 21 can be provided with multiple second driving devices 23 to control the flow rates at different positions in the separation pipeline 21 to preset values.

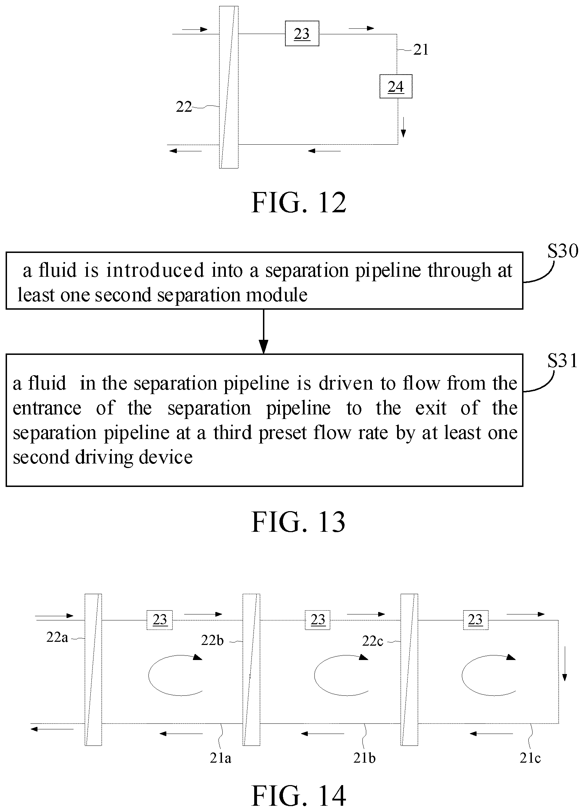

FIG. 12 shows a simplified schematic diagram of a separation pipeline according to an embodiment of the present disclosure. In some examples, the separation pipeline may also be connected to a treatment module 24 to retreat the separated fluid in the separation pipeline or the fluid discharged from the second outlet. The components of the separated fluid can be controlled based on the second separation module, i.e. the fluid is transferred to a second separation module after the target substance is intercepted in the enrichment pipeline by a first separation module, and specific components enter the separation pipeline through the second separation module. The separation pipeline can be used both to change the concentrations of specific components in the fluid after the target substance is intercepted by the first separation module or to allow the treatment module to retreat the specific components. The retreating includes, but is not limited to, at least one of filtration, adsorption, heating, catalysis, enrichment, concentration, chemical treatment, optical treatment, and electrical treatment.

In some embodiments of the first aspect of the present disclosure, the treatment module 24 may be provided in the separation pipeline to regulate the flow direction or flow channel of the separated fluid, i.e., the treatment module 24 is also a channel-adjusting device.

**In** an example, the channel-adjusting device may be provided as adjusting pipelines and pipeline switches in cooperation with the separation pipeline. The adjusting pipelines each is provided in the separation pipeline and the pipeline switches are provided in each of the adjusting pipelines and in the separation pipeline between the adjusting pipelines.

By controlling the pipeline switches in the channel-adjusting device, the flow cycle direction and the flow channel in the separation pipeline can be changed. For example, in the cycle separation mode, when the pipeline switch on the adjusting pipeline is closed and the pipeline switch on the separation pipeline is opened, the fluid in the separation pipeline flows in accordance with the flow channel of the separation pipeline and can be used for cycle separation. For example, when the separation pipeline needs to be cleaned, in the cleaning mode, one ends of two adjusting pipelines are connected to the separation pipeline, and the other ends of the two adjusting pipelines can be used as the entrance and exit for the separation pipeline respectively, and the two adjusting pipelines cooperate with the separation pipeline to form a new flow channel, so that the separated fluid circulating in the separation pipeline can be led out of the separation pipeline from the exit. Cleaning fluid can also be introduced into the separation pipeline to adjust the separation pipeline to the cleaning mode, and the cleaning fluid can enter the separation pipeline and the second separation module based on the entrance and then flow to the exit to clean the separation pipeline wall and the second separation module, when the cleaning is completed, the target substance in the separation pipeline and the second separation module is removed.

In some embodiments, the channel-adjusting device corresponding to the separation pipeline is a four-way valve.

The four-way valve performs regulation to achieve effects similar to those described in the previous embodiment of the adjusting pipeline, which will not be repeated here. In some examples, the four-way valve can also be rotated to achieve the adjustment or switching of different operating modes in the separation pipeline.

In practice, the four-way valve may determine its structural parameters based on the pipeline parameters of the separation pipeline and its location in the separation pipeline. For example, the four-way valve may be set at a corner of the separation pipeline or in a flat pipeline, and the corresponding internal structure of the four-way valve may be changed accordingly. In some embodiments, the four-way valve may be obtained by 3D printing.

In some examples, the treatment module 24 is a collection device, it should be understood that in the fluid treatment method of the present disclosure, the separation pipeline 21 collects a certain volume of separated fluid such as target substance in cycle separation mode while circulating fluid, therefore, the inner cavity of the separation pipeline 21 can also be used as a storage space for target substance, and the collecting device can be a container or storage space for expanding the volume of separation pipeline 21.

In some examples, at least one of a control device, a storage device, a pressure detection device, a temperature detection device, a temperature control device, an air bubble detection and elimination device, an alarm device, and a concentration detection device is also provided in the separation pipeline.

It should be noted that the embodiments provided in the present disclosure is not intended to limit the location of the control device, the control device is located in the enrichment pipeline or the control device is located in the separation pipeline, as long as the control device is electrically connected to each driving device, detection device, or sensor, etc. in the enrichment pipeline or the separation pipeline, therefore, the control device may be located outside the pipeline. The control device is used to obtain information about the working state in the enrichment pipeline or the separation pipeline and to realize the control of the working state in the enrichment pipeline or the separation pipeline.

In an embodiment of the first aspect of the present disclosure, the second inlet of the separation pipeline is communicated with the first outlet of the enrichment pipeline. In some examples, the control device is a central system for controlling the cascaded enrichment pipeline and separation pipeline. The control device is connected to both the enrichment pipeline and the separation pipeline. In other examples, the enrichment pipeline and the separation pipeline may also be connected to different control devices.

It should be noted that in the embodiments provided in the present disclosure, the order in which steps S10, S11, and S12 of the fluid treatment method are performed is not limited, for example, in practical scenarios, step S12 may be performed first, followed by step S10. The step S10, step S11, and step S12 can be seen as necessary conditions for the execution of the fluid treatment method of the present disclosure, and the fluid treatment method of the present disclosure can be implemented by satisfying each condition. The present disclosure does not limit the order of occurrence among the above conditions, and the implementation way from step S10 to step S11 to step S12 is for ease of understanding.

Similarly, the order of execution between step S20 and step S21 of introducing fluid into the separation pipeline for treatment can be changed accordingly, and will not be described here.

In some embodiments of the first aspect of the present disclosure, the fluid treatment method is used for the treatment of disease. For example, through an extracorporeal cycle clearance system that can perform the fluid treatment method of the present disclosure, enrichment and selective removal of target molecules can be achieved. The target molecules are often pathogenic factors or outcomes of disease, or are essential for disease development or health maintenance, and the removal of the target molecules facilitates treatment of the disease or reduction of complications. The diseases include, but are not limited to, at least one of familial hypercholesterolemia, hyperlipoproteinemia, systemic lupus erythematosus, autoimmune diseases, myasthenia gravis, rapidly progressive glomerulonephritis, fatty liver, liver cirrhosis, acute liver failure, hyperlipidemia, severe acute pancreatitis, sepsis, Guillain-Barre syndrome, and obesity. The fluid treatment method of the present disclosure may be used for the treatment of a variety of diseases without the limitation of the foregoing examples, but only if the fluid treatment method provided by the present disclosure is implemented or the process of the fluid treatment method is reproduced based on the device or apparatus, whereby enrichment and removal of any one or more molecules from the body is achieved and therapeutic effect on the health or disease of the patient is achieved.

In embodiments of the present disclosure, the treatment includes preventive, obstructive, curative or palliative treatments resulting in the desired physiological effect. In addition, the term "treatment" is used here for purposes that may partially or completely mitigate, delay the occurrence, inhibit the progression, reduce the severity, and/or reduce the incidence of one or more symptoms of a particular disease, abnormality, and/or medical condition.

In embodiments of the fluid treatment method for disease treatment according to the present disclosure, the target substance in the blood or tissue fluid can be sustainably and highly selectively enriched and removed by identifying the pathogenic factors in the patient's lesion, such as blood, or other tissue fluid, thereby altering the concentration of the pathogenic factors in the patient. In practical scenarios, the corresponding separation module is selected or designed by identifying pathogenic factors in blood or tissue fluid for a specific disease type, i.e., enriching specific pathogenic factors in the pipeline or removing specific pathogenic factors from the pipeline.

In an embodiment, the blood or tissue fluid led out from the patient's body may also be pre-treated by the treatment module, and then the target substance in the pre-treated plasma or tissue fluid may be enriched or removed, and the treatment time may be controlled based on the pre-determined removal target.

In a further embodiment, the target substance cycled and enriched by the enrichment pipeline or the separated fluid cycled and separated in the separation pipeline may be transferred to the treatment module for retreatment, and the treatment module may decompose, catalyze, heat, concentrate, etc. the target substance or the separated fluid.

The fluid treatment method provided in the first aspect of the present disclosure intercepts the target substance in the fluid by the enrichment pipeline and the first separation module and enables the target substance to be cyclically enriched in the enrichment pipeline. In this cycle enrichment mode, the fluid in the enrichment pipeline flows at a predetermined flow rate driven by the first driving device, thereby ensuring a dynamic balance of the total amount of fluid in the enrichment pipeline, so that the fluid treatment method of the present disclosure is sustainable in treatment. The fluid treatment method of the present disclosure allows the collection or treatment of some components of the fluid, i.e. the target substance, and the fluid discharged from the first outlet of the enrichment pipeline can be transferred to the treatment module or storage space. The fluid treatment method is sustainable, thereby achieving a controlled separation effect. Meanwhile, the total amount of fluid in the enrichment pipeline is balanced during the cycle enrichment process, which prevents a significant decrease in the separation effect as the separation proceeds, and the target substance in the fluid passes through the first separation module circularly to be intercepted, thus avoiding a continuous loss of beneficial components as the fluid undergoes component separation or exchange.

Further, in some embodiments, the fluid treatment method can achieve the treatment of fine-grained target components in fluids based on cascaded enrichment pipelines or cascaded enrichment pipelines and separation pipelines. In some scenarios, the collection or treatment of any component of the fluid can be achieved, thereby eliminating or reducing application limitations in different areas. For example, in medical applications, different components of human body fluids (including blood) can be collected or treated, in pharmaceutical processes, different active ingredients can be separately enriched, in water treatment, different solutes can be separately enriched, etc. The fluid treatment method can also be used in the fields of water-oil separation, food processing, drug purification, medical treatment, biological products, environmental protection, chemical industry, metallurgy, material separation, petroleum treatment, and water treatment, etc.

The present disclosure further provides a fluid treatment method in a second aspect. The method includes: introducing a fluid into a separation pipeline based on at least one second separation module; the separation pipeline has an entrance and an exit; the second separation module has a first side and a second side separated by a second separation component, the opposite ends of the first side of the second separation module are communicated with at least one second inlet and at least one second outlet, respectively, and opposite ends of the second side of the second separation module are communicated with the entrance and exit of the separation pipeline, respectively; and driving the fluid from the entrance to the exit in the separation pipeline at a predetermined flow rate based on at least one second driving device to control the dynamic balance of the total amount of fluid in the separation pipeline.

The fluid includes a target substance, in some embodiments, the fluid includes, but is not limited to, one or more of blood, plasma, serum, body fluid, tissue fluid, washing fluid, dialysate, recombinant protein solution, cell culture medium, microbial culture medium, pharmaceutical and medical water, liquid medicine, fluid food, plant and animal extract, natural water, industrial wastewater, recycled water, light distillate oil, methane, and liquefied gas. In some examples, the fluid may also be substance components obtained after treatment, such as filtration, of a fluid such as blood, plasma, etc. In other examples, the fluid may also be a gas mixture such as a gas mixture including methane.

The fluid treatment method of the present disclosure can be applied to different classes of fluids, generally, the separation can be implemented only if the fluids have different substance components that have selective permeability differences when passing through the separation membrane, and have a certain mobility, for example, the fluids may be liquid mixtures or gas mixtures as described above. The fluids may also be other solid-liquid mixed-phase fluids or colloids and are not limited.

**FIG.** 13 shows a flow diagram of a fluid treatment method according to a second aspect of an embodiment of the present disclosure.

In step S30, a fluid is introduced into a separation pipeline through at least one second separation module. The separation pipeline has an entrance and an exit. The second separation module has a first side and a second side separated by a second separation component, the opposite ends of the first side of the second separation module are communicated with at least one second inlet and at least one second outlet, respectively, and opposite ends of the second side of the second separation module are communicated with the entrance and exit of the separation pipeline, respectively.

In step S31, a fluid in the separation pipeline is driven to flow from the entrance of the separation pipeline to the exit of the separation pipeline at a third preset flow rate by at least one second driving device to dynamically balance the total amount of the fluid in the separation pipeline.

In some embodiments, the second separation component is a porous membrane, a reverse osmosis membrane, or a gas separation membrane.

In some embodiments, the average pore diameter or molecular weight cutoff of the porous membrane or the reverse osmosis membrane is associated with compositions of the fluid, and the porous membrane includes a microfiltration membrane, an ultrafiltration membrane, or a nanofiltration membrane.

In some embodiments, the second separation component includes one or more of a planar membrane, a tubular membrane, a roll membrane, a spiral membrane, and a hollow fiber membrane.

In some embodiments, the separation pipeline is further provided with at least one of a control device, a fluid storage device, a pressure detection device, a temperature detection device, a temperature control device, an oxygen detection device, a bubble detection and elimination device, an alarm device, and a concentration detection device.

The form of the second separation component is related to the predetermined treatment objective of the target substance in the fluid. The structure of the second separation module and the separation pipeline, the connection means of the different components, the other structures or modules such as the treatment module configurable for the separation pipeline, and the way of controlling the fluid in the separation pipeline by the second driving device can be referred to the embodiments provided in the first aspect of the present disclosure, such as the separation pipeline and the second separation module described in any of the implementations of FIGs. 11 to 12. The corresponding structures, the connection ways and control methods between structures, and the fluid treatment effects achieved are similar and will not be described herein.

In some embodiments of the second aspect of the present disclosure, the fluid cyclically flows through N cascaded separation pipelines, where the fluid in the previous separation pipeline is introduced into a corresponding second inlet of the latter separation pipeline, and N is a positive integer not less than 2.

FIG. 14 shows a simplified schematic diagram of the N cascaded separation pipelines according to an embodiment.

The previous separation pipeline and latter separation pipeline are used to illustrate any two adjacent separation pipelines of the N cascaded separation pipelines. The order of the previous and latter is a connection order, and the connection order is distinguished based on the flow direction of the fluid in the separation pipeline, i.e., the fluid always flows from the previous separation pipeline for treatment, then flows into the latter separation pipeline for retreatment, thus, the fluid always flows from the entrance in the previous separation pipeline to the second inlet connected to the second separation module in the latter separation line. Correspondingly, the second outlet connected to the second separation module in the latter separation pipeline is connected to the exit of the previous separation pipeline.

By cascading N separation pipelines, the fluid is separated and cycled for N times in accordance with the connection order of the separation pipelines. In the cycle treatment corresponding to each separation pipeline, the fluid flow rate or volume at the second inlet is equal to that at the second outlet in the cycle separation mode, i.e., all the N cascaded separation pipelines are in dynamic balance, so that continuous cycle separation can be performed in each of the separation pipelines. It should be noted that, in accordance with the connection order described, the fluid is sequentially intercepted at the first side of the second separation module corresponding to the cascaded separation pipelines. Therefore, the fluid components introduced at the second inlet of each separation pipeline are different, correspondingly, the fluid components in different separation pipelines are different.

In each of the embodiments provided in the present disclosure, the different fluid components include: different kinds of substances in the fluid or/and different concentrations or total amounts of at least one substance in the fluid.

The present disclosure provides an embodiment for treating fluids by using N cascaded separation pipelines, which can be used to intercept components with different particle sizes or molecular weights, or with different chemical properties, or molecules with different charge properties in the fluid, respectively. By providing corresponding second separation modules in the separation pipelines, based on the selective permeability of different second separation modules to components in the fluid, cycle separation of different fine-grained components can be achieved.

It should be understood that the fluid in the separation pipelines of the present disclosure is reversibly cyclic in the cycle separation mode. Based on the interception effect of the second separation module, it is possible to realize that the fluid in the separation pipeline connected to the second side of the second separation module contains specific components, while the fluid in the first side of the second separation module may contain both the specific components and the intercepted components, thus, in accordance with the connection order in the cascaded separation pipelines, the kinds of fluid components in the separation pipelines are gradually reduced.

**In** this embodiment, the fluid can be treated based on the cascaded separation pipelines, i.e., only some of the components of the fluid, such as the separated fluid, can be treated, while the substance components that do not need to be treated can return to the upstream based on a reversible cycle in the separation pipelines. For example, as in the embodiment shown in FIG. 14, when the fluid includes components a, b, c and d, in the separation process, in accordance with the connection order, the fluid components in the first separation pipeline 21 are b, c, d, where component a is intercepted at the first side of the second separation module 22a corresponding to the first separation pipeline 21a. The fluid components in the second separation pipeline 21b are c and d, where component b is intercepted at the first side of the second separation module 22b corresponding to the second separation pipeline 21b and can flow therein to the first separation pipeline 21a, specifically, component b can return to the first side of the second separation module 22a corresponding to the first separation pipeline 21a based on the reversible cycle. The fluid component in the third separation pipeline 21c is d, where component c is intercepted at the first side of the second separation module 22c corresponding to the third separation pipeline 21c and can flow therein to the second separation pipeline 21b, specifically, component c can return to the first separation pipeline 21a based on the reversible cycle. In practical scenarios, when the component d is the component to be retreated, the fluid can be retreated in the third separation pipeline 21c, and other components in the fluid such as a, b and c can be returned to the upstream pipeline, which can be used to achieve the treatment of specific components in the fluid. In the embodiments provided in the second aspect of the present disclosure, for any of the separation pipelines and their corresponding second separation module, the pipeline connected to the first side of the second separation module is the upstream pipeline, and the separation pipeline is the downstream pipeline.

In some embodiments, in the N cascaded separation pipelines, the separated fluid in the separation pipeline can be discharged out of the separation pipeline based on the exit of the adjusting pipe of the channel-adjusting device or based on the treatment module, so that the fine-grained target substance can be extracted or treated directionally, thereby achieving a change in the concentration of the fine-grained target substance in the fluid. For example, the molecular weight of each component in the fluid may range from 200 to 2000 Dalton, and N is 2, in accordance with the connection order, the second separation module of the previous separation pipeline intercepts components with molecular weight above 1100 Dalton in the fluid, and the separated fluid components in the previous separation pipeline have molecular weight ranging from 200 to 1100 Dalton; the second separation module of the latter separation pipeline intercepts components with molecular weight above 800 Dalton, and the separated fluid components in the latter separation pipeline have molecular weights ranging from 200 to 800 Dalton.

In practical scenarios, the number of the N cascaded separation pipelines is any positive integer not less than 2, correspondingly, multiple components in a fluid or components within multiple classification segments may be collected, extracted or treated. The classification segment may be a classification segment obtained by dividing the components based on at least one of their chemical properties, molecular weights or molecular particle sizes, and charge properties. It should be understood that the classification segment has multiple classification criteria, as long as a corresponding second separation component makes it possible for the substances in the classification segment to be intercepted at the first side of the second separation module. When the second separation component is a separation membrane, for example, different types of separation membranes have different selective permeability, and by setting up N sets of separation membranes, the fluid components can be sorted or intercepted for N times.

For example, when the fluid is treated based on N cascaded separation pipelines, the large molecule components can be intercepted at the first side of the second separation module of the first separation pipeline based on the molecular weight, the components that do not selectively permeate the second separation module of the second separation pipeline can be intercepted in the second separation module of the second separation pipeline based on the chemical properties of the fluid, and the charged ions are intercepted based on the charge properties of the fluid components in the third separation pipeline. In practical scenarios, for example, in medical applications, the N cascaded separation pipelines can be used, for example, to separate antibiotics, amino acids, enzymes, and other proteins in fluids.

In some embodiments of the second aspect of the present disclosure, a molecular size or a molecular weight of the target substance, or particle sizes of components of target substance, corresponding to each of the N cascaded separation pipelines, decreases from the first separation pipeline in the N cascaded separation pipelines to the Nₜₕ separation pipeline in the N cascaded separation pipelines.

In this embodiment, the components of the fluid are classified according to the molecular size or molecular weight, and the selective permeability of the second separation modules corresponding to the N cascaded separation pipelines to the fluid components can be designed based on the component size or molecular weight. For example, the average pore size of the separation membranes in the N sets of second separation modules corresponding to the N cascaded separation pipelines may be gradually reduced, or the intercepted molecular weight may be gradually reduced, in accordance with the connection order, the molecular size or molecular weight of the target substance intercepted at the first side of the second separation module of each separation pipeline may also be gradually reduced.

The second separation module corresponding to each separation pipeline can intercept some of the components in the fluid to the first side of the second separation module, so the second side of the second separation module may contain specific components in the fluid. When treating the fluid, specific components in the fluid can be treated by selecting the corresponding separation pipeline according to needs.

In some embodiments of the second aspect of the present disclosure, the method further includes the following operations: introducing the fluid in the separation pipeline into an enrichment pipeline, where the enrichment pipeline includes a first section and a second section, an entrance of the first section is communicated with at least one first inlet, and an exit of the second section is communicated with the first section; intercepting a target substance in the fluid by at least one first separation module, where each of the at least one first separation module includes a first separation component to divide a corresponding first separation module into a first side and a second side, two opposite ends of the first side of each of the at least one first separation module are communicated with the first section and the second section respectively, and the second side of each of the at least one first separation module is communicated with at least one first outlet; driving the fluid in the enrichment pipeline to cyclically flow at a preset flow rate based on at least one first driving device to enrich the target substance, where the at least one first driving device is arranged at the first section and is configured to dynamically balance a total amount of the fluid in the enrichment pipeline.

**A** second aspect of the present disclosure further provides embodiments in which a separation pipeline is cascaded with an enrichment pipeline.

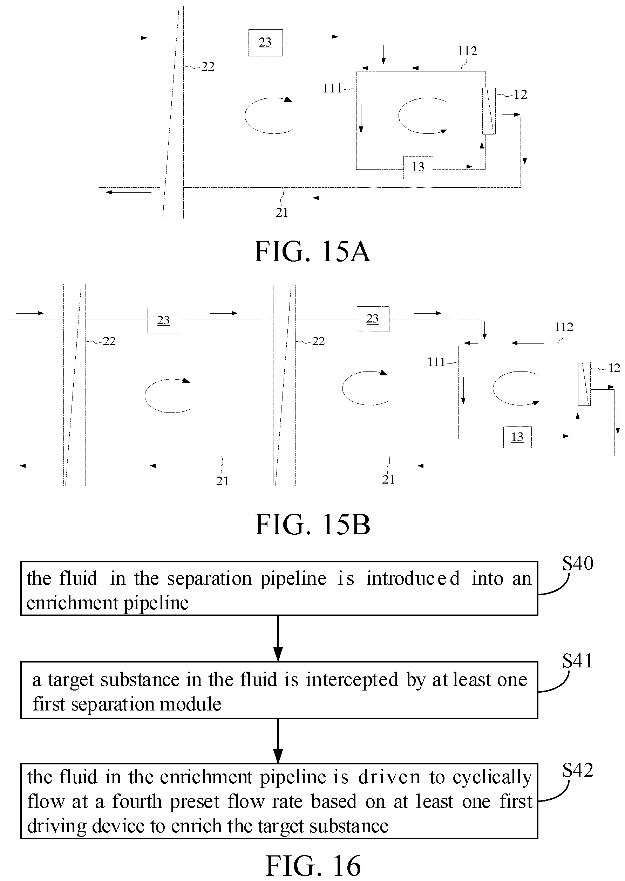

FIGs. 15A and 15B show simplified structural diagrams of cascaded separation pipeline and enrichment pipeline in different embodiments.

In embodiments of cascading the separation pipeline with the enrichment pipeline as described in the second aspect of the present disclosure, fluid from the separation pipeline is introduced to a first inlet of the enrichment pipeline, as shown in FIG. 15A; or after being treated in more than N cascaded separation pipelines, and in accordance with the connection order, the fluid in the last separation pipeline is transferred to the first inlet of the enrichment pipeline, where N is a positive integer not less than 2, such as the embodiment shown in FIG. 15B.

For example, in adjacent separation pipeline 21 and enrichment pipeline 11, the fluid flows from the entrance of the separation pipeline 21 to the first inlet of the enrichment pipeline 11, then flows through the first separation module 12 in the enrichment pipeline 11 to the first outlet, and the fluid discharged from the first outlet flows to the exit of the separation pipeline 21 to form a separation cycle. That is, in this example, the enrichment pipeline 11 may be considered as a sub-cycle device in the separation cycle.

FIG. 16 shows a flow chart of the fluid treatment method according to a second aspect of an embodiment of the present disclosure.

In step S40, the fluid in the separation pipeline is introduced into an enrichment pipeline. The enrichment pipeline includes a first section and a second section, an entrance of the first section is communicated with at least one first inlet, and an exit of the second section is communicated with the first section.

In step S41, a target substance in the fluid is intercepted by at least one first separation module. Each of the at least one first separation module includes a first separation component dividing a corresponding first separation module into a first side and a second side, two opposite ends of the first side of each of the at least one first separation module are communicated with the first section and the second section respectively, and the second side of each of the at least one first separation module is communicated with at least one first outlet.

In step S42, the fluid in the enrichment pipeline is driven to cyclically flow at a fourth preset flow rate based on at least one first driving device to enrich the target substance. The at least one first driving device is arranged at the first section and is configured to dynamically balance a total amount of the fluid in the enrichment pipeline.

In some embodiments of the second aspect of the present disclosure, the first separation component is a porous membrane, a reverse osmosis membrane, or a gas separation membrane.

In some embodiments of the second aspect of the present disclosure, the average pore diameter or molecular weight cutoff of the porous membrane or the reverse osmosis membrane is associated with the target substance. The porous membrane includes a microfiltration membrane, an ultrafiltration membrane, and a nanofiltration membrane.

In some embodiments of the second aspect of the present disclosure, the first separation component includes one or more of a planar membrane, a tubular membrane, a roll membrane, a spiral membrane, and a hollow fiber membrane.

In some embodiments of the second aspect of the present disclosure, the first separation module is a tangential flow filtration module.

In some embodiments of the second aspect of the present disclosure, the at least one first driving device controls a flow rate in the first section to be greater than a preset threshold, so that the target substance in the first separation module flows from an exit of the first section to an entrance of the second section.

In some embodiments of the second aspect of the present disclosure, the preset threshold is determined based on at least one of the following: a composition of the fluid, a temperature of the fluid, a structure of the first separation component, a material of the first separation component, a cavity structure of the first separation module, and a diameter of the enrichment pipeline.

In some embodiments of the second aspect of the present disclosure, the second section is further provided with the first driving device.

In some embodiments of the second aspect of the present disclosure, a total volume or total velocity of the fluid introduced into the enrichment pipeline from the at least one first inlet is equal to that of a fluid discharged from the enrichment pipeline by the at least one first outlet, so that the target substance is cyclically enriched.

In some embodiments of the second aspect of the present disclosure, an enrichment efficiency of the fluid treatment method is adjusted by controlling a ratio of the total volume of the fluid introduced into the enrichment pipeline per unit time to a total volume of the fluid in the enrichment pipeline.

In some embodiments of the second aspect of the present disclosure, the enrichment pipeline further includes a channel-adjusting device for adjusting the flow direction of the fluid in the enrichment pipeline. The channel-adjusting device may adjust the flow direction of the fluid in the enrichment pipeline through at least one of the following:
adjusting the flow direction of the fluid in the enrichment pipeline by adjusting a switching state of the at least one first inlet and/or the at least one first outlet; and
adjusting the flow direction of the fluid in the enrichment pipeline by adjusting a switching state of at least one adjusting channel connected to the enrichment pipeline; wherein a switching state includes an open state or a closed state.

In some embodiments of the second aspect of the present disclosure, the channel-adjusting device includes a first pipeline switch arranged at each of the at least one first inlet, for controlling a switching state of the corresponding first inlet to further control a treatment state of the fluid in the enrichment pipeline to be a concentration cycle mode or a cleaning mode.

In some embodiments of the second aspect of the present disclosure, the channel-adjusting device further comprises at least one adjusting pipeline and at least one second pipeline switch, so that an entrance of a cleaning solution and an exit of a waste fluid are formed in the enrichment pipeline.

In some embodiments of the second aspect of the present disclosure, the channel-adjusting device is a four-way valve.

In some embodiments of the second aspect of the present disclosure, the enrichment pipeline is switched to a concentration cycle mode or a diluted mode, to adjust a separation efficiency of the at least one first separation module.

In some embodiments of the second aspect of the present disclosure, the enrichment pipeline further includes an air inlet-outlet, arranged at the first section and/or the second section, for adjusting a state of air inside the enrichment pipeline or an air pressure inside the enrichment pipeline.

In some embodiments of the second aspect of the present disclosure, the enrichment pipeline further includes at least one collection device, arranged at the first section and/or the second section, for adjusting a volume of the enrichment pipeline to collect the target substance, or for collecting bubbles.

In some embodiments of the second aspect of the present disclosure, the enrichment pipeline is further provided with at least one of a control device, a fluid storage device, a pressure detection device, a temperature detection device, a temperature control device, an oxygen detection device, a bubble detection and elimination device, an alarm device, a flow rate detection device, and a concentration detection device.

In some embodiments of the second aspect of the present disclosure, the control device generates information for adjusting a working state of the enrichment pipeline based on at least one of the flow rate detection device, the pressure detection device, and the concentration detection device.

The specific structure of the enrichment pipeline and the first separation module, the connection way and position relationship among different structures or modules in the enrichment pipeline, the way of the first driving device for controlling the fluid in the enrichment pipeline, and the way that the channel-adjusting device, collection device and other equipment in the enrichment pipeline and other structures coordinate together for achieving different fluid treatment functions, can be referred to the embodiments provided in the first aspect of the present disclosure. For example, the enrichment pipeline, the first driving device, and the first separation module described in the embodiments of the second aspect of the present disclosure may be the enrichment pipeline, the first driving device in the enrichment pipeline, and the first separation module described in any of the embodiments shown in FIGs. 1 to 9.

In some embodiments of the second aspect of the present disclosure, the method further includes: performing pretreatment or retreatment on the fluid by at least one treatment module. The pretreatment or retreatment includes at least one of a filtration treatment, an adsorption treatment, a heating treatment, a catalytic treatment, an enrichment treatment, a concentration treatment, a chemical treatment, an optical treatment, and an electrical treatment. For practical applications, the treatment module may include, for example, adsorption devices, extraction devices, ion exchange processing devices, centrifugal devices, filtration devices, heating devices, etc.

The treatment module pre-treats the fluid, i.e. treats the initially obtained fluid to introduce the treated fluid into the second separation module through a second inlet. The treatment module performs the pre-treatment by causing the fluid treated by the treatment module to be transferred to the second inlet of the separation pipeline.

The treatment module retreats the fluid, i.e. treats the separated fluid in the separation pipeline to process the target substance obtained by cycle separation. Based on the selective permeability of the second separation component to the fluid components, the separated fluid contains specific components, and retreatment of specific components such as concentration collection, chemical decomposition, etc. can be achieved in combination with the treatment module. Alternatively, the retreatment means that the treatment module treats the fluid discharged from the second outlet at the second side of the second separation module. Alternatively, the retreatment means that the treatment module treats the target substance cyclically enriched in the enrichment pipeline or the fluid discharged from the first outlet of the enrichment pipeline.

The type of the pretreatment or retreatment performed by the treatment module may be determined based on the needs. For example, when the component intercepted at the first side of the second separation module is a fluid component to be filtered out and the separated fluid in the separation pipeline is a beneficial component to be purified, the treatment module may perform a concentration process on the fluid in the separation pipeline. For another example, when the target substance in the enrichment pipeline is a component to be filtered out, the treatment module may remove the target substance by chemical decomposition.

In some embodiments, in the fluid treatment method provided in the second aspect of the present disclosure, in the cascaded separation and enrichment pipelines, the second inlet and the second outlet of the second separation module are connected to the same storage section, and the storage section includes a fluid storage device, a container, and a human body. The storage section is used to achieve the storage or connection function of the fluid introduced into the second inlet and the fluid discharged out of the second outlet, therefore, the storage section may be a container or storage space, in a particular application scenario, the storage section may also be the human body's blood cycle, which is not limited herein.

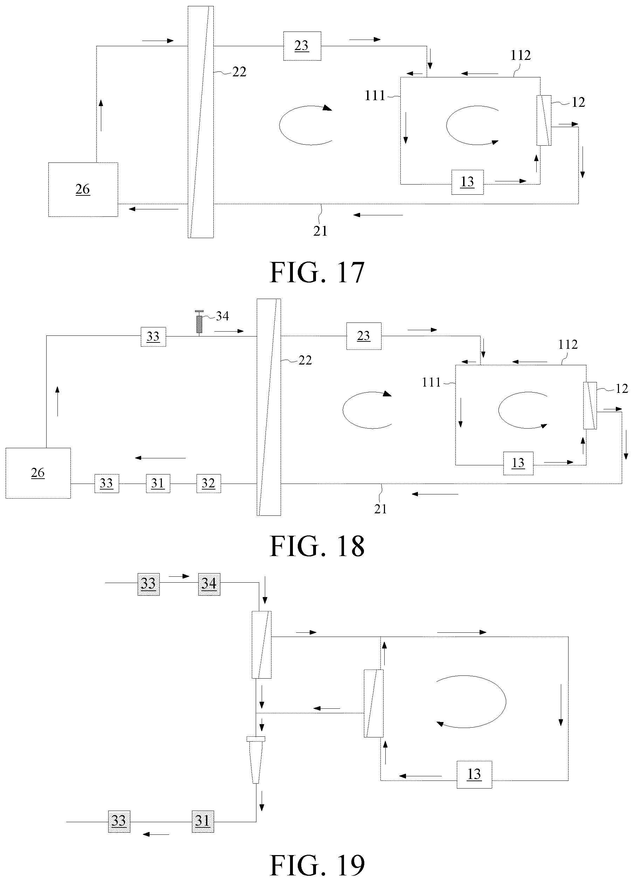

FIG. 17 shows a simplified schematic diagram of the cascaded separation pipeline and enrichment pipeline according to an embodiment of the present disclosure. For example, in an embodiment, the second inlet and second outlet are connected to the same storage section 26, and the storage section 26 is, for example, a storage device or container. The second driving device 23 controls the dynamic balance of the total amount of fluid in the separation pipeline 21, i.e., performs cycle separation, and the first driving device 13 controls the dynamic balance of the total amount of fluid in the enrichment pipeline 11, i.e., performs cycle enrichment. Correspondingly, the total amount of fluid in the storage section 26 is dynamically balanced.

In this case, a specific component, i.e. the separated fluid, in the fluid in the storage section 26 is separated out by the second separation module 22, and the separated fluid is introduced into the enrichment pipeline 11. When the enrichment pipeline 11 is in a cycle enrichment mode, the separated fluid enriches target substances in the enrichment pipeline 11, and the fluid that the target substance component is separated out is returned to the exit of the separation pipeline 21 via the second side of the first separation module, and returns to the storage section 26 based on the reversible cycle separation in the separation pipeline 21. Continuous cycle separation and cycle enrichment are performed, then the target substances in the fluid in the storage section 26 gradually decreases while the total amount of fluid remains the same. The collection of specific target substance components without generating waste fluid is achieved. The total amount of fluid in the storage section 26 remains the same, and the cycle separation and cycle enrichment process can be continued.

In another case, when the storage section is a human body, the fluid may be blood, plasma, serum, tissue fluid, or other body fluid of the human body. In this example, the second inlet and the first outlet may be connected to the human body either directly or indirectly, the direct connection indicates that the second inlet and first outlet may be connected to the cycle system of the human body, the indirect connection indicates that the second inlet or/and the first outlet is connected to a treatment module, and the treatment module is connected to the human cycle system. For example, the treatment module may be used to separate plasma from human whole blood, and the second inlet is connected to the separated plasma. Thus, the fluid treatment method based on the present disclosure does not produce waste plasma when the pathogenic factors to be filtered out of the plasma are intercepted in the enrichment pipeline, and the concentration of the target substance in the plasma in the human body can be changed based on continuous separation of the plasma in the human body, thereby avoiding the problem of continuous loss of beneficial components.

In some examples, the separation pipeline or the enrichment pipeline may be a separation module, and it is easy to understand that both the separation pipeline and the enrichment pipeline can achieve the separation effect based on the corresponding separation module, therefore, both the separation pipeline and the enrichment pipeline can be used as a separation device or a filtration device. For example, in a practical scenario, the second inlet of the separation pipeline is connected to the human blood cycle system, and the cellular components of the blood are intercepted in the first side of the second separation module based on the second separation component, and the plasma passing through the second separation component flows in the separation pipeline, and at the same time, the plasma in the separation pipeline is transferred to the first inlet of the enrichment pipeline, so that the target substance in the plasma can be enriched. When the target substance is a pathogenic factor to be removed, the fluid discharged from the first output of the enrichment pipeline is the purified plasma, which is returned to the separation pipeline and can be reversibly cycled based on the separation pipeline to the first side of the second separation module, so that the purified plasma can be mixed with cellular components and returned to the human blood cycle system. In this practical scenario, the removal of the pathogenic factor from the plasma can be sustained and no waste fluid is generated during the plasma purification process. At the same time, other components in the plasma except for the pathogenic factor can be returned to the upstream pipeline, i.e. the human body, based on reversible cycle separation, and the construction of the pipeline system is simplified in practical applications.

It should be noted that in the second aspect of the embodiments provided in the present disclosure, the control device may be located in the enrichment pipeline or the separation pipeline, and the present disclosure is not intended to limit the location of the control device as long as the control device is electrically connected to each driving device, detection device, or sensor, etc. in the enrichment pipeline or the separation pipeline, therefore, the control device may be located outside the pipeline. The control device is used to obtain information about the working state in the enrichment pipeline or the separation pipeline and to realize the control of the working state in the enrichment pipeline or the separation pipeline.

In some embodiments of the second aspect of the present disclosure, the separated fluid in the separation pipeline is transferred to the first inlet of the enrichment pipeline. In some examples, the control device is a central system for controlling the cascaded enrichment pipeline and separation pipeline, and the control device is connected to both the enrichment pipeline and the separation pipeline. In other examples, the enrichment pipeline and the separation pipeline may also be connected to different control devices.

It should be noted that in the embodiments provided in the second aspect of the present disclosure, the order in which steps S30 and S31 of the fluid treatment method are performed is not limited, for example, in practical scenarios, step S31 may be performed first, followed by step S30. The step S30 and step S31 can be seen as necessary conditions for the execution of the fluid treatment method in the second aspect of the present disclosure, and the fluid treatment method can be implemented by satisfying each condition. The present disclosure does not limit the order of occurrence between the conditions, and the implementation way from step S30 to step S31 is for ease of understanding.

Similarly, step S40, step S41, and step S42 can also be used as treatment conditions in the enrichment pipeline, and the corresponding actual order of implementation can be changed accordingly, which will not be repeated here.

In some embodiments of the second aspect of the present disclosure, the fluid treatment method is used for the treatment of disease. For example, through an extracorporeal cycle clearance system that can perform the fluid treatment method of the present disclosure, enrichment and selective removal of target molecules can be achieved. The target molecules are often pathogenic factors or outcomes of disease, or are essential for disease development or health maintenance, and the removal of the target molecules facilitates treatment of the disease or reduction of complications. The diseases include, but are not limited to, at least one of familial hypercholesterolemia, hyperlipoproteinemia, systemic lupus erythematosus, autoimmune diseases, myasthenia gravis, rapidly progressive glomerulonephritis, fatty liver, liver cirrhosis, acute liver failure, hyperlipidemia, severe acute pancreatitis, sepsis, Guillain-Barre syndrome, and obesity. The fluid treatment method of the present disclosure may be used for the treatment of a variety of diseases without the limitation of the foregoing examples, but only if the fluid treatment method provided by the present disclosure is implemented or the process of the fluid treatment method is reproduced based on the device or apparatus, whereby enrichment and removal of any one or more molecules from the body is achieved and therapeutic effect on the health or disease of the patient is achieved.

In embodiments of the fluid treatment method for disease treatment according to the present disclosure, the target substance in the blood or tissue fluid can be sustainably cycled and separated by identifying the pathogenic factors in the patient's lesion, such as blood, or other tissue fluid, thereby altering the concentration of the pathogenic factors in the patient. In practical scenarios, the corresponding separation module is selected or designed by identifying pathogenic factors in blood or tissue fluid for a specific disease type, i.e., allowing specific pathogenic factors flowing in the separation pipeline to treat the pathogenic factors in combination with the treatment module.

In an embodiment, the blood or tissue fluid led out from the patient's body may also be pre-treated by the treatment module, and then the target substance in the pre-treated plasma or tissue fluid may be enriched or removed, and the treatment time may be controlled based on the pre-determined removal target.

In a further embodiment, the target substance cycled and enriched by the enrichment pipeline or the separated fluid cycled and separated in the separation pipeline may be transferred to the treatment module for retreating, and the treatment module may decompose, catalyze, heat, concentrate, etc. the target substance or the separated fluid.

The fluid treatment method provided in the second aspect of the present disclosure intercepts the components in the fluid by the second separation module and causes the specific components in the fluid to be cyclically separated in the separation pipeline, and in this cycle separation mode, the fluid in the separation pipeline flows at a predetermined flow rate driven by the second driving device, thereby ensuring a dynamic balance of the total amount of fluid in the separation pipeline. The fluid treatment method of the present disclosure is sustainable in treatment and enables the collection of target substances in the fluid or achieves a change in the concentration of fluid components. The fluid or separated fluid from the second outlet of the separation pipeline can be accessed to the treatment module or storage space, thus avoiding the continuous loss of beneficial components during the separation or exchange of components in the fluid.

Furthermore, in some embodiments, the fluid treatment method can realize the processing of fine-grained target components in the fluid based on cascaded separation pipelines or cascaded separation pipelines and enrichment pipelines. At the same time, based on the reversible cycle characteristics of the separation pipelines, the target molecules or preset specific components that need to be removed or processed can be intercepted in different cycles, and other fluid components that do not need to be treated can be transferred to the upstream pipeline based on the reversible cycle, thus enabling directional and selective enrichment, removal, collection, or other treatment of specific components and avoiding the declined separation effect over time. In some scenarios, the fluid treatment method can be used to collect or process any component in the fluid and can eliminate or minimize application restrictions in different fields. For example, different components in human body fluids (including blood) can be collected or treated in medical applications, different active components can be enriched separately in pharmaceutical preparation, and different solutes can be enriched, separated, and treated separately in water treatment.

The present disclosure further provides a fluid treatment device in the third aspect. The device includes at least one cycle enrichment module. The cycle enrichment module includes an enrichment pipeline, at least one first separation module, and at least one first driving device. The enrichment pipeline includes a first section and a second section. An entrance of the first section communicates with the first inlet, and an exit of the second section communicates with the first section. The at least one first separation module includes a first separation component to separate the first separation module into a first side and a second side. Two opposite ends of the first side are respectively communicated with the first section and the second section, and the second side is communicated with at least one first outlet. The at least one first driving device is arranged in the first section for driving the fluid to cyclically flow in the enrichment pipeline to dynamically balance the total amount of fluid in the enrichment pipeline, so that the first separation module enriches the target substance in a cycle enrichment mode.

**FIG.** 2 shows a simplified schematic diagram of the cyclic enrichment module in an embodiment. The cycle enrichment module includes the enrichment pipeline 11. The enrichment pipeline 11 includes the first section 111 and the second section 112. The entrance of the first section 111 is communicated with the first inlet, and the exit of the second section 112 is communicated with the first section.

In this case, the first section 111 and the second section 112 of the enrichment pipeline 11 can be connected with each other in a certain way to enable the fluid in the enrichment pipeline 11 to flow cyclically. The exit of the second section 112 is connected to the first section 111, therefore, after entering the first section 111 from the first inlet, the fluid can flow to the second section 112 and re-enter the first section 111 from the exit of the second section 112, thus forming a cyclic flow.

The at least one first separation module 12 includes the first separation component dividing the first separation module into the first side and the second side, where the first side is communicated with the exit of the first section 111 and the entrance of the second section 112, and the second side is communicated with at least one first outlet.

In some embodiments in the third aspect of the present disclosure, the fluid contains the target substance, and the fluid includes one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, methane, light distillate oil, and liquefied gas.

In some embodiments in the third aspect of the present disclosure, the first separation component is a porous membrane, a reverse osmosis membrane, or a gas separation membrane.

In some embodiments in the third aspect of the present disclosure, the average pore diameter or a molecular weight cutoff of the porous membrane or the reverse osmosis membrane is associated with the target substance, where the porous membrane includes a microfiltration membrane, an ultrafiltration membrane, and a nanofiltration membrane.

In some embodiments in the third aspect of the present disclosure, the first separation component includes one or more of a planar membrane, a tubular membrane, a roll membrane, a spiral membrane, and a hollow fiber membrane.

In some embodiments in the third aspect of the present disclosure, the first separation module is a tangential flow filtration module.

In some embodiments in the third aspect of the present disclosure, the at least one first driving device controls the flow rate in the first section to be greater than a preset threshold, so that the target substance in the first separation module flows from the exit of the first section to the entrance of the second section.

In some embodiments in the third aspect of the present disclosure, the preset threshold is determined by at least one of the following: the composition of the fluid, the temperature of the fluid, the structure of the first separation component, the material of the first separation component, the cavity structure of the first separation module, and the diameter of the enrichment pipeline.

In some embodiments in the third aspect of the present disclosure, the second section of the cycle enrichment module is further provided with the first driving device.

In some embodiments in the third aspect of the present disclosure, in the cycle enrichment mode, the total volume or total velocity of the fluid introduced into the enrichment pipeline from the first inlet is equal to that of the fluid discharged from the enrichment pipeline through the first outlet.

In some embodiments in the third aspect of the present disclosure, the cycle enrichment module controls the ratio of the total volume of the fluid introduced into the enrichment pipeline per unit time to the total volume of the fluid in the enrichment pipeline to adjust the enrichment efficiency.

In some embodiments in the third aspect of the present disclosure, the enrichment pipeline is further provided with a channel-adjusting device for adjusting the flow direction of the fluid in the enrichment pipeline, where the channel-adjusting device adjusts the flow direction of the fluid in the enrichment pipeline by at least one of the following ways: adjusting the switching state of the first inlet and/or the first outlet; and adjusting the switching state of the at least one adjusting channel connected to the enrichment pipeline.

In some embodiments in the third aspect of the present disclosure, the channel-adjusting device includes the first pipeline switch arranged at the first inlet, for controlling the switching state of the first inlet, so that the state of the fluid in the enrichment pipeline can be transferred to a concentration cycle mode or a cleaning mode.

In some embodiments in the third aspect of the present disclosure, the channel-adjusting device further includes at least one adjusting pipeline and at least one second pipeline switch, so that an entrance of the cleaning solution and an exit of the waste fluid are formed in the enrichment pipeline.

In some embodiments in the third aspect of the present disclosure, the channel-adjusting device is a four-way rotary valve.

In some embodiments in the third aspect of the present disclosure, the cycle enrichment module is switched to a concentration cycle mode or a diluted mode through the channel-adjusting device, to adjust the separation efficiency of the first separation module.

In some embodiments in the third aspect of the present disclosure, the cycle enrichment module further includes an air inlet-outlet, arranged in the first section and/or the second section, for adjusting the air state or air pressure inside the enrichment pipeline.

In some embodiments in the third aspect of the present disclosure, the cycle enrichment module further includes at least one collection device, arranged in the first section and/or the second section, for adjusting the volume of the enrichment pipeline to collect the target substance, or for collecting bubbles.

In some embodiments in the third aspect of the present disclosure, the cycle enrichment module further includes at least one of a control device, a fluid storage device, a flow rate detection device, a pressure detection device, a temperature detection device, a temperature control device, an oxygen detection device, a bubble detection and elimination device, an alarm device, and a concentration detection device.

In some embodiments in the third aspect of the present disclosure, the control device generates information for adjusting the internal working state of the enrichment pipeline based on at least one of the flow rate detection device, the pressure detection device, and the concentration detection device.

In some embodiments in the third aspect of the present disclosure, the first inlet and the first outlet are connected to the same storage section. The storage section includes a fluid storage device, a container, or a human body.

In the embodiments in the third aspect of the present disclosure, the cycle enrichment module may be a hardware device for implementing the fluid treatment method described in the embodiments provided by the first aspect of the present disclosure. For example, the cycle enrichment module may be the device realizing the cycle enrichment in the fluid treatment method according to any embodiment shown in FIGs. 1 to 12.

**In** some embodiments in the third aspect of the present disclosure, the fluid treatment device includes N cascaded cycle enrichment modules, where N is a positive integer not less than 2, in any two adjacent cycle enrichment modules, the first outlet of the former cycle enrichment module is communicated with the first inlet of the latter cycle enrichment module.

**In** this example, the connection relationship, the treatment to the fluid, and the treatment effect of the different cycle enrichment modules in the fluid treatment device may refer to the embodiments provided by the first aspect of the present disclosure. For example, FIG. 9 shows a simplified schematic diagram of the cascaded cycle enrichment modules.

In some embodiments, the molecular sizes or molecular weights of the target substance, or the particle sizes of components corresponding to the N cascaded cycle enrichment modules decrease sequentially as the arranging order of the N cascaded cycle enrichment modules.

The cascaded cycle enrichment modules may be a device having multiple cascaded enrichment pipelines for the implementation of the embodiments provided by the first aspect of the present disclosure. The specific implementation manner and treatment effect may refer to the embodiments provided by the first aspect, and details will not be repeated herein.

In some embodiments in the third aspect of the present disclosure, the fluid treatment device further includes at least one cycle separation module, where the cycle separation module includes the separation pipeline, having an entrance and an exit; the second separation module, containing the second separation component, where the second separation component divides the second separation module into the first side and the second side, two opposite ends of the first side of the second separation module are communicated with at least one second inlet and at least one second outlet respectively, two opposite ends of the second side of the second separation module are communicated with the entrance of the separation pipeline and the exit of the separation pipeline respectively, and the second inlet is communicated with the first outlet of the cyclic enrichment module; and at least one second driving device, arranged at the separation pipeline, for driving the fluid in the separation pipeline to flow from the entrance of the separation pipeline to the exit of the separation pipeline at a preset flow rate, so that a total amount of the fluid in the separation pipeline are in a dynamical balance under the cycle separation mode.

The connection mode and positional relationship of the separation pipeline, the second separation module, and the second driving device is shown in FIGs. 11 to 12. The cycle separation module can form any cycle separation mode in FIGs. 11 and 12. Specifically, a part of the fluid from the second inlet can pass through the second separation component to enter the separation pipeline 21, thus forming a cycle, in addition, due to the second outlet being connected to the first side of the second separation module 22, a reversible cycle separation is achieved by the cycle separation module.

In some embodiments in the third aspect of the present disclosure, the second separation component is a porous membrane, a reverse osmosis membrane, or a gas separation membrane.

In some embodiments in the third aspect of the present disclosure, the average pore diameter or molecular weight cutoff of the porous membrane or the reverse osmosis membrane is associated with the compositions of the fluid, where the porous membrane includes a microfiltration membrane, an ultrafiltration membrane, or a nanofiltration membrane.

In some embodiments in the third aspect of the present disclosure, the second separation component includes one or more of a plate membrane, a tubular membrane, a roll membrane, a spiral membrane, and a hollow fiber membrane.

In some embodiments in the third aspect of the present disclosure, the second separation module is a tangential flow filtration module.

**In** some embodiments in the third aspect of the present disclosure, the cycle enrichment module further includes at least one of a control device, a fluid storage device, a pressure detection device, a temperature detection device, a temperature control device, an oxygen detection device, a bubble detection and elimination device, an alarm device, and a concentration detection device.

The cycle separation module may be a device realizing the cycle separation according to the embodiments provided by the first aspect of the present disclosure, so as to process the fluid under the cycle separation mode according to the first aspect of the present disclosure.

At the same time, the present disclosure further provides an embodiment where the fluid treatment device contains cascaded cycle enrichment modules and cascaded cycle separation modules. In this embodiment, the fluid is introduced to the corresponding second inlet of the cycle separation module from the first outlet of the cycle enrichment module, or the fluid is sequentially processed from the first cycle enrichment module to the last cycle enrichment module of N or more cascaded cycle enrichment modules, and the first outlet of the last cycle enrichment module is connected to the corresponding second inlet of the cycle separation module, where, N is a positive integer not less than 2.

The structure, connection relationship, and the treatment effect of the cascaded cycle enrichment modules and cycle separation modules may refer to the embodiments indicating the cascaded enrichment pipelines and separation pipelines in the first aspect of the present disclosure, and the implementation mode in FIG. 11 is an example, and details will not be repeated herein.

In some embodiments in the third aspect of the present disclosure, the fluid treatment device further includes at least one treatment module performing pretreatment or retreatment on the fluid in the fluid treatment device. The pretreatment or retreatment includes at least one of a filtration treatment, an adsorption treatment, a heating treatment, a catalytic treatment, an enrichment treatment, a concentration treatment, a chemical treatment, an optical treatment, and an electrical treatment.

The pretreatment indicates processing the initially obtained fluid and then introducing the treated fluid into the cycle enrichment module through the first inlet. The retreatment indicates processing the fluid or the target substance in the cycle enrichment module or the fluid discharged from the first outlet. The retreatment may also indicate processing the fluid from the second inlet of the cycle separation module, processing the fluid in the separation pipeline, or processing the fluid discharged from the second outlet of the cycle separation module.

In some embodiments in the third aspect of the present disclosure, the fluid treatment device is an extracorporeal cycle device. The extracorporeal cycle device includes, but is not limited to, a hemodialysis device, a plasma exchange device, an extracorporeal peritoneal dialysis device, or an extracorporeal membrane oxygenation device.

In some embodiments, the extracorporeal cycle device alone can be used as a medical device or therapeutic device, and may also be integrated into a medical device or machine involving the extracorporeal treatment of blood or other body fluids to form a new device.

The extracorporeal cycle device can be used for the selective treatment of various tissue fluids, blood, plasma, and specific components of the human body. Since the fluid treatment device has superior sustainability, generates no waste liquid, and loses no beneficial small molecules, the extracorporeal cycle device can collect specific components, change the concentrations of specific components, or remove specific components during the treatment of human tissue fluid or blood, and allow other components to be returned to the human body to form tissue fluid or extracorporeal cycle treatment of blood.

In some embodiments of the third aspect of the present disclosure, the fluid treatment device is used for disease treatment. The method for achieving disease treatment by the fluid treatment device and the corresponding feasible treatment schemes may refer to the embodiments provided by the first aspect of the present disclosure, and details will not be repeated herein.

It should be understood that the fluid treatment device provided by the third aspect of the present disclosure may be an apparatus performing the fluid treatment method described in any of the embodiments provided by the first aspect of the present disclosure. And the specific structure, connection relationship, combination mode with the treatment module, and the fluid treatment effect of the cycle enrichment module and the cycle separation module in the fluid treatment device can refer to the embodiments in the second aspect of the present disclosure.

In some scenarios, the cycle enrichment module or the cycle separation module in the fluid treatment device can be a sales unit respectively. The fluid treatment device can be a device in which the modules are connected, or a device that can be connected to other devices, (i.e. an independent cycle enrichment module or cycle separation module). For example, when the fluid treatment device includes a plurality of cycle enrichment modules and cycle separation modules, the cycle enrichment modules and the cycle separation modules can be connected in different modes to meet different processing requirements or adapt to different scene requirements, in addition, only part of the modules can optionally be connected.

The present disclosure further provides a cycle separation device for performing fluid treatment in the fourth aspect, The cycle separation device includes at least one cycle separation module. The cycle separation module includes: a separation pipeline, having an entrance and an exit; a second separation module, including a second separation component, where the second separation component divides the second separation module into a first side and a second side, two opposite ends of the first side of the second separation module are communicated with at least one second inlet and at least one second outlet respectively, and two opposite ends of the second side of the second separation module are communicated with the inlet of the separation pipeline and the outlet of the separation pipeline respectively; and at least one second driving device, arranged at the separation pipeline, for driving the fluid in the separation pipeline to flow from the entrance of the separation pipeline to the exit of the separation pipeline at a preset flow rate, so that a dynamical balance of the total amount of the fluid in the separation pipeline can be formed under the cycle separation mode.

FIG. 12 shows a simplified schematic diagram of the cycle separation module according to an embodiment. In the cycle separation module, a part of the fluid from the second inlet can flow through the second separation component and then enter the separation line 21 to form a cycle, and the second outlet is connected to the first side of the second separation module 22, therefore, the reversible cycle separation can be realized in the cycle separation module.

In some embodiments of the fourth aspect of the present disclosure, the fluid contains the target substance, and the fluid includes one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, methane, light distillate oil, and liquefied gas.

In some embodiments of the fourth aspect of the present disclosure, the second separation component is a porous membrane, a reverse osmosis membrane, or a gas separation membrane.

In some embodiments of the fourth aspect of the present disclosure, the average pore diameter or molecular weight cutoff of the porous membrane or the reverse osmosis membrane is associated with the compositions of the fluid, and the porous membrane includes a microfiltration membrane, an ultrafiltration membrane, or a nanofiltration membrane.

In some embodiments of the fourth aspect of the present disclosure, the second separation component includes one or more of a plate membrane, a tubular membrane, a roll membrane, a spiral membrane, and a hollow fiber membrane.

In some embodiments of the fourth aspect of the present disclosure, the second separation module is a tangential flow filtration module.

In some embodiments of the fourth aspect of the present disclosure, the cycle separation module further includes at least one of a control device, a fluid storage device, a pressure detection device, a temperature detection device, a temperature control device, a bubble detection and elimination device, an alarm device, and a concentration detection device.

In some embodiments of the fourth aspect of the present disclosure, the cycle separation device further includes at least one treatment module, for performing pretreatment or retreatment on the fluid. The pretreatment or retreatment includes at least one of filtration, adsorption, heating, catalysis, enrichment, concentration, a chemical treatment, an optical treatment, and an electrical treatment. In practical applications, the treatment module may be an adsorption device, an extraction device, an ion exchange processing device, a centrifugal device, a filtration device, a heating device, etc.

In some embodiments of the fourth aspect of the present disclosure, the treatment module may be arranged in the separation pipeline to adjust the flow direction or flow channel of the separated fluid, that is, the treatment module may also be a channel-adjusting device. In some examples, the treatment module in the cycle separation module may also be a collection device.

The specific structure and the connection mode of the separation pipeline, the second separation module, and the second driving device in the cycle separation module, and other modules or hardware (such as the treatment module) that can be incorporated into the cycle separation module may refer to the embodiments provided in the second aspect of the present disclosure, details will not be repeated herein.

The cycle separation module in the cycle separation device provided by the fourth aspect of the present disclosure may be used for implementing the cycle separation in any one of the embodiments provided by the second aspect of the present disclosure, and correspondingly, the treatment effect in the embodiment provided by the second aspect of the present disclosure may be realized.

In some embodiments of the fourth aspect of the present disclosure, the cycle separation device includes N cascaded cycle separation modules, where N is a positive integer not less than 2. In any two adjacent cycle separation modules, the separation pipeline of the former cycle separation module is communicated with the second inlet and the second outlet of the latter cycle separation module.

In some embodiments of the fourth aspect of the present disclosure, the molecular size or molecular weight of the target substance, or the particle sizes of components decrease sequentially from the first cycle separation module to the Nₜₕ cycle separation module of the N cascaded cycle separation modules.

In an embodiment in which the cycle separation device includes N cascaded cycle separation modules, the structure, connection relationship, and fluid treatment manner of the N cascaded cycle separation modules may refer to the embodiment including multiple cascaded separation pipelines provided in the second aspect of the present disclosure, for example, the implementation schemes shown in FIG. 14.

In some embodiments of the fourth aspect of the present disclosure, the cycle separation device further includes at least one cycle enrichment module. The cycle enrichment module includes: an enrichment pipeline, having a first section and a second section, the entrance of the first section is connected to the first inlet, the exit of the second section is connected to the first section; at least one first separation module connected to the exit of the first section, the first separation module includes the first separation component dividing the first separation module into the first side and the second side, the first side communicates with the entrance of the second section, the second side communicates with at least one first outlets, and the first inlet is connected to the separation pipeline of the cycle separation module; and at least one first driving device, arranged in the first section, for driving the fluid in the enrichment pipeline to flow circularly, so that a dynamic balance of the total amount of fluid in the enrichment pipeline is controlled and the first separation module enriches the target substance under the enrichment cycle mode.

FIG. 2 shows a simplified schematic diagram of the cycle enrichment module according to an embodiment. The cycle enrichment module includes the enrichment pipeline 11, having the first section 111 and the second section 112, where the entrance of the first section 111 is communicated with the first inlet, and the exit of the second section 112 is communicated with the first section.

The first section 111 and the second section 112 of the enrichment pipeline 11 can be connected to each other in a certain way, so that the liquid flow circularly in the enrichment pipeline 11. The exit of the second section 112 is communicated with the first section 111, therefore, after entering the first section 111 from the first inlet, the fluid can flow to the second section 112 and then return to the first section 111 from the exit of the second section 112, thus forming a flow cycle.

The at least one first separation module 12 includes the first separation component dividing the first separation module 12 into the first side and the second side, where the first side is communicated with the entrance of the first section and the exit of the second section, and the second side is communicated with at least one first outlet.

The fourth aspect of the present disclosure further provides an embodiment of a cycle separation device containing cascaded cycle separation modules and cycle enrichment modules. The structure, connection mode, and treatment manner of the cycle separation module and the cycle enrichment module may refer to the embodiment including cascaded separation pipelines and enrichment pipelines provided in the second aspect of the present disclosure, for example, the implementation schemes as shown in FIGs. 15a and 15b.

In some embodiments of the fourth aspect of the present disclosure, the first separation component is a porous membrane, a reverse osmosis membrane, or a gas separation membrane.

In some embodiments of the fourth aspect of the present disclosure, the average pore diameter or molecular weight cutoff of the porous membrane or the reverse osmosis membrane is associated with the target substance, and the porous membrane includes a microfiltration membrane, an ultrafiltration membrane, or a nanofiltration membrane.

In some embodiments of the fourth aspect of the present disclosure, the first separation component includes one or more of a planar membrane, a tubular membrane, a roll membrane, a spiral membrane, and a hollow fiber membrane.

In some embodiments of the fourth aspect of the present disclosure, the first separation module is a tangential flow filtration module.

In some embodiments of the fourth aspect of the present disclosure, the at least one first driving device controls the flow rate of the fluid in the first section to be greater than a preset threshold, so that the target substance in the first separation module flows from the exit of the first section to the entrance of the second section.

In some embodiments of the fourth aspect of the present disclosure, the preset threshold is determined by at least one of the following: the composition of the fluid, the temperature of the fluid, the structure of the first separation component, the material of the first separation component, the cavity structure of the first separation module, and the diameter of the enrichment pipeline.

In some embodiments of the fourth aspect of the present disclosure, the second section is further provided with the first driving device.

In some embodiments of the fourth aspect of the present disclosure, in the cycle enrichment mode of the cycle enrichment module, the total volume or total velocity of the fluid introduced into the enrichment pipeline from the first inlet is equal to that of the fluid discharged from the enrichment pipeline through the first outlet.

In some embodiments of the fourth aspect of the present disclosure, the cycle enrichment module controls the ratio of the total volume of the fluid introduced into the enrichment pipeline per unit time to the total volume of the fluid in the enrichment pipeline to adjust the enrichment efficiency.

In some embodiments of the fourth aspect of the present disclosure, the cycle enrichment module further includes a channel-adjusting device, arranged in the first section or/and the second section, for adjusting the flow direction of the fluid in the enrichment pipeline. The channel-adjusting device adjusts the flow direction of the fluid in the enrichment pipeline by at least one of the following ways: adjusting the switching state of the first inlet and/or the first outlet; and adjusting the switching state of at least one adjusting channel connected to the enrichment pipeline.

In some embodiments of the fourth aspect of the present disclosure, the channel-adjusting device includes the first pipeline switch, arranged at the first inlet, for controlling the switching state of the first inlet to further control the treatment state of the fluid in the enrichment pipeline to be a concentration cycle mode or a cleaning mode.

In some embodiments of the fourth aspect of the present disclosure, the channel-adjusting device includes at least one adjusting pipeline and at least one second pipeline switch to form the entrance of the cleaning solution and the exit of the waste fluid in the enrichment pipeline.

In some embodiments of the fourth aspect of the present disclosure, the channel-adjusting device is a four-way rotary valve.

In some embodiments of the fourth aspect of the present disclosure, the enrichment pipeline is switched to the concentration cycle mode or the diluted mode through the channel-adjusting device, to adjust the separation efficiency of the first separation module.

In some embodiments of the fourth aspect of the present disclosure, the cycle enrichment module further includes an air inlet-outlet, arranged at the first section or/and the second section, for adjusting the air state inside the enrichment pipeline and the air pressure inside the enrichment pipeline.

In some embodiments of the fourth aspect of the present disclosure, the cycle enrichment module further includes at least one collection device, arranged in the first section and/or the second section, for adjusting the volume of the enrichment pipeline to collect the target substance, or for collecting bubbles.

In some embodiments of the fourth aspect of the present disclosure, the cycle enrichment module further includes at least one of a control device, a fluid storage device, a pressure detection device, a temperature detection device, a temperature control device, a bubble detection and elimination device, an alarm device, and a concentration detection device.

In some embodiments of the fourth aspect of the present disclosure, the cycle enrichment module further includes a flow rate detection device. The flow rate detection device may detect the dynamic state inside the enrichment pipeline, so as to form adjustment information for the working state inside the enrichment pipeline.

The cycle enrichment module in the cycle separation device provided by the fourth aspect of the present disclosure may be used for implementing the cycle enrichment in any one of the embodiments provided by the second aspect of the present disclosure and realize the treatment effect in the embodiment provided by the second aspect of the present disclosure, and details will not be repeated herein.

In some embodiments of the fourth aspect of the present disclosure, the cycle separation device further includes at least one treatment module, for performing pretreatment or retreatment on the fluid in the cycle separation device. The pretreatment or retreatment includes at least one of a filtration, an adsorption, a heating, a catalysis, an enrichment, a concentration, a chemical treatment, an optical treatment, and an electrical treatment. In practical applications, the treatment module include adsorption devices, extraction devices, ion exchange treatment devices, centrifugal devices, filtration devices, heating devices, etc.

The pretreatment indicates processing the initially obtained fluid and then introducing the treated fluid into the cycle separation module through the second inlet. The retreatment indicates processing the separated fluid in the clinic separation module or the fluid discharged from the second outlet. The retreatment may also indicate processing the fluid at the first inlet of the cycle enrichment module, processing the fluid in the enrichment pipeline, or processing the fluid discharged from the first outlet of the cycle enrichment module.

In some embodiments of the fourth aspect of the present disclosure, the cycle separation device is an extracorporeal cycle device. The extracorporeal cycle device may be a blood purification device, a plasma exchange device, an extracorporeal peritoneal dialysis device, or an extracorporeal membrane oxygenation device. It should be understood that the extracorporeal cycle device is not limited to the above.

In some embodiments, the cycle separation device alone can be used as a medical device or therapeutic device, and may also be integrated into a medical device or machine (involving the extracorporeal treatment of blood or other body fluids) to form a new device.

In some embodiments of the fourth aspect of the present disclosure, the cycle separation device can be used for disease treatment, for example, by the enrichment and selective removal of target molecules. The way for the cycle separation device to realize the disease treatment and the disease that can utilize the cycle separation device can refer to the embodiments provided in the second aspect of the present disclosure, therefore, details will not be repeated herein.

It should be understood that the cycle separation device provided by the fourth aspect of the present disclosure may be used for performing the fluid treatment method described in any one of the embodiments provided by the second aspect of the present disclosure. And the specific structure, connection relationship, combination mode, and fluid treatment effect of the cycle separation module and the cycle enrichment module may refer to the embodiments provided in the second aspect of the present disclosure.

In some scenarios, the cycle separation module or the cycle enrichment module in the cycle separation device can be a sales unit respectively. The cycle separation device can be a device in which the modules are connected, or a device (i.e. an independent cycle enrichment module or cycle separation module) that can be connected to other devices. For example, when the fluid treatment device includes a plurality of cycle enrichment modules and cycle separation modules, the cycle enrichment modules and the cycle separation modules can be connected in different modes to meet different processing requirements or adapt to different scene requirements, in addition, only part of the modules can optionally be connected.

The present disclosure further provides a cycle treatment system in the fifth aspect. The cycle treatment system includes at least one fluid treatment device as described in any embodiment of the third aspect of the present disclosure or/and at least one cycle separation device as described in any embodiment of the fourth aspect of the present disclosure, and a pipeline system, where the pipeline system includes a fluid-introducing pipeline and a fluid-returning pipeline.

The cycle treatment system includes any of the following:
Example A1, the cycle treatment system includes one fluid treatment device and the pipeline system; Example A2, the cycle treatment system includes two or more fluid treatment devices and the pipeline system.
Example B1, the cycle treatment system includes one cycle separation device and the pipeline system; Example B2, the cycle treatment system includes two or more cycle separation devices and the pipeline system.
Example C, the cycle treatment system includes at least one fluid treatment device, at least one cycle separation device, and the pipeline system.

In the above-mentioned Examples A2, B2, and C, the fluid treatment device or/and the cycle separation device can be connected in series or parallel. The fluid treatment device or/and the cycle separation device connected in series indicates the fluid treatment device or/and the cycle separation device are connected sequentially. For example, when two fluid treatment devices are connected in series, the fluid undergoing the cyclic treatment of the first fluid treatment device flows to the second fluid treatment device. The fluid treatment device or/and the cycle separation device connected in parallel indicates the inlets of different equipment or devices can be connected to the same pipeline, container, or module, or the fluids processed by different fluid treatment devices or cycle separation devices can be connected to the same pipeline, container, or module. The parallel connection of the fluid treatment devices may refer to that of the electrical components.

It should be understood that the fluid treatment device and the cycle separation device can be used to collect or process specific components in the fluid, thus the content or concentration of the specific components in the fluid is changed, and changes of the different fluid compositions can be achieved by changing the connection between the fluid treatment device or/and the cycle separation device.

In some embodiments of the fifth aspect of the present disclosure, the pipeline system further includes at least one of an anticoagulation system, a fluid storage device, a control device, a flow rate detection device, a pressure detection device, a temperature detection device, a temperature control device, an oxygen detection device, a bubble detection and elimination device, an alarm device, and a concentration detection device.

FIG. 18 shows a simplified schematic diagram of the cycle treatment system according to an embodiment of the present disclosure.

The pipeline system can optionally include the above-mentioned devices. It should be understood that the pipeline system is mainly used for directing the fluid. The above-mentioned devices can be arranged in the pipeline system according to actual needs in different application scenarios. For example, in the embodiment shown in FIG. 18, when the cycle treatment system is a human extracorporeal cycle device, the pipeline system is used for blood transportation, and a bubble detection and elimination device 31, an air capturing device 32, a pressure detection device 33, and an anticoagulation system 34 may typically be provided in the pipeline system.

In some embodiments, the anticoagulation system 34 is coupled to the pipeline system, for example, when the pipeline system is used for blood delivery, after adding coagulative inhibitors (such as heparin, tissue plasminogen activator, antithrombin, etc.) to the pipeline system, the coagulative inhibitors flow with the fluid in the pipeline system, thus the pipeline system turns into the anticoagulation system.

The bubble detection and elimination device 31 can be used to remove the bubbles in the fluid before entering the fluid treatment device or the cycle separation device or in the treated fluid discharged from the fluid treatment device or the cycle separation device. For example, in a medical scenario, the bubbles in the treated fluid (such as blood) need to be removed before being introduced back into the body.

FIG. 19 shows a simplified schematic diagram of a cycle treatment system according to another embodiment of the present disclosure.

The cycle treatment system includes the pipeline system and the fluid treatment device. The pipeline system includes the fluid-introducing pipeline and the fluid-returning pipeline. In the example shown in FIG. 19, the fluid treatment device may be the cycle enrichment module. The fluid-introducing pipeline is connected to the treatment module, and the treatment module can be a separation device, such as a tangential flow filtration module or a centrifugal device. A part of the fluid separated from the separation device flows to the fluid treatment device for being processed, the fluid discharged from the first outlet flows to the downstream of the separation device, therefore, the other part of the fluid not separated by the separation device and the fluid discharged from the first outlet may converge and then enter the fluid-returning pipeline.

When the cycle treatment system is applied for medical treatment, the fluid-introducing pipeline introduces whole blood from the human body, and the separation device separates the whole blood into a cell part and a plasma part, the cell part as a retention solution is transferred to the fluid-returning pipeline, and the plasma part is transferred to the first inlet of the fluid treatment device. Specific components such as low-density lipoprotein in the plasma part are enriched in the fluid treatment device, and the filtrate from the fluid treatment device is transferred to the fluid-returning pipeline, thus, the directional removal (enrichment) of low-density lipoprotein in whole blood is realized by converging the filtrate and the cell part. In some implementations, the cell part and the filtrate of the plasma part are optionally transferred to a collection device that is connected to the fluid-returning pipeline. In some embodiments, the separation device may effectively filter out the target molecule in the plasma, and the fluid treatment device may effectively enrich the target molecule to realize an enrichment (and removal) of any target molecule in the plasma.

In some implementations, the pipeline system is optionally provided with a collection device, and the pipeline system may also be provided with an anticoagulation system 34, a pressure detection system 33, a control device, a driving device, a bubble detection and elimination device 31, etc.

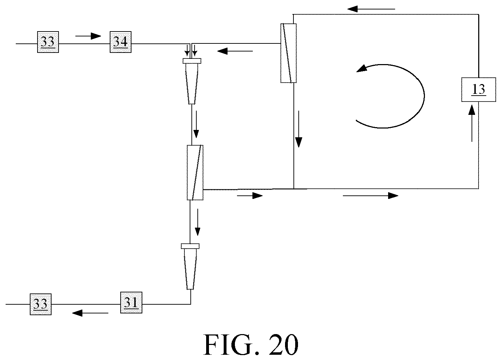

FIG. 20 shows a simplified schematic diagram of the cycle treatment system according to another embodiment of the present disclosure.

Specifically, the cycle treatment system includes the pipeline system and the fluid treatment device. The pipeline system includes the fluid-introducing pipeline and the fluid-returning pipeline. In this embodiment, the fluid treatment device may be the cycle enrichment module. The fluid-introducing pipeline is connected to the treatment module (such as a filtration device or a separation device). The fluid entering the treatment module is separated into a retention fluid and a filtration fluid, where the filtration fluid filtered out or separated by the treatment module flows to the first inlet of the cycle enrichment module, and the fluid processed by the cycle enrichment module is transferred to the upstream of the treatment module through the first outlet (i.e. the fluid processed by the cycle enrichment module returns to the treatment module for further treatment). The retention fluid in the treatment module flows to the fluid-returning pipeline.

When the cycle treatment system is applied for medical treatment, the cycle treatment system may be an extracorporeal cycle system. The fluid-introducing pipeline and the fluid-retuning pipeline introduce blood from the human body and return the treated blood to the human body, respectively. The treatment module may be a filtration module and separates out part of the whole blood, such as a plasma part. The plasma part is transferred to the first inlet of the fluid treatment device through pipelines, and the filtrated plasma treated by the fluid treatment device flows out the first outlet and enters the upstream of the filtration module to flow in accordance with the flowing direction. Afterward, the filtrated plasma is re-processed by the filtration module, and renewed whole blood is obtained in a branch of the filtration module. Finally, the renewed whole blood is transferred back to the human body through the fluid-returning pipeline. In this embodiment, the cycle treatment of the plasma is realized by the fluid treatment device, and the filtration module can filter the plasma circularly by setting the connection mode of the fluid treatment device and the treatment module.

In some implementations, the pipeline system is optionally provided with a collection device, and the pipeline system may also be provided with an anticoagulation system 34, a pressure detection system 33, a control device, a driving device, a bubble detection and elimination device 31, etc.

The control device (not shown in the drawing) can control the temperature control device, the bubble detection and elimination device, the alarm device, etc. in the pipeline system, in addition, the control device can receive detection information such as temperature information, oxygen information in the pipeline, pressure information, etc., so as to control the working status (such as on or off state, operating mode, operating power, etc.) of each device in the pipeline system based on the detection information.

The pipeline system includes the fluid-introducing pipeline and the fluid-returning pipeline. In some embodiments, the fluid-introducing pipeline and the fluid-returning pipeline may be connected to the same container, storage space, or human body, for example, the fluid-introducing pipeline introduces blood from the human body to the fluid treatment device or the cycle separation device, and the fluid-returning pipeline returns the treated blood back to the human body.

In some examples, the material of the pipeline in the pipeline system is adaptable to the fluid. For example, the pipeline system can be a special blood pipeline, a special peristaltic pump pipeline, a disposable hemodialysis blood pipe, a high-pressure sterilized silicone pipe, a special corrosive liquid pipeline, a high biocompatible pipeline, etc. When the fluid treatment method is applied in medical scenarios, the pipeline system is, for example, a blood delivery pipeline or a liquid medicine delivery pipeline, and the material of the pipeline includes, but is not limited to, soft polyvinyl chloride plastic, high-performance polyolefin thermoplastic elastomer (TPE), nano-biomedical material, and resin material.

In some embodiments of the fifth aspect of the present disclosure, the cycle treatment system is an extracorporeal cycle clearance system, an extracorporeal enrichment clearance device, a hemodialysis device, a plasma exchange device, an extracorporeal peritoneal dialysis device, or an extracorporeal membrane oxygenation device. In other embodiments, the cycle treatment system can also be used as a module being integrated into other extracorporeal cycle equipment, such as artificial liver, artificial kidney, hemodialysis device, peritoneal dialysis device, plasma exchange device, plasma purification device, blood lipid purification device, molecular adsorbent recirculating system, extracorporeal membrane oxygenation device, leukocyte depletion device, extracorporeal cycle life support system, etc.

In some embodiments, the cycle treatment system alone may be a medical device or therapeutic device, or may be integrated into a medical device or machine involving the extracorporeal treatment of blood or other body fluids to form a new device.

In some embodiments of the fifth aspect of the present disclosure, the cycle treatment system is used for at least one of cell collection, microbial collection, and material collection.

It should be understood that both the fluid treatment device and the cycle separation device in the cycle treatment system can realize the collection of specific components where the specific components can be cells, microorganisms, or other materials in some scenarios.

In some practical applications, each fluid treatment device or cycle separation device alone can be an independent sales unit. Different connection modes between the fluid treatment device and the cycle separation device can result in different fluid treatment effects. The cycle treatment system with a specific connection relationship can also be a sales unit. In addition, the cycle treatment system can be a system in which the components have been connected, or can be each independent component (such as the fluid treatment device) that can be connected to other components. In some embodiments, the system in which the components are connected may also include an integrated shell or a module shape. For example, an interface is set outside the shell and the fluid treatment device and the cycle separation device are arranged inside the shell. The shell can be switched between the on and off state, and the fluid is introduced into the cycle treatment system when the shell is in the on state.

The present disclosure further provides a medical device in the sixth aspect. The medical device includes the cycle treatment system as described in the embodiment provided by the fifth aspect of the present disclosure.

The medical device may be used for medical purposes. And the medical device may be an instrument, device, apparatus, material, or other articles used alone or in combination for the human body, and may also include required software. The medical device may be used for the prevention, diagnosis, treatment, monitoring, mitigation, and compensation of diseases.

In an embodiment provided by the present disclosure, the medical device is, for example, an extracorporeal cycle clearance system, an extracorporeal enrichment clearance device, a hemodialysis device, a plasma exchange device, an extracorporeal peritoneal dialysis device, or an extracorporeal membrane oxygenation device. In other embodiments, the medical device may also be a component module that can be integrated into other extracorporeal cycle devices, such as an artificial liver, an artificial kidney, a hemodialysis device, a peritoneal dialysis device, a plasma exchange device, a plasma purification device, a blood lipid purification device, a molecular adsorbent recirculating system, an extracorporeal membrane oxygenation device, a leukocyte depletion device, an extracorporeal cycle life support system, etc.

In some embodiments, the medical device alone may be a medical device or therapeutic device, or the medical device may be integrated into other medical devices or machines involving the extracorporeal treatment of blood or other body fluids to form a new device.

When the cycle treatment system of the fifth aspect of the present disclosure is used for medical purposes, it can be regarded as a medical device.

The medical purposes include the treatment of the fluids from the human body, and further include the treatment of medicines, pharmaceutical water, medical water, etc. For example, tap water can be processed by the cycle treatment system to obtain diluted water that can be used as concentrated dialysate.

In some embodiments of the sixth aspect of the present disclosure, the medical device achieves disease treatment by selectively altering the concentration of specific molecules or combinations of molecules. These specific molecules or combinations of molecules are often pathogenic factors or outcomes after the development of the disease, or are essential to the development of the disease or the maintenance of health. Controlling the concentration of target molecules is beneficial to the treatment of the disease or the control of the complications, where the disease includes, but is not limited to, at least one of familial hypercholesterolemia, hyperlipoproteinemia, systemic lupus erythematosus, autoimmune diseases, myasthenia gravis, rapidly progressive glomerulonephritis, fatty liver, liver cirrhosis, acute liver failure, hyperlipidemia, severe acute pancreatitis, sepsis, Guillain-Barré syndrome, obesity, phenylketonuria, mucopolysaccharidosis, hypophosphatasia, hereditary hyperammonemia, hyperuricemia, gout, hyperglycemia, uroketosis, and diabetes.

In embodiments provided in the present disclosure, the treatment includes a preventive (i.e., prophylactic), curative, or palliative treatment that can lead to the desired physiological effect. In addition, the term "treatment" herein refers to the purpose of partially or completely reducing, delaying the occurrence, inhibiting the progression, reducing the severity, and/or reducing the probability of one or more symptoms of a specific disease, abnormality, and/or medical condition.

It should be understood that in clinical applications, the medical device of the present disclosure can be used to change the concentration of specific molecules or molecular combinations in the patient's body. And considering different disease types, the medical intervention required for the patient may include other means, for example, for patients with renal insufficiency, the medical device of the present disclosure can eliminate the accumulated pathogenic factors and metabolic waste in the patient's body, at the same time, drugs and other treatment methods such as surgery, diet nursing, etc. can be used in combination with the present disclosure due to complications or comorbidities accompanying the renal insufficiency, such as anemia, pyelonephritis, urinary tract diseases, etc.

In some embodiments, the medical device may be used in combination with a drug for the treatment of a disease. For example, changing the concentration of specific molecules or combination of molecules in a patient's body through the medical device of the present disclosure, such as reducing the concentration of specific molecules or generating specific molecules, and at the same time, using drug therapy in combination with the medical device which is used for the patient's disease treatment, and the drug is, for example, a drug for disease complications, an anticoagulant drug for plasma exchange, a vasoactive drug for the disease itself, an anti-infective drug and other disease treatment-related drugs. The treatment device described in the present disclosure and drugs can independently treat the disease or be beneficial to health, and the two can work together to realize a therapeutic effect or achieve a better therapeutic effect.

The present disclosure further provides a computer-readable storage medium storing at least one program in the seventh aspect. When the at least one program is invoked by the processor, the at least one program executes and realizes the fluid treatment method described in any one of the embodiments provided in the first aspect or the second aspect of the present disclosure.

If the function is implemented in the form of a software function unit and is sold or used as an individual product, it can be stored in a computer-readable storage medium. Based on the above understanding, a part of the technical solution of the present disclosure that essentially contributes to the prior art or a part of the technical solution may be in the form of a software product. The software product is stored in a storage medium and includes several instructions to enable a computer device (it may be a personal computer, a server, or a network device, etc.) to perform all or part of the steps of the method described in the embodiments of the present disclosure.

In an embodiment provided by the present disclosure, the computer-readable storage medium may include a read-only memory, a random-access memory, an EEPROM, a CD-ROM, or other optical disk memory, a magnetic disk memory, or other magnetic storage device, a flash memory, a USB flash disk, a mobile hard disk, or any other medium that can be used to store desired program code having the form of instructions or data structures and can be accessed by a computer. In addition, any connection can be appropriately referred to as a computer-readable medium. For example, if the instruction is sent from a website, a server, or other remote source using coaxial cable, optical fiber and cable, twisted-pair cable, digital subscriber line (DSL), or wireless technology such as infrared ray, radio, and microwave, then the coaxial cable, optical fiber and cable, twisted-pair cable, DSL, or wireless technology such as infrared ray, radio, and microwave are included in the definition of the medium. Furthermore, it should be understood that the computer readable and writable storage media and data storage media do not include connections, carrier waveforms, signals, or other transient media, but are intended to aim at non-transient, tangible storage media. Magnetic disks and optical disks used in the present disclosure include compact disc (CD), laser disc, optical disc, digital versatile disc (DVD), floppy disc, and Blu-ray disc, in which the magnetic disks typically copy data magnetically, and the optical disks copy data optically with a laser.

In one or more exemplary aspects, the functions of the computer program for the fluid treatment method described in the first aspect or the second aspect of the present disclosure may be implemented in the way of hardware, software, firmware, or any combination thereof. When the functions of the computer program for the fluid treatment method are implemented in the way of software, these functions can be stored or transmitted to the computer-readable media as one or more instructions or codes. The steps of the method or algorithm disclosed in this disclosure may be embodied as a processor-executable software module, where the processor-executable software module may be on a tangible, non-temporary computer readable and writable storage medium. The tangible, non-temporary computer readable and writable storage media can be any available media that a computer can access.

The flowcharts and block diagrams in the above-mentioned drawings of the present disclosure illustrate the architectures, functions, and operations of possible implementations of systems, methods, and computer program products in accordance with various embodiments of the present disclosure. Each box in the flowchart or block diagram may represent a module, a program segment, or a part of codes that contains one or more executable instructions for implementing the specified logical function. It should be noted that in some alternative implementations, the functions marked in the box can also be achieved in a different order other than those marked in the drawings. For example, two boxes represented in succession can actually be executed substantially in parallel, and depending on the function involved, they can sometimes be executed in reverse order. It should also be noted that each box in the block diagram and/or flow chart, as well as the combination of the boxes in the block diagram and/or flow chart, can be implemented by a dedicated hardware-based system that performs specified functions or operations, or can be implemented by a combination of dedicated hardware and computer instructions.

The above embodiments only illustrate the principles and efficacy of the present disclosure, and are not intended to limit the disclosure. Those skilled can make modifications or changes to the above-mentioned embodiments without going against the spirit and the scope of the present disclosure. Therefore, all equivalent modifications or changes made by those who have common knowledge in the art without departing from the spirit and technical concept disclosed by the present disclosure shall be still covered by the claims of the present disclosure.

## Claims

1. A fluid treatment method, comprising:
introducing a fluid into an enrichment pipeline; wherein the enrichment pipeline comprises a first section and a second section, an entrance of the first section is communicated with at least one first inlet, and an exit of the second section is communicated with the entrance of the first section;
intercepting a target substance in the fluid by at least one first separation module; wherein each of the at least one first separation module comprises a first separation component to divide a corresponding first separation module into a first side and a second side, two opposite ends of the first side are communicated with an exit of the first section and an entrance of the second section respectively, and the second side is communicated with at least one first outlet; and
driving the fluid in the enrichment pipeline to cyclically flow at a first preset flow rate based on at least one first driving device to enrich the target substance; wherein the at least one first driving device is arranged at the first section and is configured to dynamically balance a total amount of the fluid in the enrichment pipeline.

2. The fluid treatment method according to claim 1, wherein the fluid introduced into the enrichment pipeline contains the target substance, and the fluid comprises one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, methane, light distillate oil, and liquefied gas.

3. The fluid treatment method according to claim 1, wherein the first separation component is a porous membrane, a reverse osmosis membrane, or a gas separation membrane.

4. The fluid treatment method according to claim 3, wherein the porous membrane or the reverse osmosis membrane has an average pore diameter or a molecular weight cutoff associated with the target substance; wherein the porous membrane comprises a microfiltration membrane, an ultrafiltration membrane, or a nanofiltration membrane.

5. The fluid treatment method according to claim 3, wherein the first separation component comprises one or more of a planar membrane, a tubular membrane, a roll membrane, a spiral membrane, and a hollow fiber membrane.

6. The fluid treatment method according to claim 3, wherein each of the at least one first separation module is a tangential flow filtration module.

7. The fluid treatment method according to claim 6, wherein the at least one first driving device controls a flow rate in the first section to be above a preset threshold, so that the target substance flows from the exit of the first section to the entrance of the second section through the at least one first separation module.

8. The fluid treatment method according to claim 7, wherein the preset threshold is determined based on at least one of a composition of the fluid, a temperature of the fluid, a structure of the first separation component, a material of the first separation component, a cavity structure of the at least one first separation module, and a diameter of the enrichment pipeline.

9. The fluid treatment method according to claim 1, wherein the second section is further provided with one of the at least one first driving device.

10. The fluid treatment method according to claim 1, wherein a total volume or total velocity of the fluid introduced into the enrichment pipeline from the at least one first inlet is equal to that of a fluid discharged from the enrichment pipeline by the at least one first outlet to cyclically enrich the target substance.

11. The fluid treatment method according to claim 10, wherein an enrichment efficiency of the fluid treatment method is adjusted by controlling a ratio of the total volume of the fluid introduced into the enrichment pipeline per unit time to a total volume of a fluid in the enrichment pipeline.

12. The fluid treatment method according to claim 1, wherein the enrichment pipeline is further provided with a channel-adjusting device for adjusting a flow direction of a fluid in the enrichment pipeline; wherein the channel-adjusting device adjusts the flow direction of the fluid in the enrichment pipeline by at least one of the following ways:
adjusting the flow direction of the fluid in the enrichment pipeline by adjusting a switching state of the at least one first inlet and/or the at least one first outlet; and
adjusting the flow direction of the fluid in the enrichment pipeline by adjusting a switching state of at least one adjusting channel connected to the enrichment pipeline.

13. The fluid treatment method according to claim 12, wherein the channel-adjusting device comprises a first pipeline switch arranged at each of the at least one first inlet, for controlling a switching state of the corresponding first inlet to further control a treatment state of the fluid in the enrichment pipeline to be a concentration cycle mode or a cleaning mode.

14. The fluid treatment method according to claim 13, wherein the channel-adjusting device further comprises at least one adjusting pipeline and at least one second pipeline switch arranged at the at least one adjusting pipeline so that an entrance of a cleaning solution and an exit of a waste fluid are formed in the enrichment pipeline.

15. The fluid treatment method according to claim 12, wherein the channel-adjusting device is a four-way rotary valve.

16. The fluid treatment method according to claim 12, wherein the enrichment pipeline is switched to a concentration cycle mode or a dilution mode through the channel-adjusting device, to adjust a separation efficiency of the at least one first separation module.

17. The fluid treatment method according to claim 1, wherein the enrichment pipeline further comprises an air inlet-outlet, arranged at the first section and/or the second section, for adjusting an air pressure inside the enrichment pipeline.

18. The fluid treatment method according to claim 1 or 17, wherein the enrichment pipeline further comprises at least one collection device, arranged at the first section and/or the second section, for adjusting a volume of the enrichment pipeline to collect the target substance, or for collecting bubbles.

19. The fluid treatment method according to claim 1, wherein the enrichment pipeline further comprises at least one of a control device, a fluid storage device, a pressure detection device, a temperature detection device, a temperature control device, an oxygen detection device, a bubble detection and elimination device, an alarm device, a flow rate detection device, and a concentration detection device.

20. The fluid treatment method according to claim 1, wherein the control device generates information for adjusting an internal working state of the enrichment pipeline based on at least one of the flow rate detection device, the pressure detection device, and the concentration detection device.

21. The fluid treatment method according to claim 1, wherein the at least one first inlet and the at least one first outlet are connected to a same storage section; wherein the storage section comprises a fluid storage device, a container, or a human body.

22. The fluid treatment method according to claim 1, wherein N enrichment pipelines are cascaded to further enrich the target substance, and the N enrichment pipelines comprise a first-stage enrichment pipeline and a second-stage enrichment pipeline adjacent to the first-stage enrichment pipeline, and the method further comprises: introducing a fluid discharged from at least one first outlet of the first-stage enrichment pipeline into at least one first inlet of the second-stage enrichment pipeline, wherein N is a positive integer not less than 2.

23. The fluid treatment method according to claim 22, wherein a molecular size or a molecular weight of the target substance, or particle sizes of components of the target substance as enriched by each of the N cascaded enrichment pipelines, decreases as enrichment sequentially proceeds in the N cascaded enrichment pipelines.

24. The fluid treatment method according to claim 1, comprising:
introducing a fluid discharged from the at least one first outlet of the enrichment pipeline into a separation pipeline through at least one second separation module; wherein the separation pipeline has an entrance and an exit; each of the at least one second separation module comprises a second separation component to divide a corresponding second separation module into a first side and a second side, two opposite ends of the first side of each of the at least one second separation module are communicated with at least one second inlet and at least one second outlet respectively, two opposite ends of the second side of each of the at least one second separation module are communicated with the entrance and exit of the separation pipeline respectively, and the at least one second inlet is communicated with the at least one first outlet; and
driving a fluid in the separation pipeline to flow from the entrance of the separation pipeline to the exit of the separation pipeline at a second preset flow rate by at least one second driving device, to dynamically balance a total amount of the fluid in the separation pipeline.

25. The fluid treatment method according to claim 24, wherein the second separation component is a porous membrane, a reverse osmosis membrane, or a gas separation membrane.

26. The fluid treatment method according to claim 25, wherein the porous membrane or the reverse osmosis membrane has an average pore diameter or a molecular weight cutoff associated with compositions of the fluid; wherein the porous membrane comprises a microfiltration membrane, an ultrafiltration membrane, or a nanofiltration membrane.

27. The fluid treatment method according to claim 24, wherein the second separation component comprises one or more of a plate membrane, a tubular membrane, a roll membrane, a spiral membrane, and a hollow fiber membrane.

28. The fluid treatment method according to claim 24, wherein each of the at least one second separation module is a tangential flow filtration module.

29. The fluid treatment method according to claim 24, wherein the separation pipeline is further provided with at least one of a control device, a storage device, a collection device, a pressure detection device, a temperature detection device, a temperature control device, a bubble detection and elimination device, an alarm device, a flow rate detection device, and a concentration detection device.

30. The fluid treatment method according to claim 1, or 22, or 24, further comprising: performing pretreatment or retreatment on the fluid by at least one treatment module; wherein the pretreatment or retreatment comprises at least one of a filtration treatment, an adsorption treatment, a heating treatment, a catalytic treatment, an enrichment treatment, a concentration treatment, a chemical treatment, an optical treatment, and an electrical treatment.

31. The fluid treatment method according to claim 1, wherein the fluid treatment method is used for medical treatment and comprises selectively altering a concentration of specific molecules or combinations of molecules; wherein to be treated diseases comprise one or more of familial hypercholesterolemia, hyperlipoproteinemia, systemic lupus erythematosus, autoimmune diseases, myasthenia gravis, rapidly progressive glomerulonephritis, fatty liver, liver cirrhosis, acute liver failure, hyperlipidemia, severe acute pancreatitis, sepsis, Guillain-Barre syndrome, and obesity.

32. A fluid treatment method, comprising:
introducing a fluid into a separation pipeline through at least one second separation module; wherein the separation pipeline has an entrance and an exit, each of the at least one second separation module comprises a second separation component to divide a corresponding first separation module into a first side and a second side, two opposite ends of the first side of each of the at least one second separation module are communicated with at least one second inlet and at least one second outlet respectively, and two opposite ends of the second side of each of the at least one second separation module are communicated with the entrance and exit of the separation pipeline; and
driving a fluid in the separation pipeline to flow from the entrance of the separation pipeline to the exit of the separation pipeline at a third preset flow rate by at least one second driving device, to dynamically balance a total amount of the fluid in the separation pipeline.

33. The fluid treatment method according to claim 32, wherein the fluid introduced into the separation pipeline contains a target substance, and the fluid comprises one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, methane, light distillate oil, and liquefied gas.

34. The fluid treatment method according to claim 32, wherein the second separation component is a porous membrane, a reverse osmosis membrane, or a gas separation membrane.

35. The fluid treatment method according to claim 34, wherein the porous membrane or the reverse osmosis membrane has an average pore diameter or a molecular weight cutoff associated with compositions of the fluid; wherein the porous membrane comprises a microfiltration membrane, an ultrafiltration membrane, or a nanofiltration membrane.

36. The fluid treatment method according to claim 32, wherein each of the at least one second separation module is a tangential flow filtration module.

37. The fluid treatment method according to claim 32, wherein the second separation component comprises one or more of a plate membrane, a tubular membrane, a roll membrane, a spiral membrane, and a hollow fiber membrane.

38. The fluid treatment method according to claim 32, wherein the separation pipeline is further provided with at least one of a control device, a fluid storage device, a pressure detection device, a temperature detection device, a temperature control device, an oxygen detection device, a bubble detection and elimination device, an alarm device, and a concentration detection device.

39. The fluid treatment method according to claim 32, wherein N separation pipelines are cascaded to enable the fluid cyclically flow through the separation pipelines, and the separation pipelines comprise a first-stage separation pipeline and a second-stage separation pipeline adjacent to the first-stage separation pipeline, and the method further comprises: introducing a fluid in the first-stage separation pipeline into a corresponding second inlet of the second-stage separation pipeline, wherein N is a positive integer not less than 2.

40. The fluid treatment method according to claim 39, wherein a molecular size or a molecular weight of the target substance, or particle sizes of components of target substance as separated by each of the N cascaded separation pipelines, decreases as separation sequentially proceeds in the N cascaded separation pipelines.

41. The fluid treatment method according to claim 32, comprising:
introducing the fluid in the separation pipeline into an enrichment pipeline; wherein the enrichment pipeline comprises a first section and a second section, an entrance of the first section is communicated with at least one first inlet, and an exit of the second section is communicated with the entrance of the first section;
intercepting a target substance in the fluid by at least one first separation module; wherein each of the at least one first separation module comprises a first separation component to divide a corresponding first separation module into a first side and a second side, two opposite ends of the first side of each of the at least one first separation module are communicated with the first section and the second section respectively, and the second side of each of the at least one first separation module is communicated with at least one first outlet; and
driving the fluid in the enrichment pipeline to cyclically flow at a fourth preset flow rate based on at least one first driving device to enrich the target substance; wherein the at least one first driving device is arranged at the first section and is configured to dynamically balance a total amount of the fluid in the enrichment pipeline.

42. The fluid treatment method according to claim 41, wherein the first separation component is a porous membrane, a reverse osmosis membrane, or a gas separation membrane.

43. The fluid treatment method according to claim 41, wherein the porous membrane or the reverse osmosis membrane has an average pore diameter or a molecular weight cutoff associated with the target substance, wherein the porous membrane comprises a microfiltration membrane, an ultrafiltration membrane, or a nanofiltration membrane.

44. The fluid treatment method according to claim 41, wherein the first separation component comprises one or more of a planar membrane, a tubular membrane, a roll membrane, a spiral membrane, and a hollow fiber membrane.

45. The fluid treatment method according to claim 41, wherein each of the at least one first separation module is a tangential flow filtration module.

46. The fluid treatment method according to claim 45, wherein the at least one first driving device controls a flow rate in the first section to be above a preset threshold, so that the target substance in the first separation module flows from an exit of the first section to an entrance of the second section.

47. The fluid treatment method according to claim 46, wherein the preset threshold is determined based on at least one of a composition of the fluid, a temperature of the fluid, a structure of the first separation component, a material of the first separation component, a cavity structure of the first separation module, and a diameter of the enrichment pipeline.

48. The fluid treatment method according to claim 41, wherein the second section is further provided with one of the at least one first driving device.

49. The fluid treatment method according to claim 41, wherein a total volume or total velocity of the fluid introduced into the enrichment pipeline from the at least one first inlet is equal to that of a fluid discharged from the enrichment pipeline by the at least one first outlet to cyclically enrich the target substance.

50. The fluid treatment method according to claim 41, wherein an enrichment efficiency of the fluid treatment method is adjusted by controlling a ratio of the total volume of the fluid introduced into the enrichment pipeline per unit time to a total volume of the fluid in the enrichment pipeline

51. The fluid treatment method according to claim 41, wherein the enrichment pipeline is further provided with a channel-adjusting device for adjusting a flow direction of a fluid in the enrichment pipeline; wherein the channel-adjusting device adjusts the flow direction of the fluid in the enrichment pipeline by at least one of the following ways:
adjusting the flow direction of the fluid in the enrichment pipeline by adjusting a switching state of the at least one first inlet and/or the at least one first outlet; and
adjusting the flow direction of the fluid in the enrichment pipeline by adjusting a switching state of at least one adjusting channel connected to the enrichment pipeline.

52. The fluid treatment method according to claim 51, wherein the channel-adjusting device comprises a first pipeline switch arranged at each of the at least one first inlet, for controlling a switching state of the corresponding first inlet to further control a treatment state of the fluid in the enrichment pipeline to be a concentration cycle mode or a cleaning mode.

53. The fluid treatment method according to claim 52, wherein the channel-adjusting device further comprises at least one adjusting pipeline and at least one second pipeline switch arranged at the at least one adjusting pipeline so that an entrance of a cleaning solution and an exit of a waste fluid are formed in the enrichment pipeline.

54. The fluid treatment method according to claim 51, wherein the channel-adjusting device is a four-way rotary valve.

55. The fluid treatment method according to claim 51, wherein the enrichment pipeline is switched to a concentration cycle mode or a dilution mode, to adjust a separation efficiency of the at least one first separation module.

56. The fluid treatment method according to claim 41, wherein the enrichment pipeline further comprises an air inlet-outlet, arranged at the first section and/or the second section, for adjusting a state of air inside the enrichment pipeline or an air pressure inside the enrichment pipeline.

57. The fluid treatment method according to claim 41 or 56, wherein the enrichment pipeline further comprises at least one collection device, arranged at the first section and/or the second section, for adjusting a volume of the enrichment pipeline to collect the target substance, or for collecting bubbles.

58. The fluid treatment method according to claim 41, wherein the enrichment pipeline further comprises at least one of a control device, a fluid storage device, a pressure detection device, a temperature detection device, a temperature control device, an oxygen detection device, a bubble detection and elimination device, an alarm device, a flow rate detection device, and a concentration detection device.

59. The fluid treatment method according to claim 58, wherein the control device generates information for adjusting an internal working state of the enrichment pipeline based on at least one of the flow rate detection device, the pressure detection device, and the concentration detection device.

60. The fluid treatment method according to claim 32, or 39, or 41, further comprising: performing pretreatment or retreatment on the fluid by at least one treatment module; wherein the pretreatment or retreatment comprises at least one of a filtration treatment, an adsorption treatment, a heating treatment, a catalytic treatment, an enrichment treatment, a concentration treatment, a chemical treatment, an optical treatment, and an electrical treatment.

61. The fluid treatment method according to claim 41, wherein the at least one second inlet and the at least one second outlet are connected to a same storage section; wherein the storage section comprises a fluid storage device, a container, or a human body.

62. The fluid treatment method according to claim 32, wherein the fluid treatment method is used for medical treatment and comprises selectively altering a concentration of specific molecules or combinations of molecules; wherein to be treated diseases comprise one or more of familial hypercholesterolemia, hyperlipoproteinemia, systemic lupus erythematosus, autoimmune diseases, myasthenia gravis, rapidly progressive glomerulonephritis, fatty liver, liver cirrhosis, acute liver failure, hyperlipidemia, severe acute pancreatitis, sepsis, Guillain-Barre syndrome, and obesity.

63. A fluid treatment device, comprising: at least one cycle enrichment module; wherein the cycle enrichment module comprises:
an enrichment pipeline, comprising a first section and a second section; wherein an entrance of the first section is communicated with at least one first inlet, and an exit of the second section is communicated with the first section;
at least one first separation module; wherein each of the at least one first separation module comprises a first separation component to divide a corresponding first separation module into a first side and a second side; wherein two opposite ends of the first side are communicated with the first section and the second section respectively, and the second side is communicated with at least one first outlet; and
at least one first driving device, arranged at the first section, for driving a fluid to cyclically flow in the enrichment pipeline to dynamically balance a total amount of the fluid in the enrichment pipeline, so that the at least one first separation module enriches the target substance in a cycle enrichment mode.

64. The fluid treatment device according to claim 63, wherein the fluid contains the target substance, and the fluid comprises one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, methane, light distillate oil, and liquefied gas.

65. The fluid treatment device according to claim 63, wherein the first separation component is a porous membrane, a reverse osmosis membrane, or a gas separation membrane.

66. The fluid treatment device according to claim 63, wherein the porous membrane or the reverse osmosis membrane has an average pore diameter or a molecular weight cutoff associated with the target substance; wherein the porous membrane comprises a microfiltration membrane, an ultrafiltration membrane, or a nanofiltration membrane.

67. The fluid treatment device according to claim 63, wherein the first separation component comprises one or more of a planar membrane, a tubular membrane, a roll membrane, a spiral membrane, and a hollow fiber membrane.

68. The fluid treatment device according to claim 63, wherein the at least one first separation module is a tangential flow filtration module.

69. The fluid treatment device according to claim 68, wherein the at least one first driving device controls a flow rate in the first section to be above a preset threshold, so that the target substance in the at least one first separation module flows from an exit of the first section to an entrance of the second section.

70. The fluid treatment device according to claim 69, wherein the preset threshold is determined based on at least one of a composition of the fluid, a temperature of the fluid, a structure of the first separation component, a material of the first separation component, a cavity structure of the first separation module, and a diameter of the enrichment pipeline.

71. The fluid treatment device according to claim 63, wherein the second section is further provided with one of the at least one first driving device.

72. The fluid treatment device according to claim 63, wherein in the cycle enrichment mode, a total volume or total velocity of the fluid introduced into the enrichment pipeline from the at least one first inlet is equal to that of a fluid discharged from the enrichment pipeline by the at least one first outlet.

73. The fluid treatment device according to claim 63, wherein the cycle enrichment module controls a ratio of the total volume of the fluid introduced into the enrichment pipeline per unit time to a total volume of a fluid in the enrichment pipeline to adjust an enrichment efficiency.

74. The fluid treatment device according to claim 63, wherein the enrichment pipeline is further provided with a channel-adjusting device for adjusting a flow direction of a fluid in the enrichment pipeline; wherein the channel-adjusting device adjusts the flow direction of the fluid in the enrichment pipeline by at least one of the following ways:
adjusting the flow direction of the fluid in the enrichment pipeline by adjusting a switching state of the at least one first inlet and/or the at least one first outlet; and
adjusting the flow direction of the fluid in the enrichment pipeline by adjusting a switching state of at least one adjusting channel connected to the enrichment pipeline.

75. The fluid treatment device according to claim 74, wherein the channel-adjusting device comprises a first pipeline switch arranged at each of the at least one first inlet, for controlling a switching state of the corresponding first inlet to further control a treatment state of the fluid in the enrichment pipeline to be a concentration cycle mode or a cleaning mode.

76. The fluid treatment device according to claim 75, wherein the channel-adjusting device further comprises at least one adjusting pipeline and at least one second pipeline switch arranged at the at least one adjusting pipeline so that an entrance of a cleaning solution and an exit of a waste fluid are formed in the enrichment pipeline.

77. The fluid treatment device according to claim 74, wherein the channel-adjusting device is a four-way rotary valve.

78. The fluid treatment device according to claim 74, wherein the enrichment pipeline is switched to a concentration cycle mode or a dilution mode through the channel-adjusting device, to adjust a separation efficiency of the at least one first separation module.

79. The fluid treatment device according to claim 63, wherein the cycle enrichment module further comprises an air inlet-outlet, arranged at the first section or/and the second section, for adjusting a state of air inside the enrichment pipeline and an air pressure inside the enrichment pipeline.

80. The fluid treatment device according to claim 63 or 79, wherein the cycle enrichment module further comprises at least one collection device, arranged at the first section and/or the second section, for adjusting a volume of the enrichment pipeline to collect the target substance, or for collecting bubbles.

81. The fluid treatment device according to claim 63, wherein the cycle enrichment module further comprises at least one of a control device, a fluid storage device, a flow rate detection device, a pressure detection device, a temperature detection device, a temperature control device, an oxygen detection device, a bubble detection and elimination device, an alarm device, and a concentration detection device.

82. The fluid treatment device according to claim 81, wherein the control device generates information for adjusting an internal working state of the enrichment pipeline based on at least one of the flow rate detection device, the pressure detection device, and the concentration detection device.

83. The fluid treatment device according to claim 63, wherein the at least one first inlet and the at least one first outlet are connected to a same storage section; wherein the storage section comprises a fluid storage device, a container, or a human body.

84. The fluid treatment device according to claim 63, comprising: N cascaded cycle enrichment modules, wherein N is a positive integer not less than 2; wherein, in any two adjacent cycle enrichment modules comprising a former cycle enrichment module and a latter cycle enrichment module, a first outlet of the former cycle enrichment module is communicated with a first inlet of the latter cycle enrichment module.

85. The fluid treatment device according to claim 84, wherein a molecular size or a molecular weight of the target substance, or particle sizes of components of the target substance as enriched by each of the N cascaded cycle enrichment modules, decreases as enrichment sequentially proceeds in the N cascaded cycle enrichment modules.

86. The fluid treatment device according to claim 63, further comprising: at least one cycle separation module; wherein each of the at least one cycle separation module comprises:
a separation pipeline, having an entrance and an exit;
a second separation module, comprising a second separation component; wherein the second separation component divides the second separation module into a first side and a second side; wherein two opposite ends of the first side of the second separation module are communicated with at least one second inlet and at least one second outlet respectively, and two opposite ends of the second side of the second separation module are communicated with the entrance of the separation pipeline and the exit of the separation pipeline respectively; the at least one second inlet is communicated with a first outlet of the cycle enrichment module; and
at least one second driving device, arranged at the separation pipeline, for driving a fluid in the separation pipeline from the entrance of the separation pipeline to the exit of the separation pipeline at a preset flow rate, to dynamically balance a total amount of the fluid in the separation pipeline in a cycle separation mode.

87. The fluid treatment device according to claim 86, wherein the second separation component is a porous membrane, a reverse osmosis membrane, or a gas separation membrane.

88. The fluid treatment device according to claim 87, wherein the porous membrane or the reverse osmosis membrane has an average pore diameter or a molecular weight cutoff associated with compositions of the fluid; wherein the porous membrane comprises a microfiltration membrane, an ultrafiltration membrane, or a nanofiltration membrane.

89. The fluid treatment device according to claim 86, wherein the second separation module is a tangential flow filtration module.

90. The fluid treatment device according to claim 86, wherein the second separation component comprises one or more of a plate membrane, a tubular membrane, a roll membrane, a spiral membrane, and a hollow fiber membrane.

91. The fluid treatment device according to claim 86, wherein the cycle separation module further comprises at least one of a control device, a fluid storage device, a pressure detection device, a temperature detection device, a temperature control device, an oxygen detection device, a bubble detection and elimination device, an alarm device, and a concentration detection device.

92. The fluid treatment device according to claim 63, further comprising: at least one treatment module, for performing pretreatment or retreatment on the fluid in the fluid treatment device, wherein the pretreatment or retreatment comprises at least one of filtration, adsorption, heating, catalysis, enrichment, concentration, a chemical treatment, an optical treatment, and an electrical treatment.

93. The fluid treatment device according to claim 63, wherein the fluid treatment device is an extracorporeal cycle device; wherein the extracorporeal cycle device is a hemodialysis device, a blood purification device, a plasma exchange device, an extracorporeal peritoneal dialysis device, or an extracorporeal membrane oxygenation device.

94. A cycle separation device, for performing fluid treatment, comprising: at least one cycle separation module, wherein the cycle separation module comprises:
a separation pipeline, having an entrance and an exit;
a second separation module, comprising a second separation component; wherein the second separation component divides the second separation module into a first side and a second side; wherein two opposite ends of the first side of the second separation module are communicated with at least one second inlet and at least one second outlet respectively, and two opposite ends of the second side of the second separation module are communicated with the entrance of the separation pipeline and the exit of the separation pipeline respectively; and
at least one second driving device, arranged at the separation pipeline, for driving a fluid in the separation pipeline to flow from the entrance of the separation pipeline to the exit of the separation pipeline at a preset flow rate, to dynamically balance a total amount of the fluid in the separation pipeline in a cycle separation mode.

95. The cycle separation device according to claim 94, wherein the fluid contains the target substance, and the fluid comprises one or more of blood, plasma, serum, body fluid, tissue fluid, washing fluid, dialysate, recombinant protein solution, cell culture medium, microbial culture medium, pharmaceutical and medical water, liquid medicine, fluid food, animal and plant extract, natural water, industrial wastewater, recycled water, methane, light distillate oil, and liquefied gas.

96. The cycle separation device according to claim 94, wherein the second separation component is a porous membrane, a reverse osmosis membrane, or a gas separation membrane.

97. The cycle separation device according to claim 96, wherein the porous membrane or the reverse osmosis membrane has an average pore diameter or a molecular weight cutoff associated with compositions of the fluid; wherein the porous membrane comprises a microfiltration membrane, an ultrafiltration membrane, or a nanofiltration membrane.

98. The cycle separation device according to claim 97, wherein the second separation module is a tangential flow filtration module.

99. The cycle separation device according to claim 97, wherein the second separation component comprises one or more of a plate membrane, a tubular membrane, a roll membrane, a spiral membrane, and a hollow fiber membrane.

100. The cycle separation device according to claim 94, wherein the cycle separation module further comprises at least one of a control device, a fluid storage device, a pressure detection device, a temperature detection device, a temperature control device, a bubble detection and elimination device, an alarm device, and a concentration detection device.

101. The cycle separation device according to claim 94, comprising: N cascaded cycle separation modules, wherein N is a positive integer not less than 2; wherein, in any two adjacent cycle separation modules comprising a former cycle enrichment module and a latter cycle enrichment module, a separation pipeline of the former cycle enrichment module is communicated with a second inlet and a second outlet of the latter cycle separation module.

102. The cycle separation device according to claim 101, wherein t a molecular size or a molecular weight of the target substance, or particle sizes of components of the target substance as separated by each the N cascaded cycle separation modules, decreases as separation sequentially proceeds in the N cycle separation modules.

103. The cycle separation device according to claim 94, further comprising: at least one cycle enrichment module; wherein the cycle enrichment module comprises:
an enrichment pipeline, comprising a first section and a second section; wherein an entrance of the first section is communicated with at least one first inlet, and an exit of the second section is communicated with the first section;
at least one first separation module, communicated with an exit of the first section; wherein each of the at least one first separation module comprises a first separation component to divide a corresponding first separation module into a first side and a second side; wherein the first side of each of the at least one first separation module is communicated with an entrance of the second section, the second side of each of the at least one first separation module is communicated with at least one first outlet, and the at least one first inlet is communicated with the separation pipeline of the cycle separation module; and
at least one first driving device, arranged at the first section, for driving a fluid to cyclically flow in the enrichment pipeline to dynamically balance a total amount of the fluid in the enrichment pipeline, so that the at least one first separation module enriches the target substance in a cycle enrichment mode.

104. The cycle separation device according to claim 103, wherein the first separation component is a porous membrane, a reverse osmosis membrane, or a gas separation membrane.

105. The cycle separation device according to claim 104, wherein the porous membrane or the reverse osmosis membrane has an average pore diameter or a molecular weight cutoff associated with the target substance, wherein the porous membrane comprises a microfiltration membrane, an ultrafiltration membrane, or a nanofiltration membrane.

106. The cycle separation device according to claim 103, wherein the first separation component comprises one or more of a planar membrane, a tubular membrane, a roll membrane, a spiral membrane, and a hollow fiber membrane.

107. The cycle separation device according to claim 103, wherein each of the at least one first separation module is a tangential flow filtration module.

108. The cycle separation device according to claim 107, wherein the at least one first driving device controls a flow rate in the first section to be above a preset threshold, so that the target substance in the first separation module flows from the exit of the first section to the entrance of the second section.

109. The cycle separation device according to claim 108, wherein the preset threshold is determined based on at least one of a composition of the fluid, a temperature of the fluid, a structure of the first separation component, a material of the first separation component, a cavity structure of the at least one first separation module, and a diameter of the enrichment pipeline.

110. The cycle separation device according to claim 103, wherein the second section is further provided with one of at least one first driving device.

111. The cycle separation device according to claim 103, wherein in the cycle enrichment mode, the cycle enrichment module controls a total volume or total velocity of the fluid introduced into the enrichment pipeline from the at least one first inlet to be equal to that of a fluid discharged from the enrichment pipeline by the at least one first outlet.

112. The cycle separation device according to claim 103, wherein the cycle enrichment module controls a ratio of the total volume of the fluid introduced into the enrichment pipeline per unit time to a total volume of a fluid in the enrichment pipeline to adjust an enrichment efficiency.

113. The cycle separation device according to claim 103, wherein the cycle enrichment module comprises a channel-adjusting device, arranged at the first section and/or the second section, for adjusting a flow direction of a fluid in the enrichment pipeline; wherein the channel-adjusting device adjusts the flow direction of the fluid in the enrichment pipeline by at least one of the following ways:
adjusting the flow direction of the fluid in the enrichment pipeline by adjusting a switching state of the at least one first inlet and/or the at least one first outlet; and
adjusting the flow direction of the fluid in the enrichment pipeline by adjusting a switching state of at least one adjusting channel connected to the enrichment pipeline.

114. The cycle separation device according to claim 113, wherein the channel-adjusting device comprises a first pipeline switch arranged at each of the at least one first inlet, for controlling a switching state of the corresponding first inlet to further control the fluid in the enrichment pipeline to be a concentration cycle mode or a cleaning mode.

115. The cycle separation device according to claim 114, wherein the channel-adjusting device further comprises at least one adjusting pipeline and at least one second pipeline switch arranged at the at least one adjusting pipeline so that an entrance of a cleaning solution and an exit of a waste fluid are formed in the enrichment pipeline.

116. The cycle separation device according to claim 113, wherein the channel-adjusting device is a four-way rotary valve.

117. The cycle separation device according to claim 113, wherein the enrichment pipeline is switched to a concentration cycle mode or a dilution mode through the channel-adjusting device, to adjust a separation efficiency of the at least one first separation module.

118. The cycle separation device according to claim 103, wherein the cycle enrichment module further comprises an air inlet-outlet, arranged at the first section and/or the second section, for adjusting a state of air inside the enrichment pipeline and an air pressure inside the enrichment pipeline.

119. The cycle separation device according to claim 103 or 118, wherein the cycle enrichment module further comprises at least one collection device, arranged at the first section and/or the second section, for adjusting a volume of the enrichment pipeline to collect the target substance, or for collecting bubbles.

120. The cycle separation device according to claim 103, wherein the cycle enrichment module further comprises at least one of a control device, a fluid storage device, a pressure detection device, a temperature detection device, a temperature control device, a bubble detection and elimination device, an alarm device, and a concentration detection device.

121. The cycle separation device according to claim 103, wherein the cycle enrichment module further comprises a flow rate detection device, for detecting a dynamic state inside the enrichment pipeline to generate information for adjusting an internal working state of the enrichment pipeline.

122. The cycle separation device according to claim 94, further comprising: at least one treatment module, for performing pretreatment or retreatment on the fluid in the cycle separation device; wherein the pretreatment or retreatment comprises filtration, adsorption, heating, catalysis, enrichment, concentration, a chemical treatment, an optical treatment, and an electrical treatment.

123. The cycle separation device according to claim 94, wherein the cycle separation device is an extracorporeal cycle device; wherein the extracorporeal cycle device is a hemodialysis device, a blood purification device, a plasma exchange device, an extracorporeal peritoneal dialysis device, or an extracorporeal membrane oxygenation device.

124. A cycle treatment system, comprising:
at least one fluid treatment device according to any one of claims 63-93 and/or at least one cycle separation device according to any one of claims 94-123; and
a pipeline system, comprising a fluid-introducing pipeline and a fluid-returning pipeline.

125. The cycle treatment system according to claim 124, wherein the pipeline system further comprises at least one of an anticoagulation system, a control device, a storage device, a pressure detection device, a temperature detection device, a temperature control device, an oxygen detection device, a bubble detection and elimination device, an alarm device, and a concentration detection device.

126. The cycle treatment system according to claim 124, wherein the cycle treatment system is for at least one of cell collection, microbial collection, and material collection.

127. A medical device, comprising: a cycle treatment system according to any one of claims 124-126.

128. The medical device according to claim 127, wherein the medical device is used for medical treatment and comprises selectively altering a concentration of specific molecules or combinations of molecules; wherein to be treated diseases comprise one or more of familial hypercholesterolemia, hyperlipoproteinemia, systemic lupus erythematosus, autoimmune diseases, myasthenia gravis, rapidly progressive glomerulonephritis, fatty liver, liver cirrhosis, acute liver failure, hyperlipidemia, severe acute pancreatitis, sepsis, Guillain-Barre syndrome, and obesity.

129. The medical device according to claim 128, wherein the medical device is for disease treatment through combination with drugs.

130. A non-transitory computer-readable storage medium, wherein at least one program is stored on the computer-readable storage medium, and the fluid treatment method according to any one of claims 1-31, or the fluid treatment method according to any one of claims 32-62 is implemented when the at least one program is executed by a processor.
